# EUROPEAN PATENT APPLICATION

(11) **EP 4 628 488 A1**
(43) Date of publication of application: **08.10.2025**
(21) Application number: 23902747.7
(22) Date of filing: 13.12.2023
(51) Int. Cl.: C07D 405/14, C07D 403/12, C07D 417/14, C07D 403/14, A61K 31/506, A61P 35/00, A61P 31/12, A61P 37/00

(54) **CYCLIN K DEGRADER AND USE THEREOF**

(30) Priority: 16.12.2022 CN 202211629648; 18.10.2023 CN 202311349401
(71) Applicant: Adlai Nortye Biopharma Co., Ltd., Hangzhou, Zhejiang 311121 (CN)
(72) Inventor: XU, Beidi, Hangzhou, Zhejiang 311121 (CN); YU, Zhiyong, Hangzhou, Zhejiang 311121 (CN); HAN, Xiaojun, Hangzhou, Zhejiang 311121 (CN); CHANG, Xin, Hangzhou, Zhejiang 311121 (CN); QIU, Qingchong, Hangzhou, Zhejiang 311121 (CN); ZHANG, Ling, Hangzhou, Zhejiang 311121 (CN); WANG, Yao, Hangzhou, Zhejiang 311121 (CN); HE, Nanhai, Hangzhou, Zhejiang 311121 (CN); WANG, Xiaohan, Hangzhou, Zhejiang 311121 (CN); XIA, Xiangyu, Hangzhou, Zhejiang 311121 (CN)
(74) Representative: Office Freylinger
(86) International application number: PCT/CN2023/138417
(87) International publication number: WO 2024/125551

(57) **Abstract**

Provided are a Cyclin K degrader compound, and preparation method and uses thereof. Specifically, provided are the compounds represented by formula (I) and pharmaceutical compositions thereof, and use thereof for preventing and/or treating Cyclin K-related diseases.

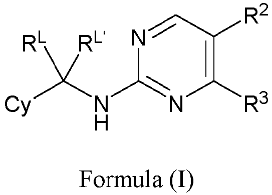

## Description

### Technical field

The present invention relates to a compound that degrades cyclin K, and methods of using the same to treat/prevent cyclin K-related disorders.

### Background

Tumor is the second largest killer threatening human health. About 10 million people die from tumors around the world every year. The abnormal expression of cellular proteins is considered to be an important factor leading to the occurrence and development of tumors, so most drugs target these abnormally expressed proteins. Compared with traditional inhibitor-based drug development, drug-induced protein degradation is a new strategy for targeting these tumor-associated proteins. According to the mechanism of action, protein degron can be divided into three categories, namely proteolysis targeting chimera (PROTAC), monovalent degron and molecular glue degron (Burslem, G. M. & Crews, C. M. Chem. Rev. 117, 11269-11301 (2017).).

PROTAC is currently the most widely used protein degradation technology, usually consisting of a protein targeting binding region, an E3 ubiquitin ligase recruitment region and a linker. PROTAC molecules bind to target proteins and recruit E3 ligases, which ubiquitinate the target proteins and ultimately lead to the degradation of the target proteins. Due to the large molecular weight of PROTACs (usually between 700-1200), their membrane permeability and oral bioavailability are poor. The molecular weight of monovalent degron and molecular glue is much smaller than PROTAC, which is more consistent with Lipinski's five principles (den Besten, W. etal. Nat Chem Biol 16, 1157-1158 (2020)). Monovalent degron induces degradation by binding to the protein and changing its conformation or other changes. Molecular glue ultimately leads to the degradation of the target protein by inducing the interaction between Culin-RING E3 ligase and the target protein. Molecular glue-mediated degradation of target proteins can be independent of the ligand pocket of the target protein, such as thalidomide analogs (Simonetta, K.R. etal. Nat Commun 10, 1402 (2019)) and arylsulfonamide analogs (Baek, K. etal. Nat Chem Biol 16, 2-3 (2020)) that have been reported so far and so on were developed according to this type of mechanism. Therefore, molecular glues can bring new hope to targets that were previously difficult to drug due to the lack of suitable ligand pockets.

CCNK is cell cycle protein K, also known as Cyclin K. It is the most important cyclin of cyclin-dependent kinase 12/13 (CDK12/13) (Pawel Lukasik,eral.Int J Mol Sci. 2021 Mar; 22(6) ): 2935). It can participate in the regulation of multiple biological processes such as transcription, post-transcriptional modification, cell cycle, and cell proliferation by forming a complex with CDK12/13. Early studies have shown that CDK12/13 forms a complex with Cyclin K and phosphorylates the C-terminal domain of RNA polymerase II to regulate its activity, thereby regulating the expression of DNA damage repair genes, such as BRCA1, ATR, FANC1, etc. (Malgorzata Krajewska et al. Nat Commun. 2019 Apr 15; 10(1): 1757.). CDK12/13 is considered a potential tumor treatment target (Cells 2020, 9 (6), 1483;). By designing a degron targeting Cyclin K, it affects the formation of the complex between CDK12/13 and Cyclin K protein, which provides a new idea for inhibiting the functions of CDK12/13. In 2020, Benjamin L. Ebert et al. reported (Nature 2020, 585, 293-297.) that the CDK inhibitor CR8 induces the degradation of Cyclin K protein through the mechanism of a molecular glue degrader. However, there are currently few molecular glue-based degradation agents for Cyclin K and they are unsatisfactory. It is urgent to find compounds with good degradation activity for Cyclin K.

### Summary of the Invention

The present invention provides a class of compounds exhibiting favorable Cyclin K degradation activity, as well as their stereoisomers, tautomers, solvates, pharmaceutically acceptable salts, metabolites, isotope derivatives, N-oxides, or prodrugs. The present invention further provides the use of said compounds and pharmaceutical compositions comprising the same in the treatment or prevention of Cyclin K-related disorders.

In one aspect, the present invention provides a compound having the structure of formula (I), or a pharmaceutically acceptable salt, prodrug, isotope derivative, stereoisomer, tautomer, N-oxide, solvate, or metabolite thereof: 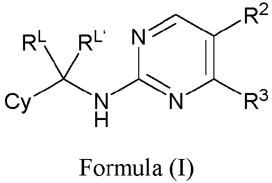 wherein, Cy represents an 11- to 14-membered tricyclic fused aromatic ring, and Cy represents 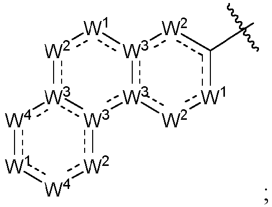 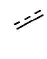 each independently represents a single bond or a double bond;
W¹ each independently represents CR, N, or a bond;
W² each independently represents CR⁰, N, NR^{a}, S, or O;
W³ each independently represents C or N, provided that no more than two W³ groups are simultaneously N;
W⁴ each independently represents CR¹, N, NR^{a}, S, or O
R, R⁰, and R¹ each independently represent hydrogen, halogen, nitro, cyano, -R^{a}, - OR^{a}, -SR^{a}, -NR^{a}R^{b}, -C(O)R^{a}, -C(O)OR^{a}, -C(O)NR^{a}R^{b}, -NR^{a}C(O)R^{b}, -S(O)₂R^{a}, - S(O)R^{a}, -S(O)₂NR^{a}R^{b}, -P(O)R^{a}R^{b}, a C₁-C₆ alkyl group, a C₂-C₆ alkenyl group, or a C₂-C₆ alkynyl group, wherein the alkyl, alkenyl, or alkynyl group may each independently be optionally substituted with 0 to 3 substituents selected from -OR^{a}, -SR^{a}, -NR^{a}R^{b}, - NR^{a}C(O)R^{b}, -C(O)R^{a}, -C(O)OR^{a}, -C(O)NR^{a}R^{b}, -S(O)₂R^{a}, -S(O)R^{a}, -S(O)₂NR^{a}R^{b}, and -P(O)R^{a}R^{b}; wherein in -NR^{a}C(O)R^{b}, R^{b} is optionally substituted with 0, 1, or 2 substituents selected from -(C₀-C₃ alkylene)OR^{a}, -(C₀-C₃ alkylene)SR^{a}, and -(C₀-C₃ alkylene)NR^{a}R^{b};
R^{L} and R^{L'} each independently represent hydrogen, fluorine, a C₁-C₆ alkyl group, or a C₃-C₆ cycloalkyl group, and R^{L} and R^{L'} may together with the carbon atom to which they are attached form a 3- to 6-membered ring;
R² represents a halogen, -R^{a}, -OR^{a}, -SR^{a}, a nitro group, a cyano group, -NR^{a}R^{b}, - NR^{a}C(O)R^{b}, -C(O)R^{a}, -C(O)OR^{a}, -C(O)NR^{a}R^{b}, -S(O)₂R^{a}, -S(O)R^{a}, -S(O)₂NR^{a}R^{b}, - P(O)R^{a}R^{b}, a (C₂-C₆) alkenyl group, or a (C₂-C₆) alkynyl group;
R³ represents a C₁-C₆ alkyl group, a C₁-C₆ alkenyl group, a C₁-C₆ alkynyl group, a C₃-C₁₀ cycloalkyl group, a 3- to 10-membered heterocycloalkyl group, a C₆-C₁₀ aryl group, a 5- to 10-membered heteroaryl group, -NR^{M}R^{N}, -NHR^{M}, -OR^{M}, or -SR^{M};
when R³ represents a C₁-C₆ alkyl group, a C₁-C₆ alkenyl group, a C₁-C₆ alkynyl group, a C₃-C₁₀ cycloalkyl group, or a 3- to 10-membered heterocycloalkyl group, it may optionally be substituted with 0, 1, 2, or 3 substituents independently selected from oxo, nitro, halogen, cyano, -R^{a}, -(C₀-C₆ alkylene)-OR^{a}, -(C₀-C₆ alkylene)-SR^{a}, -(C₀-C₆ alkylene)-NR^{a}R^{b}, -(C₀-C₆ alkylene)-NR^{a}C(O)R^{b}, -(C₀-C₆ alkylene)-C(O)R^{a}, - (C₀-C₆ alkylene)-C(O)OR^{a}, -(C₀-C₆ alkylene)-C(O)NR^{a}R^{b}, -(C₀-C₆ alkylene)-S(O)₂R^{a}, -(C₀-C₆ alkylene)-S(O)R^{a}, -(C₀-C₆ alkylene)-S(O)₂NR^{a}R^{b}, or -(C₀-C₆ alkylene)-P(O)R^{a}R^{b};
when R³ represents a C₆-C₁₀ aryl group or a 5- to 10-membered heteroaryl group, it may optionally be substituted with 0, 1, 2, or 3 substituents independently selected from nitro, halogen, cyano, -R^{a}, -(C₀-C₆ alkylene)-OR^{a}, -(C₀-C₆ alkylene)-SR^{a}, -(C₀-C₆ alkylene)-NR^{a}R^{b}, -(C₀-C₆ alkylene)-NR^{a}C(O)R^{b}, -(C₀-C₆ alkylene)-C(O)R^{a}, - (C₀-C₆ alkylene)-C(O)OR^{a}, -(C₀-C₆ alkylene)-C(O)NR^{a}R^{b}, -(C₀-C₆ alkylene)-S(O)₂R^{a}, -(C₀-C₆ alkylene)-S(O)R^{a}, -(C₀-C₆ alkylene)-S(O)₂NR^{a}R^{b}, or -(C₀-C₆ alkylene)-P(O)R^{a}R^{b};
when R³ represents -NR^{M}R^{N}, -NHR^{M}, -OR^{M}, or -SR^{M}, R^{M} and R^{N} each independently represent a C₁-C₆ alkyl group, a -(C₀-C₆ alkylene)(C₃-C₁₀ cycloalkyl) group, a -(C₀-C₆ alkylene)(3- to 10-membered heterocycloalkyl) group, a -(C₀-C₆ alkylene)(C₆-C₁₀ aryl) group, or a -(C₀-C₆ alkylene)(5- to 10-membered heteroaryl) group; R^{M} and R^{N} may each independently be optionally substituted with 0, 1, 2, or 3 substituents selected from oxo, nitro, halogen, cyano, -R^{a}, a 4- to 8-membered heterocycloalkyl group, -(C₀-C₆ alkylene)-OR^{a}, -(C₀-C₆ alkylene)-SR^{a}, -(C₀-C₆ alkylene)-NR^{a}R^{b}, -(C₀-C₆ alkylene)-NR^{a}C(O)R^{b}, -(C₀-C₆ alkylene)-C(O)R^{a}, -(C₀-C₆ alkylene)-C(O)OR^{a}, -(C₀-C₆ alkylene)-C(O)NR^{a}R^{b}, -(C₀-C₆ alkylene)-S(O)₂R^{a}, - (C₀-C₆ alkylene)-S(O)R^{a}, -(C₀-C₆ alkylene)-S(O)₂NR^{a}R^{b}, or -(C₀-C₆ alkylene)-P(O)R^{a}R^{b}; wherein when R^{M} or R^{N} represents a -(C₀-C₆ alkylene)(3- to 10-membered heterocycloalkyl) group containing a nitrogen atom, and the substituent is positioned on the nitrogen atom, the carbon atom adjacent to the nitrogen atom in the substituent may be further substituted with an oxo group;
wherein, R^{a} and R^{b} each independently represent a hydrogen atom, a C₁-C₆ alkyl group, or a C₃-C₈ cycloalkyl group, wherein the alkyl group or cycloalkyl group may each independently be optionally substituted with 0, 1, 2, or 3 halogen atoms.

In some embodiments of the present invention, Cy represents: 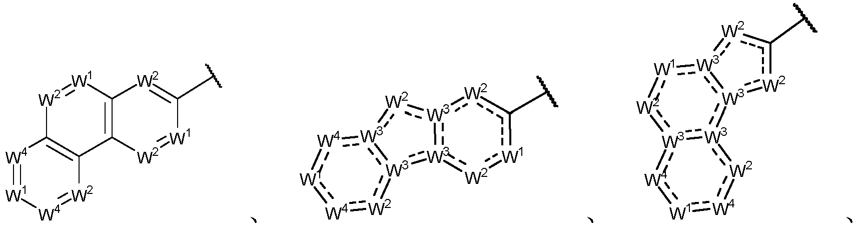 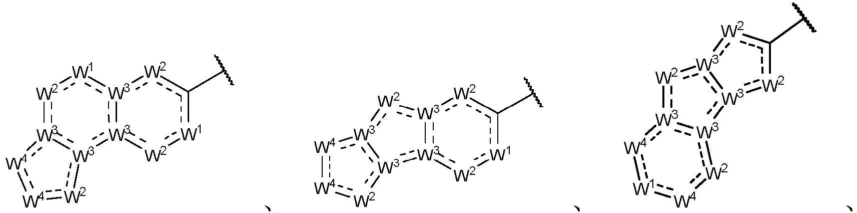 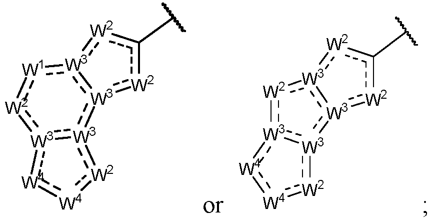 wherein each W¹ is independently selected from CR or N; and W², W³, and W⁴ are as defined above with respect to Formula (I).

In some embodiments of the present invention, Cy represents: 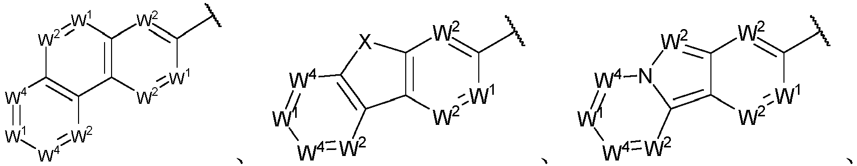 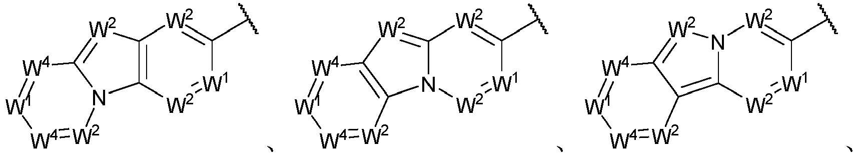 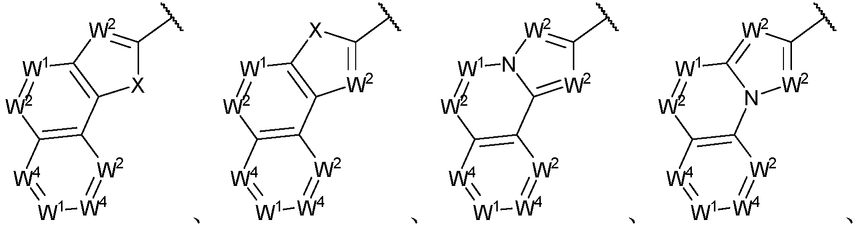 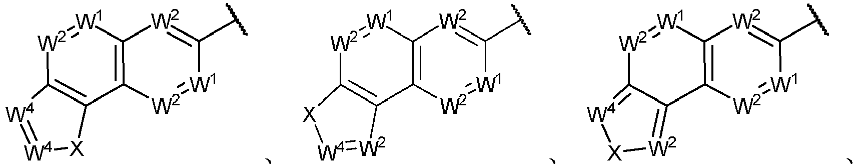 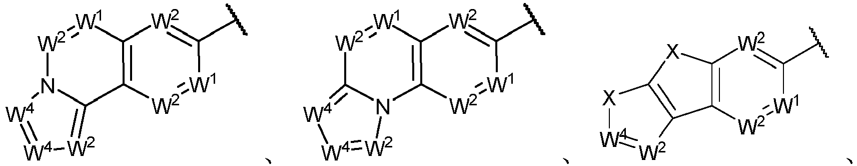 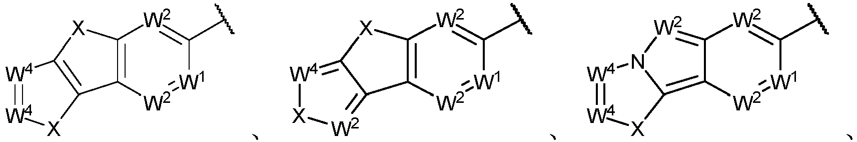 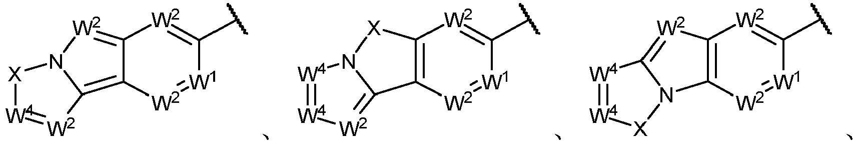 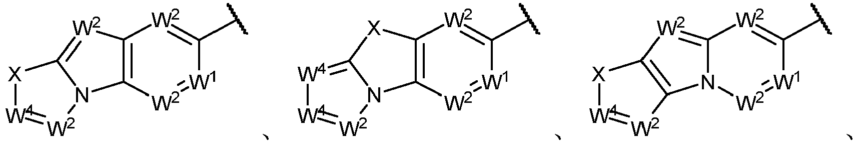 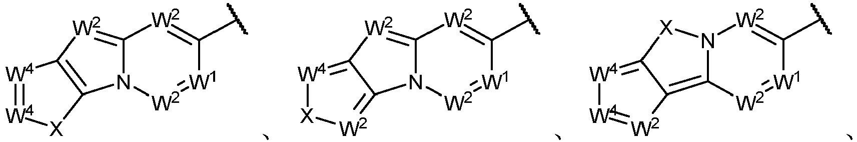 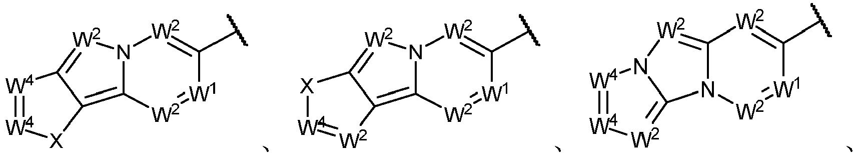 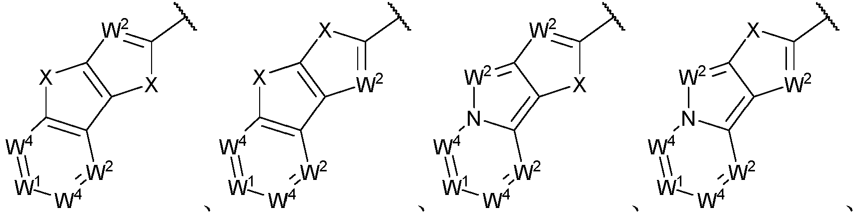 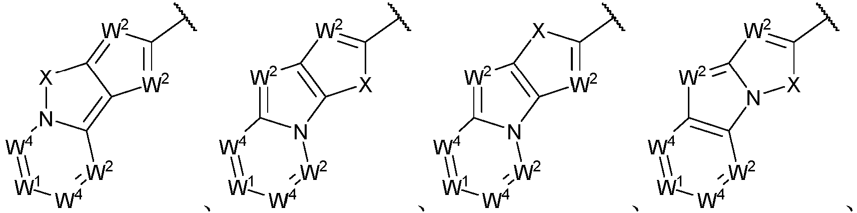 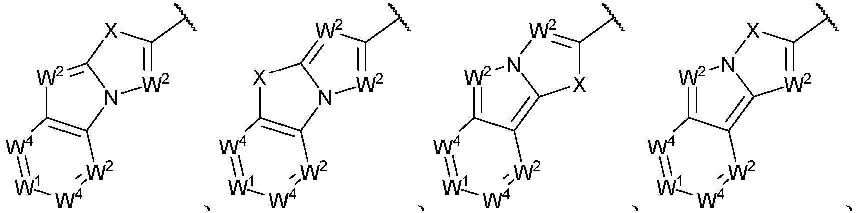 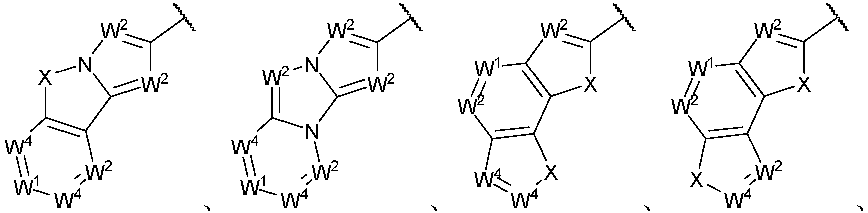 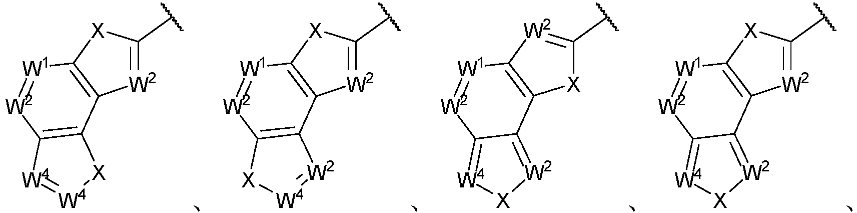 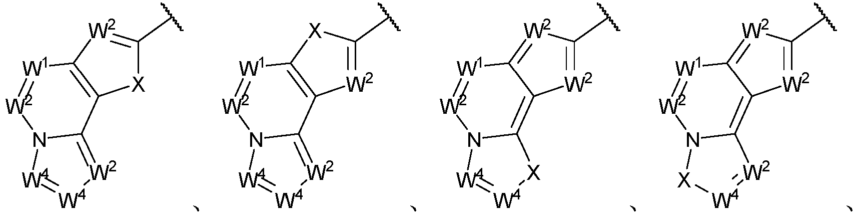 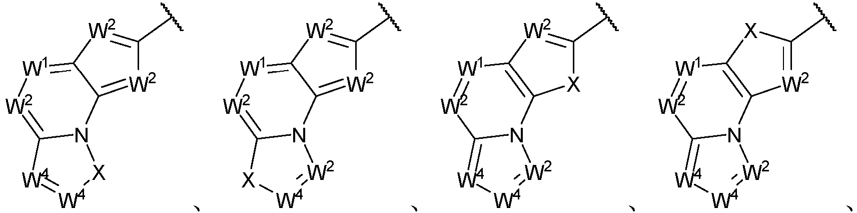 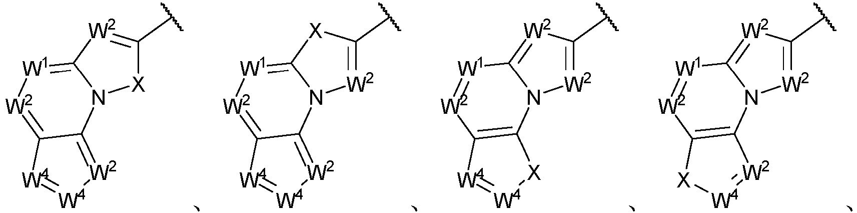 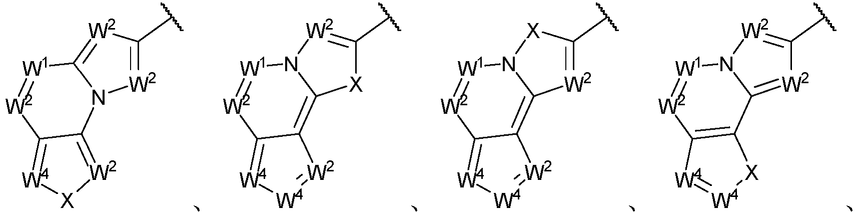 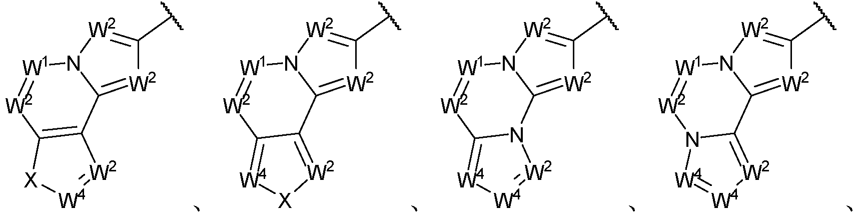 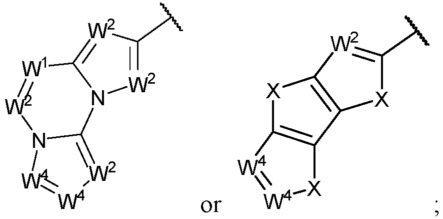 wherein each X is independently selected from NR^{a}, O, or S; each W¹ is independently selected from CR or N; each W² is independently selected from CR⁰ or N; and each W⁴ is independently selected from CR¹ or N.

In some embodiments of the present invention, Cy represents: 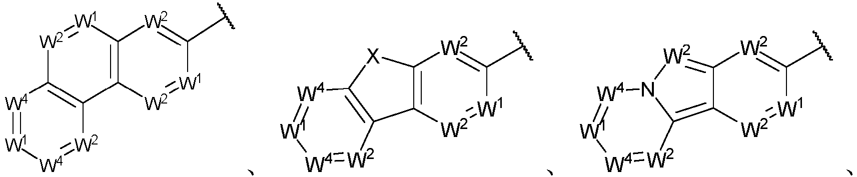 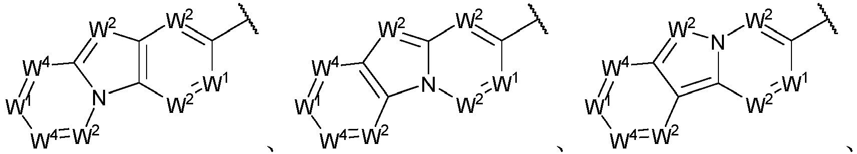 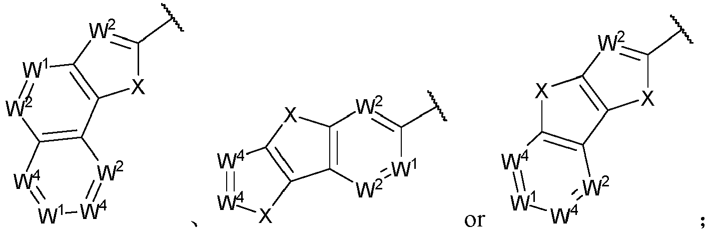 wherein each X is independently selected from NR^{a}, O, or S; each W¹ is independently selected from CR or N; each W² is independently selected from CR⁰ or N; and each W⁴ is independently selected from CR¹ or N.

In some embodiments of the present invention, each W¹ is independently selected from CR or N; wherein each R is independently selected from hydrogen, halogen, cyano, -R^{a}, or -OR^{a}; preferably, each R is independently selected from hydrogen, halogen, or - R^{a}; more preferably, each R is independently selected from hydrogen, C₁-C₆ alkyl.

In some embodiments of the present invention, each W² is independently selected from CR⁰ or N; wherein each R⁰ is independently selected from hydrogen, halogen, cyano, -R^{a}, or -OR^{a}; preferably, each R⁰ is independently selected from hydrogen, halogen, cyano, or -R^{a}; more preferably, each R is independently selected from hydrogen, cyano, or C₁-C₆ alkyl.

In some embodiments of the present invention, each W⁴ is independently selected from CR¹ or N; wherein each R¹ is independently selected from hydrogen, halogen, cyano, C₁-C₆ alkyl, C₃-C₈ cycloalkyl, -OR^{a}, -SR^{a}, -N R^{a}R^{b}, -C(O) R^{a}, -C(O)O R^{a}, - C(O)N R^{a}R^{b}, -N R^{a}C(O)R^{b}, -S(O)₂ R^{a}, or -S(O) R^{a}, wherein each C₁-C₆ alkyl may be independently substituted with 0, 1, 2, or 3 substituents selected from halogen, -O R^{a}, -S R^{a}, -N R^{a}R^{b}, -N R^{a}C(O)R^{b}, -C(O) R^{a}, -C(O)O R^{a}, -C(O)N R^{a}R^{b}, -S(O)₂ R^{a}, or - S(O) R^{a}; wherein the R^{b} in -N R^{a}C(O)R^{b} may optionally be substituted with 0, 1, or 2 substituents independently selected from -(C₀-C₃ alkylene)O R^{a}, -(C₀-C₃ alkylene)S R^{a}, and -(C₀-C₃ alkylene)N R^{a}R^{b}; preferably, the R^{b} in -N R^{a}C(O)R^{b} may optionally be substituted with 0, 1, or 2 substituents independently selected from -(C₀-C₃ alkylene)OH, -(C₀-C₃ alkylene)SH, and -(C₀-C₃ alkylene)NH₂; more preferably, in - N R^{a}C(O)R^{b}, R^{b} may optionally be substituted with 0, 1, or 2 substituents independently selected from -CH₂OH, -CH₂SH, and -CH₂NH₂.

In some preferred embodiments of the present invention, W₄ independently represents CR¹ or N, wherein R¹ independently represents hydrogen, a halogen atom, a cyano group, a C₁-C₆ alkyl group, a C₃-C₈ cycloalkyl group, -OR^{a}, -SR^{a}, -N R^{a}R^{b}, - C(O)R^{a}, -C(O)N R^{a}R^{b}, -N R^{a}C(O)R^{b}, or -S(O)₂R^{a}, wherein the C₁-C₆ alkyl group may optionally be substituted with 0, 1, 2, or 3 substituents independently selected from halogen atoms, -OR^{a}, -SR^{a}, -N R^{a}R^{b}, and -N R^{a}C(O)R^{b}, wherein, in -N R^{a}C(O)R^{b}, R^{b} may optionally be substituted with 0, 1, or 2 substituents independently selected from -(C₀-C₃ alkylene)OR^{a}, -(C₀-C₃ alkylene)SR^{a}, and -(C₀-C₃ alkylene)N R^{a}R^{b}; preferably, in -N R^{a}C(O)R^{b}, R^{b} may optionally be substituted with 0, 1, or 2 substituents independently selected from -(C₀-C₃ alkylene)OH, -(C₀-C₃ alkylene)SH, and -(C₀-C₃ alkylene)NH₂; more preferably, in -N R^{a}C(O)R^{b}, R^{b} may optionally be substituted with 0, 1, or 2 substituents independently selected from -CH₂OH, -CH₂SH, and -CH₂NH₂.

In some more preferred embodiments of the present invention, W⁴ independently represents CR¹ or N, wherein R¹ independently represents hydrogen, halogen, cyano, C₁-C₆ alkyl, C₃-C₈ cycloalkyl, -(C₀-C₆ alkylene)OR^{a}, -(C₀-C₆ alkylene)SR^{a}, -(C₀-C₆ alkylene)NR^{a}R^{b}, or -(C₀-C₆ alkylene)NR^{a}C(O)R^{b}.

**In** some embodiments of the present invention, R^{L} and R^{L}' each independently represent hydrogen or fluorine; preferably, R^{L} and R^{L}' are both hydrogen.

In some embodiments of the present invention, R² represents a halogen, -R^{a}, -OR^{a}, -SR^{a}, nitro, cyano, -NR^{a}R^{b}, or -NR^{a}C(O)R^{b}; preferably, R² represents a halogen or -R^{a}; more preferably, R² is -CF₃.

In some embodiments of the present invention, R³ represents a C₁-C₆ alkyl, C₃-C₁₀ cycloalkyl, or 3- to 10-membered heterocycloalkyl group, each of which may independently be substituted with 0, 1, 2, or 3 substituents selected from oxo, halogen, cyano, -R^{a}, -(C₀-C₆ alkylene)OR^{a}, -(C₀-C₆ alkylene)SR^{a}, -(C₀-C₆ alkylene)NR^{a}R^{b}, - (C₀-C₆ alkylene)NR^{a}C(O)R^{b}, -(C₀-C₆ alkylene)C(O)R^{a}, -(C₀-C₆ alkylene)C(O)OR^{a}, - (C₀-C₆ alkylene)C(O)NR^{a}R^{b}, -(C₀-C₆ alkylene)S(O)₂R^{a}, -(C₀-C₆ alkylene)S(O)R^{a}, - (C₀-C₆ alkylene)S(O)₂NR^{a}R^{b}, or -(C₀-C₆ alkylene)P(O)R^{a}R^{b}.

In some embodiments of the present invention, R³ represents a C₆-C₁₀ aryl or a 5-to 10-membered heteroaryl group, each of which may independently be substituted with 0, 1, 2, or 3 substituents selected from halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}.

In some embodiments of the present invention, R³ represents -NR^{M}R^{N}, -NHR^{M}, - OR^{M}, or -SR^{M}, wherein R^{M} and R^{N} are each independently selected from C₁-C₆ alkyl, - (C₀-C₆ alkylene)(C₃-C₁₀ cycloalkyl), -(C₀-C₆ alkylene)(3- to 10-membered heterocycloalkyl), -(C₀-C₆ alkylene)(C₆-C₁₀ aryl), or -(C₀-C₆ alkylene)(5- to 10-membered heteroaryl); R^{M} and R^{N} may each optionally be substituted with 0, 1, 2, or 3 substituents selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}; wherein, when R^{M} or R^{N} represents a -(C₀-C₆ alkylene)(3- to 10-membered heterocycloalkyl) containing a nitrogen atom and the substituent is located on said nitrogen atom, the carbon atom adjacent to said nitrogen atom in the substituent may be further substituted with an oxo group.

In some embodiments of the present invention, R³ represents -NR^{M}R^{N}, -NHR^{M}, - OR^{M}, or -SR^{M}, wherein R^{M} and R^{N} each independently represent C₁-C₆ alkyl, -(C₀-C₆ alkylene)(C₃-C₁₀ cycloalkyl), -(C₀-C₆ alkylene)(3- to 10-membered heterocycloalkyl), -(C₀-C₆ alkylene)(C₆-C₁₀ aryl), or -(C₀-C₆ alkylene)(5- to 10-membered heteroaryl); Each of R^{M} and R^{N} is optionally substituted with 0, 1, 2, or 3 substituents independently selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}.

In some embodiments of the present invention, R³ represents -NR^{M}R^{N}, -NHR^{M}, - OR^{M}, or -SR^{M}, wherein each of R^{M} and R^{N} independently represents hydrogen or C₁-C₆ alkyl, -(C₀-C₆ alkylene)(C₃-C₁₀ cycloalkyl), -(C₀-C₆ alkylene)(3- to 10-membered heterocycloalkyl), -(C₀-C₆ alkylene)(C₆-C₁₀ aryl), or -(C₀-C₆ alkylene)(5- to 10-membered heteroaryl); each of R^{M} and R^{N} may independently be substituted with 0, 1, 2, or 3 substituents selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, and -(C₀-C₆ alkylene)P(O)R^{a}R^{b}.

In some preferred embodiments of the present invention, R³ represents -NHR^{M}, - OR^{M}, or -SR^{M}, wherein R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl). R^{M} is independently optionally substituted with 0, 1, 2, or 3 substituents selected from oxo, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, and -(C₀-C₆ alkylene)P(O)R^{a}R^{b}.

**In** some preferred embodiments of the present invention, R³ represents -NHR^{M}, - OR^{M}, or -SR^{M}, wherein R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl). The R^{M} may optionally be substituted with 0, 1, 2, or 3 substituents independently selected from oxo, halogen, cyano, -R^{a}, -(C₀-C₆ alkylene)OR^{a}, -(C₀-C₆ alkylene)SR^{a}, -(C₀-C₆ alkylene)NR^{a}R^{b}, -NR^{a}C(O)R^{b}, -C(O)R^{a}, -C(O)NR^{a}R^{b}, -S(O)₂R^{a}, -S(O)R^{a}, -S(O)₂NR^{a}R^{b}, or -P(O)R^{a}R^{b}; more preferably, the R^{M} is independently substituted with 0, 1, 2, or 3 substituents selected from R^{a}, -(C₀-C₆ alkylene)OR^{a}, -(C₀-C₆ alkylene)SR^{a}, -(C₀-C₆ alkylene)NR^{a}R^{b}, and -C(O)NR^{a}R^{b}.

**In** some preferred embodiments of the present invention, R³ represents -NHR^{M}, - OR^{M}, or -SR^{M}, wherein each R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl). The R^{M} may optionally be substituted with 0, 1, 2, or 3 substituents independently selected from oxo, halogen, cyano, -R^{a}, -OR^{a}, -SR^{a}, -NR^{a}R^{b}, -NR^{a}C(O)R^{b}, -C(O)R^{a}, -C(O)NR^{a}R^{b}, -S(O)₂R^{a}, -S(O)R^{a}, -S(O)₂NR^{a}R^{b}, or -P(O)R^{a}R^{b}; more preferably, each R^{M} is independently substituted with 0, 1, 2, or 3 substituents selected from -R^{a}, - OR^{a}, -SR^{a}, -NR^{a}R^{b}, and -C(O)NR^{a}R^{b}.

In some more preferred embodiments of the present invention, R³ represents - NHR^{M}, -OR^{M}, or -SR^{M}, wherein each R^{M} is independently selected from C₁-C₆ alkyl, - (C₀-C₆ alkylene)(C₃-C₁₀ cycloalkyl), and -(C₀-C₆ alkylene)(3- to 10-membered heterocycloalkyl). Each R^{M} may optionally be substituted with 0, 1, 2, or 3 substituents independently selected from oxo, halogen, cyano, -OR^{a}, -SR^{a}, -NR^{a}R^{b}, -NR^{a}C(O)R^{b}, - C(O)R^{a}, -C(O)NR^{a}R^{b}, -S(O)₂R^{a}, -S(O)R^{a}, -S(O)₂NR^{a}R^{b}, or -P(O)R^{a}R^{b}; more preferably, each R^{M} is independently substituted with 0, 1, 2, or 3 substituents selected from -OR^{a}, -SR^{a}, -NR^{a}R^{b}, and -C(O)NR^{a}R^{b}.

In some embodiments of the present invention, R³ represents -NR^{M}R^{N}, -NHR^{M}, - OR^{M}, or -SR^{M}, and preferably R³ represents -NHR^{M}, -OR^{M}, or -SR^{M}, wherein R^{M} and R^{N} are each independently selected from -(C₀-C₆ alkylene)(3- to 10-membered heterocycloalkyl) containing a nitrogen atom, which may be optionally substituted with 0, 1, 2, or 3 substituents selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}, wherein the substituents are located on the nitrogen atom, and the carbon atom adjacent to the nitrogen atom in the substituent may be further substituted with oxo.

In some embodiments of the present invention, R^{a} and R^{b} are each independently hydrogen, C₁-C₃ alkyl, or C₃-C₆ cycloalkyl, wherein the alkyl and cycloalkyl groups may optionally be substituted with 0, 1, 2, or 3 halogen atoms.

In some preferred embodiments of the present invention, R^{a} and R^{b} are each independently hydrogen or C₁-C₃ alkyl, wherein the alkyl group may optionally be substituted with 0, 1, 2, or 3 halogen atoms.

The present invention encompasses any combination of the above embodiments.

More preferably, in some embodiments of the present invention, the compound represented by formula (I) is any one of the following compounds:

| | | | |
|---|---|---|---|
| 1 | 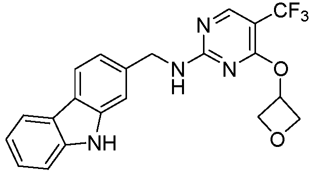 | 2 | 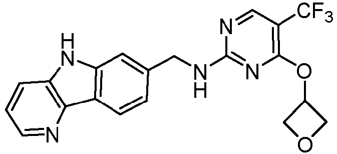 |
| 3 | 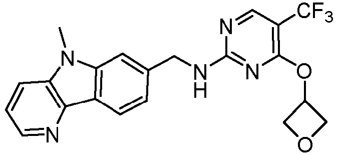 | 4 | 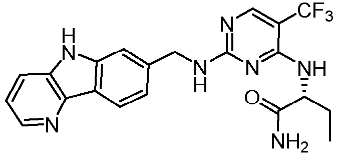 |
| 5 | 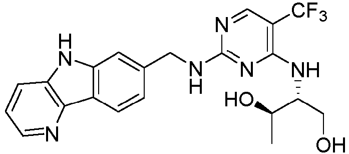 | 6 | 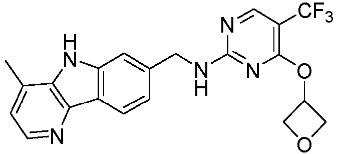 |
| 7 | 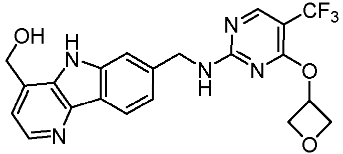 | 8 | 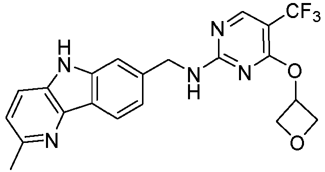 |
| 9 | 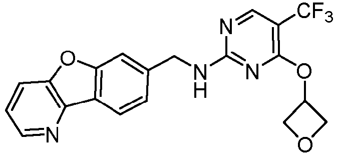 | 10 | 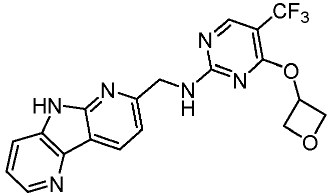 |
| 11 | 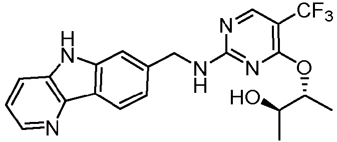 | 12 | 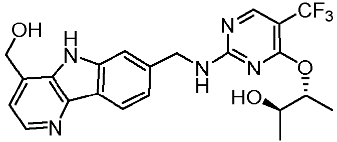 |
| 13 | 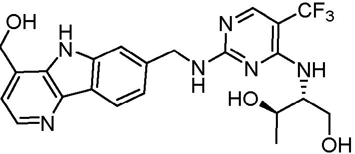 | 14 | 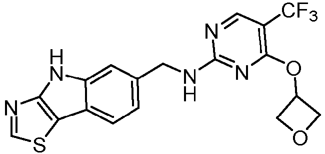 |
| 15 | 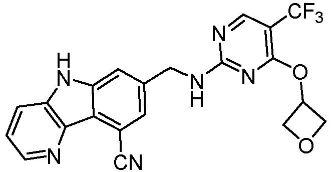 | 16 | 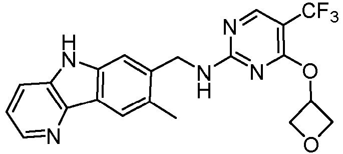 |
| 17 | 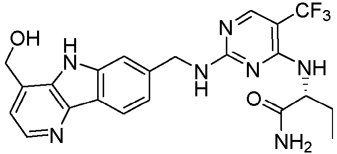 | 18 | 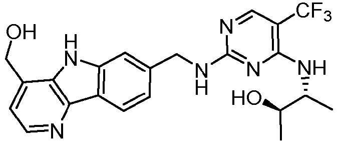 |
| 19 | 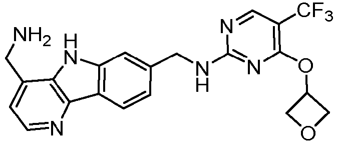 | 20 | 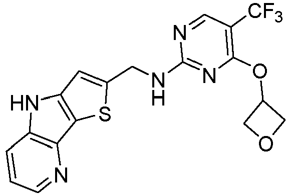 |
| 21 | 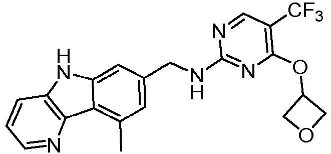 | 22 | 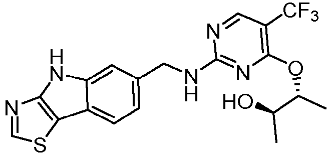 |
| 23 | 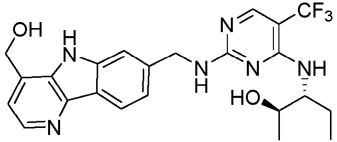 | 24 | 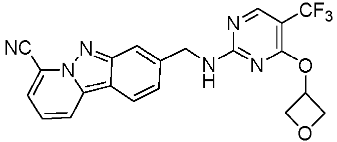 |
| 25 | 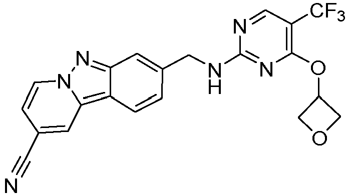 | 26 | 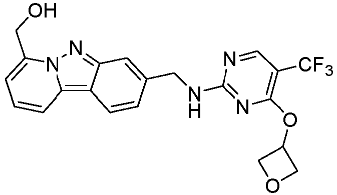 |
| 27 | 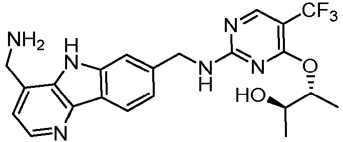 | 28 | 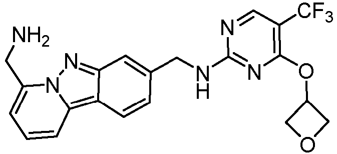 |
| 29 | 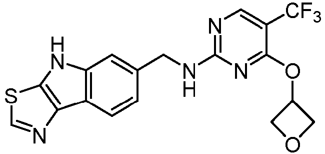 | 30 | 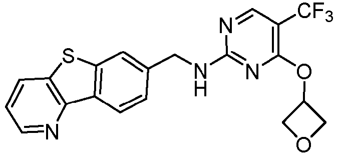 |
| 31 | 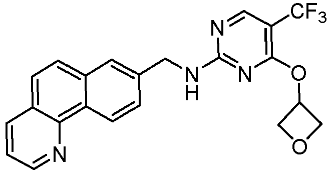 | 32 | 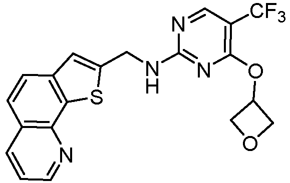 |
| 33 | 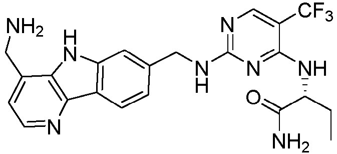 | 34 | 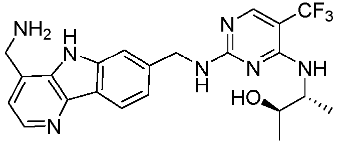 |
| 35 | 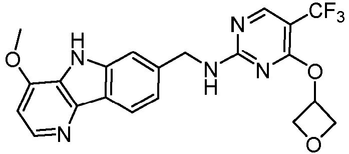 | 36 | 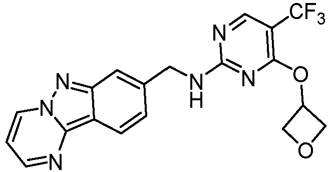 |
| 37 | 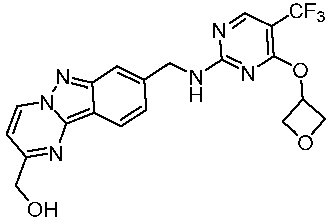 | 38 | 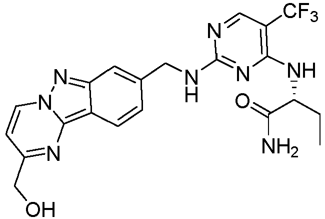 |
| 39 | 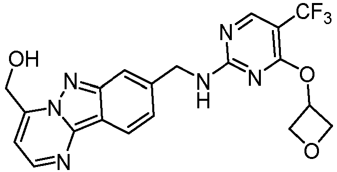 | 40 | 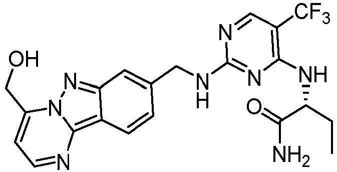 |
| 41 | 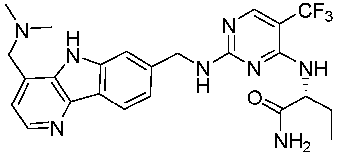 | 42 | 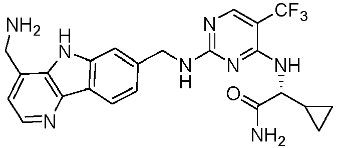 |
| 43 | 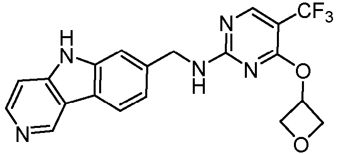 | 44 | 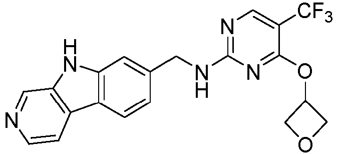 |
| 45 | 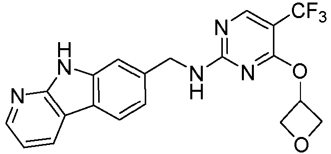 | 46 | 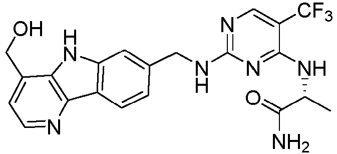 |
| 47 | 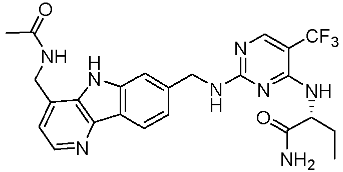 | 48 | 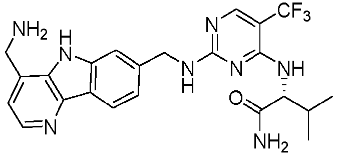 |
| 49 | 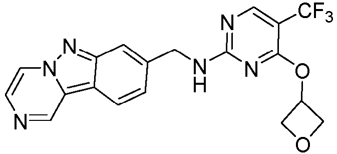 | 50 | 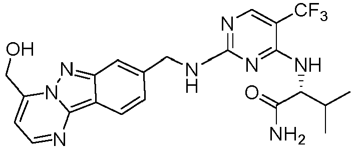 |
| 51 | 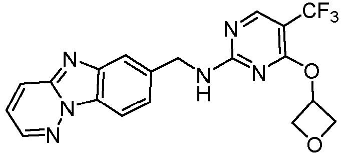 | 52 | 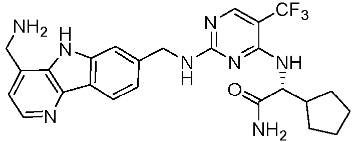 |
| 53 | 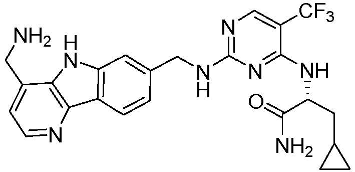 | 54 | 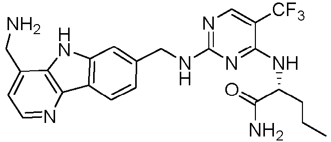 |
| 55 | 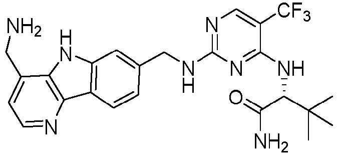 | 56 | 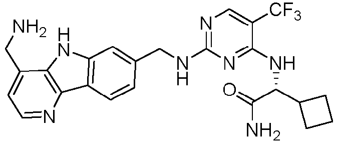 |
| 57 | 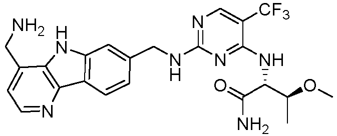 | 58 | 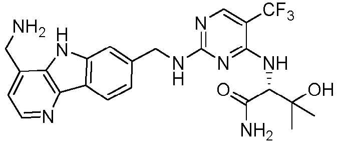 |
| 59 | 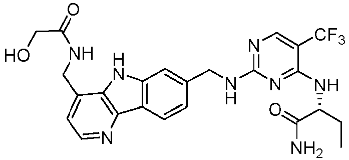 | 60 | 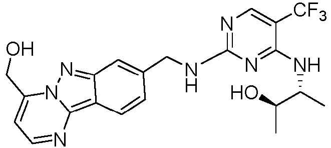 |
| 61 | 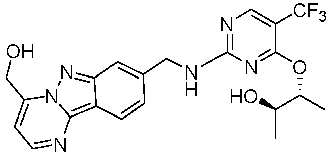 | 62 | 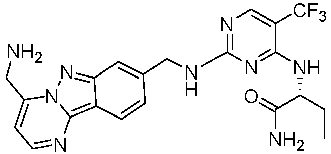 |
| 63 | 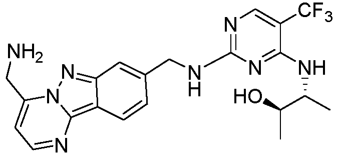 | 64 | 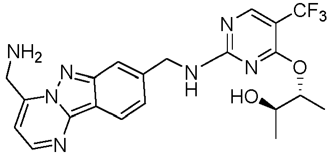 |
| 65 | 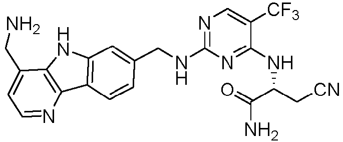 | 66 | 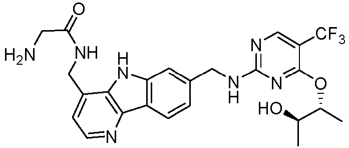 |
| 67 | 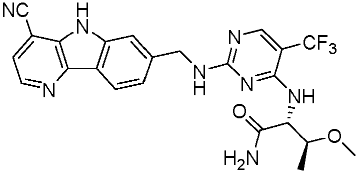 | 68 | 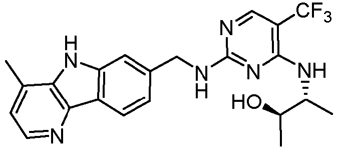 |
| 69 | 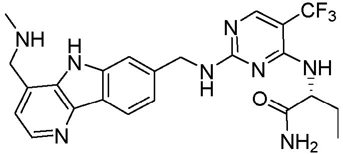 | 70 | 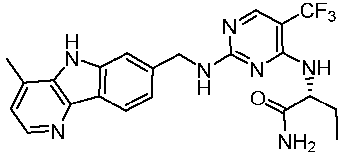 |
| 71 | 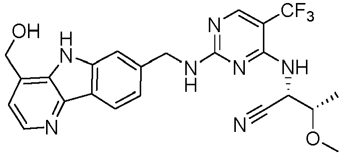 | 72 | 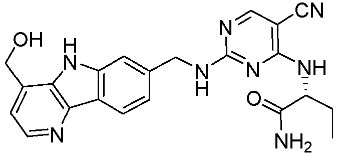 |
| 73 | 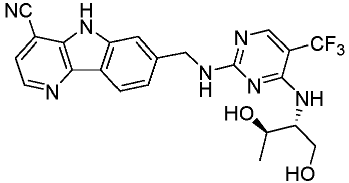 | 74 | 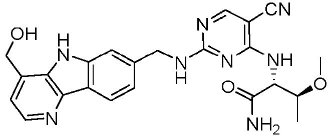 |
| 75 | 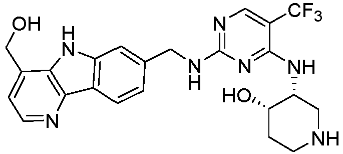 | 76 | 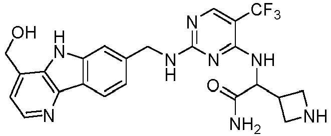 |
| 77 | 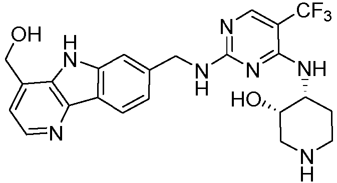 | 78 | 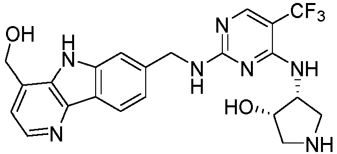 |
| 79 | 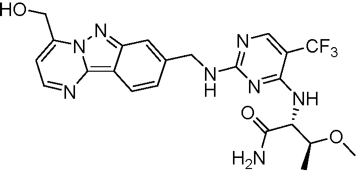 | 80 | 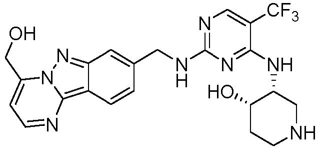 |
| 81 | 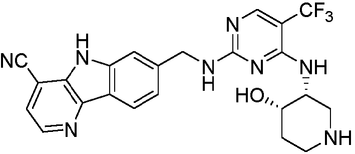 | 82 | 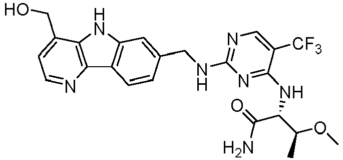 |
| 83 | 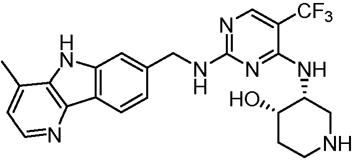 | 84 | 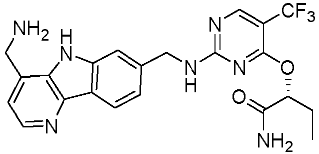 |
| 85 | 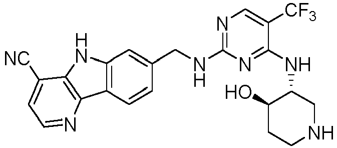 | 86 | 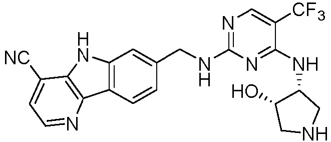 |
| 87 | 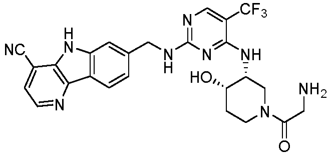 | 88 | 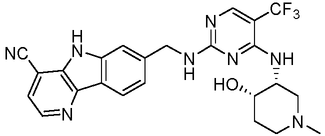 |
| 89 | 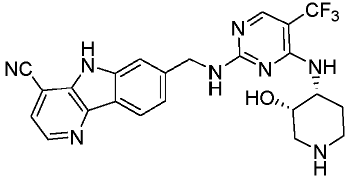 | 90 | 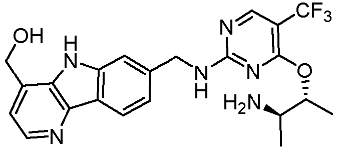 |
| 91 | 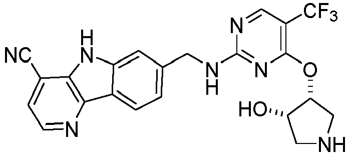 | 92 | 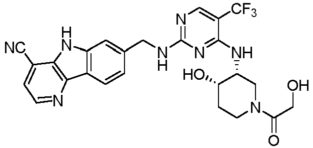 |
| 93 | 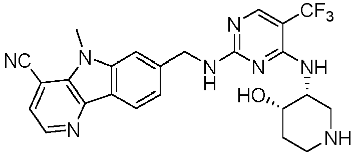 | 94 | 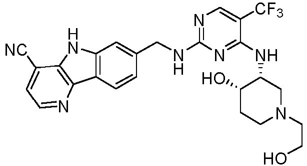 |
| 95 | 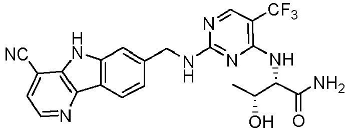 | 96 | 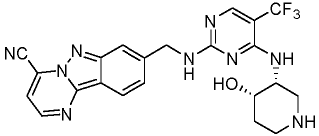 |
| 97 | 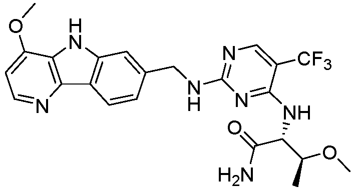 | 98 | 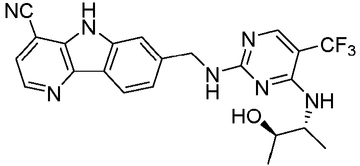 |
| 99 | 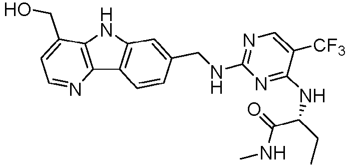 | 100 | 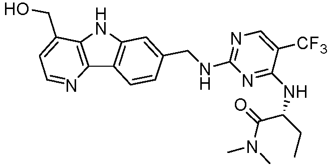 |
| 101 | 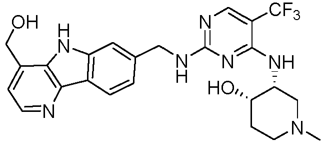 | 102 | 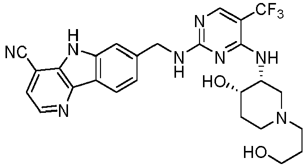 |
| 103 | 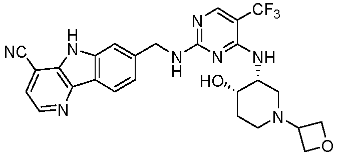 | 104 | 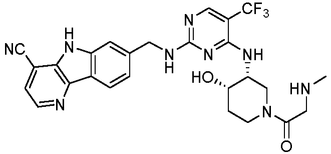 |

In another aspect, the present invention relates to a pharmaceutical composition comprising a compound as described herein, or a stereoisomer, tautomer, solvate, pharmaceutically acceptable salt, metabolite, isotope derivative, N-oxide, or prodrug thereof, and optionally a pharmaceutically acceptable carrier, diluent, or excipient.

In another aspect, the present invention relates to the use of a compound of formula (I) as described herein, or a stereoisomer, tautomer, solvate, pharmaceutically acceptable salt, metabolite, isotope derivative, N-oxide, or prodrug thereof, or the pharmaceutical composition as described herein, in the preparation of a medicament for the prevention or treatment of a disease or disorder associated with Cyclin K protein. In particular, the disease or disorder is selected from tumor, cancer, viral infection, inflammation-related disease, and autoimmune disease.

In another aspect, the present invention relates to a method for treating a disease or disorder associated with Cyclin K protein, comprising administering to a mammal in need thereof a compound as described herein, or a stereoisomer, tautomer, solvate, pharmaceutically acceptable salt, metabolite, isotope derivative, N-oxide, or prodrug thereof, or the pharmaceutical composition as described herein.

### Brief description of the drawings

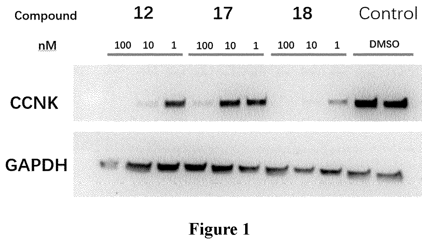

Figure 1. Compounds 12, 17, and 18 induced the degradation of Cyclin K after treating HEK293 cells for 6 h at different concentrations.

### Embodiments

### DETAILED DESCRIPTION OF THE INVENTION

It is specifically noted that, herein, when referring to a "compound" having a specific structural formula, stereoisomers, diastereomers, enantiomers, racemic mixtures and isotopic derivatives thereof are generally also encompassed.

It is well known to those skilled in the art that a salt, solvate, or hydrate of a compound is an alternative form of the compound, and they can all be converted into the compound under certain conditions. Therefore, it should be noted that when a compound is mentioned in this article, it generally also includes its pharmaceutically acceptable salts, and further includes its solvates and hydrates.

Similarly, when a compound is referred to herein, prodrugs, metabolites, and N-oxides thereof are also generally included.

The "stereoisomer" of the compound of formula (I) of the present invention means that when the compound of formula (I) has an asymmetric carbon atom, enantiomers will be produced; when the compound has a carbon-carbon double bond or a cyclic structure, cis-trans isomers will be produced; when the compound has a ketone or oxime, etc., it will produce tautomers. All enantiomers, diastereomers, racemates, cis-trans isomers, tautomers, geometric isomers, diastereomers, rotational isomers, and mixtures thereof of the compound of formula (I) are included in the scope of the present invention.

The "pharmaceutically acceptable salt" of the present invention refers to a pharmaceutically acceptable acid and base addition salt or a solvate thereof. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-(CH2)n-COOH (wherein n is 0-4)), etc. Alkaline salts: sodium salts, potassium salts, calcium salts, ammonium salts, etc. A variety of non-toxic pharmaceutically acceptable addition salts known to those skilled in the art.

The pharmaceutically acceptable salts of the present invention can be prepared by conventional methods, for example by dissolving the compound of the present invention in a water-miscible organic solvent (such as acetone, methanol, ethanol and acetonitrile), adding thereto an excess of organic acid or an aqueous inorganic acid solution to precipitate the salt from the resulting mixture, remove the solvent and remaining free acid therefrom, and then isolate the precipitated salt.

The precursors or metabolites described in the present invention can be precursors or metabolites known in the art, as long as the precursors or metabolites are converted into compounds through in vivo metabolism. For example, "prodrugs" refer to those prodrugs of the compounds of the present invention which, within the scope of reasonable medical judgment, are suitable for contact with tissues of humans and lower animals without undue toxicity, irritation, allergic reactions, etc., and demonstrate a reasonable benefit/risk ratio and are effective for its intended use. The term "prodrug" refers to a compound that is rapidly converted in vivo to produce the parent compound of the formula above, for example by metabolism in the body.

### Definition

Unless otherwise stated, the terms used in this application (including the specification and claims) are defined as follows. It must be noted that, in the specification and the appended claims, the singular forms "a," "a" and "an" include plural referents unless the context clearly dictates otherwise. If not otherwise stated, conventional methods of mass spectrometry, nuclear magnetic resonance, HPLC, protein chemistry, biochemistry, recombinant DNA technology, and pharmacology were used. In this application, the use of "or" or "and" means "and/or" unless stated otherwise.

In the description and claims, a given chemical formula or name shall cover all stereo and optical isomers as well as the racemates in which the above-mentioned isomers are present. Unless otherwise indicated, all chiral (enantiomeric and diastereomeric) and racemic forms are within the scope of the invention. Many geometric isomers of C=C double bonds, C=N double bonds, ring systems, etc. may also exist in the compounds, and all the above stable isomers are encompassed by the present invention. This invention describes the cis- and trans- (or E- and Z-) geometric isomers of the compounds of the invention, and they can be separated into mixtures of isomers or separate isomeric forms. The compounds of the present invention can be isolated in optically active or racemic form. All methods for preparing the compounds of the invention and the intermediates prepared therein are considered to be part of the invention. When preparing enantiomeric or diastereomeric products, they can be separated by conventional methods, for example by chromatography or fractional crystallization. Depending on the process conditions, the final product of the invention is obtained in free (neutral) or salt form. Both free forms and salts of these end products are within the scope of this invention. If desired, one form of the compound can be converted into another form. The free base or acid can be converted into a salt; the salt can be converted into the free compound or another salt; and mixtures of isomeric compounds of the invention can be separated into individual isomers. The compounds of the present invention, their free forms and salts, may exist in a variety of tautomeric forms in which hydrogen atoms are transposed to other parts of the molecule and thereby the chemical bonds between the atoms of the molecule are rearranged. It is to be understood that all tautomeric forms which may exist are included in the present invention.

In the present invention, when the listed linking groups do not specify their connection direction, their connection direction is arbitrary. For example, L in 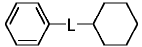 is -C(O)NH- , in this case, -C(O)NH- can be connected to phenyl and cyclohexyl in the order of reading from left to right to form 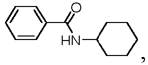 or it can be connected to phenyl and cyclohexyl in the opposite order of reading from left to right to form 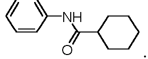 The combination of the linking group and the connected group is allowed only when such a stable compound is produced.

Unless otherwise defined, the definitions of the substituents in the present invention are independent and not related to each other. For example, for R^{a} (or R^{b}) in the substituent, it is independent in the definition of different substituents. Specifically, when one definition is chosen for R^{a} (or R^{b}) in one substituent, it does not mean that R^{a} (or R^{b}) has the same definition in other substituents. More specifically, for example (not exhaustive) for NR^{a}R^{b}, when the definition of R^{a} (or R^{b}) is selected from hydrogen, it does not mean that in -C(O)-NR^{a}R^{b}, R^{a} (or R^{b}) must be hydrogen. On the other hand, when there is more than one R^{a} (or R^{b}) in a certain substituent, these R^{a} (or R^{b}) are also independent. For example, in the substituent -(CR^{a}R^{b})m-O-(CR^{a}R^{b})ₙ-, when m+n is greater than or equal to 2, the m+n R^{a} (or R^{b}) are independent, and they can have the same or different meanings.

Unless otherwise defined, when a substituent is noted as "optionally substituted", the substituent is selected from, for example, the following substituents, such as alkyl, cycloalkyl, aryl, heterocyclyl, halogen, hydroxy, alkoxy, oxo, alkanoyl, aryloxy, alkanoyloxy, amino, alkylamino, arylamino, arylalkylamino, disubstituted amino (wherein 2 amino substituents are selected from alkyl radical, aryl or arylalkyl), alkanoylamino, aroylamino, aralkanoylamino, substituted alkanoylamino, substituted arylamino, substituted aralkanoylamino, thio, alkylthio, arylthio, arylalkylthio, arylthiocarbonyl, arylalkylthiocarbonyl, alkylsulfonyl, arylsulfonyl, arylalkylsulfonyl, aminosulfonyl such as -SO2NH2, substituted sulfonylamino, nitro, cyano, carboxyl, carbamoyl such as -CONH2, substituted carbamoyl such as -CONHalkyl, -CONHaryl, -CONHarylalkyl or two optional In the case of substituents from alkyl, aryl or arylalkyl, alkoxycarbonyl, aryl, substituted aryl, guanidino, heterocyclic, e.g. indolyl, imidazolyl, furyl, thienyl , Thiazolyl, pyrrolidinyl, pyridyl, pyrimidinyl, pyrrolidinyl, piperidinyl, morpholinyl, piperazinyl, homopiperazinyl, etc. and substituted heterocyclyl.

In the present invention, the term "alkyl" is intended to include branched and straight chain saturated aliphatic hydrocarbon groups with a specified number of carbon atoms. For example, "C₁-C₆ alkyl" means an alkyl group with 1 to 6 carbon atoms. Examples of alkyl groups include, but are not limited to, methyl, ethyl, n-propyl, isopropyl, n-butyl, isobutyl, tert-butyl, n-pentyl, isopentyl, neopentyl, etc. Preferred alkyl groups of the present invention include C₁-C₆ alkyl or C₁-C₄ alkyl. Alkyl groups may be unsubstituted or substituted, and when substituted, they may be substituted at any usable point of attachment, and the substituents are preferably selected from one or more of deuterium, halogen, hydroxyl, amino, cyano, alkyl, alkoxy, haloalkyl, cycloalkyl, heterocycloalkyl, aryl and heteroaryl.

In the present invention, the term "alkylene" is intended to include branched and straight-chain saturated aliphatic hydrocarbon groups having a specified number of carbon atoms, which are residues derived from the removal of two hydrogen atoms from the same carbon atom or two different carbon atoms of the parent alkane. For example, "C₀-C₆ alkylene" means an alkylene group having 0 to 6 carbon atoms, and C₀ alkylene means that the alkylene group does not exist (a bond). Examples of alkylene groups include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), etc. Preferred alkylene groups of the present invention include C₀-C₆ alkylene groups.

The term "alkenyl" denotes a straight or branched chain hydrocarbon group containing one or more double bonds and generally having a length of 2 to 20 carbon atoms. For example, "C₂-C₆ alkenyl" contains two to six carbon atoms. Alkenyl groups include, but are not limited to, e.g. vinyl, propenyl, butenyl, 1-methyl-2-buten-1-yl, and the like. Herein, alkenyl is preferably C₂-C₆ alkenyl.

The term "alkynyl" denotes a straight or branched chain hydrocarbon group containing one or more triple bonds and generally having a length of 2 to 20 carbon atoms. For example, "C₂-C₆ alkynyl" contains two to six carbon atoms. Representative alkynyl groups include, but are not limited to, for example, ethynyl, 1-propynyl, 1-butynyl, and the like. Herein, the alkynyl group is preferably a C₂-C₆ alkynyl group.

The term "alkoxy" or "alkyloxy" refers to -O-alkyl. "C₁-C₆ alkoxy" (or alkyloxy) is intended to include C₁, C₂, C₃, C₄, C₅, C₆ alkoxy. Examples of alkoxy include, but are not limited to, methoxy, ethoxy, propoxy (e.g., n-propoxy and isopropoxy), and tert-butoxy. Herein, the alkoxy group is preferably an alkoxy group having 1 to 6, more preferably 1 to 4 carbon atoms. Similarly, "alkylthio" or "thiothio" denotes an alkyl group as defined above having the indicated number of carbon atoms attached through a sulfur bridge; e.g. methyl-S- and ethyl-S-.

The term "carbonyl" refers to an organic functional group (C=O) consisting of two atoms, carbon and oxygen, linked by a double bond.

The term "aryl", alone or as part of a larger moiety such as "aralkyl", "arylalkoxy" or "aryloxyalkyl", refers to a monocyclic, bicyclic or tricyclic ring system having a total of 5 to 12 ring members, wherein at least one ring in the system is aromatic and wherein each ring in the system contains 3 to 7 ring members. In certain embodiments of the invention, "aryl" refers to an aromatic ring system which includes, but is not limited to, phenyl, biphenyl, indanyl, 1-naphthyl, 2-naphthyl, and tetrahydronaphthyl. The term "aralkyl" or "arylalkyl" refers to an alkyl residue attached to an aryl ring, non-limiting examples of which include benzyl, phenethyl, and the like. A fused aryl group can be attached to another group at a suitable position on the cycloalkyl ring or aromatic ring. Dashed lines drawn from ring systems indicate that bonds may be attached to any suitable ring atom.

The dashed line drawn from the ring system indicates that the bond can be connected to any suitable ring atom. The aryl group may be unsubstituted or substituted, and when substituted, it may be substituted at any available point of attachment, and the substituents are preferably selected from one or more of deuterium, halogen, hydroxy, amino, cyano, alkyl, alkoxy, haloalkyl, cycloalkyl, heterocycloalkyl, aryl and heteroaryl.

"Halo" or "halogen" includes fluorine, chlorine, bromine and iodine. "Haloalkyl" is intended to include branched and straight chain saturated aliphatic hydrocarbon groups having the specified number of carbon atoms substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl groups also include "fluoroalkyl groups" which are intended to include branched and straight chain saturated aliphatic hydrocarbon groups having the specified number of carbon atoms substituted with one or more fluorine atoms.

"Haloalkoxy" or "haloalkyloxy" means a haloalkyl group as defined above having the specified number of carbon atoms linked via an oxygen bridge. For example, "C₁-C₆ haloalkoxy" is intended to include C₁, C₂, C₃, C₄, C₅, C₆ haloalkoxy. Examples of haloalkoxy include, but are not limited to, trifluoromethoxy, 2,2,2-trifluoroethoxy, and pentafluoroethoxy. Similarly, "haloalkylthio" or "thiohaloalkoxy" means a sulfur-bridged haloalkyl group as defined above having the specified number of carbon atoms; for example, trifluoromethyl-S- and pentafluoroethyl-S-.

In this invention, the expression Cₓ₁-Cₓ₂ is used when referring to some substituent groups, which means that the number of carbon atoms in the substituent group may be x1 to x2. For example, C₀-C₈ means that the group contains 0, 1, 2, 3, 4, 5, 6, 7 or 8 carbon atoms, and C₁-C₈ means that the group contains 1, 2, 3, 4, 5 , 6, 7 or 8 carbon atoms, C₂-C₈ means that the group contains 2, 3, 4, 5, 6, 7 or 8 carbon atoms, C₃-C₈ means that the group contains 3, 4, 5 , 6, 7 or 8 carbon atoms, C₄-C₈ means that the group contains 4, 5, 6, 7 or 8 carbon atoms, C₀-C₆ means that the group contains 0, 1, 2, 3, 4 , 5 or 6 carbon atoms, C₁-C₆ means that the group contains 1, 2, 3, 4, 5 or 6 carbon atoms, C₂-C₆ means that the group contains 2, 3, 4, 5 or 6 carbon atoms, C₃-C₆ means that the group contains 3, 4, 5 or 6 carbon atoms.

In this invention, the expression "x1-x2 membered ring" is used when referring to cyclic groups (such as aryl, heteroaryl, cycloalkyl, and heterocycloalkyl), which means that the ring atom number of the group can be x1 to x2. For example, the 3-12-membered cyclic group can be a 3, 4, 5, 6, 7, 8, 9, 10, 11 or 12-membered ring, and the number of ring atoms can be 3, 4, 5, 6, 7, 8, 9, 10, 11 or 12; 3-6 membered ring means that the cyclic group can be a 3, 4, 5 or 6 membered ring, and the number of ring atoms can be 3, 4, 5 or 6; 3-8 membered ring means that the cyclic group can be 3, 4, 5, 6, 7 or 8 membered ring, and the number of ring atoms can be 3, 4, 5, 6, 7 or 8; 3-9 membered ring means that the cyclic group can be a 3, 4, 5, 6, 7, 8 or 9-membered ring, and the number of ring atoms can be 3, 4, 5, 6, 7, 8 or 9; 4-7 membered ring means that the cyclic group can be a 4, 5, 6 or 7-membered ring, and the number of ring atoms can be 4, 5, 6 or 7; 5-8-membered ring means that the cyclic group can be 5, 6, 7 or 8-membered ring, and the number of ring atoms can be 5, 6, 7 or 8; 5-12-membered ring means that the cyclic group can be 5, 6, 7, 8, 9, 10, 11 or 12-membered ring, and the number of ring atoms can be 5, 6, 7, 8, 9, 10, 11 or 12; 6-12-membered ring means that the cyclic group can be 6, 7, 8, 9, 10, 11 or 12-membered ring, and the number of ring atoms may be 6, 7, 8, 9, 10, 11 or 12. The ring atoms may be carbon atoms or heteroatoms, such as heteroatoms selected from N, O and S. When the ring is a heterocycle, the heterocycle may contain 1, 2, 3, 4 ring heteroatoms, for example heteroatoms selected from N, O and S.

In the context of the present invention, one or more halogens may be each independently selected from fluorine, chlorine, bromine and iodine.

In the present invention, the term "heteroaryl" refers to a monocyclic or polycyclic aromatic group containing one or more identical or different heteroatoms, including monocyclic heteroaryl groups and fused bicyclic or polycyclic ring systems comprising at least one heteroaromatic ring (i.e., an aromatic ring containing at least one heteroatom). The ring system may have 5, 6, 7, 8, 9, 10, 11, 12, 13, or 14 ring atoms, for example, 5 to 10 ring atoms. The heteroatoms may be oxygen, nitrogen, or sulfur. The carbon atoms and heteroatoms on the heteroaryl group may optionally be substituted with oxo groups (e.g., forming C=O, S(=O), or S(=O)₂). The heteroaryl group may be unsubstituted or substituted, and when substituted, substitution may occur at any available point of attachment. Preferred substituents include one or more selected from deuterium, halogen, hydroxy, amino, cyano, alkyl, alkoxy, haloalkyl, cycloalkyl, heterocycloalkyl, aryl, and heteroaryl.

In the present invention, the terms "heterocycloalkyl" or "heterocyclic group" refer to saturated or partially unsaturated monocyclic or polycyclic heterocyclic ring systems having 1, 2, 3, or 4 ring atoms independently selected from nitrogen (N), oxygen (O), and sulfur (S), with the remaining ring atoms being carbon. The nitrogen atom may optionally be quaternized, and the nitrogen and sulfur atoms may optionally be oxidized (e.g., to NO, SO, or SO₂). The heterocycloalkyl group includes monocyclic, bicyclic, and tricyclic ring systems, including spirocyclic, fused, and bridged heterocyclic systems. The heterocycloalkyl group may be unsubstituted or substituted, and when substituted, substitution may occur at any available point of attachment. Preferred substituents include one or more selected from deuterium, halogen, hydroxy, amino, cyano, alkyl, alkoxy, haloalkyl, cycloalkyl, heterocycloalkyl, aryl, and heteroaryl.

In the present invention, the terms "3- to 10-membered heterocycloalkyl" or "3-to 10-membered heterocyclic group" refer to saturated or partially unsaturated monocyclic or polycyclic heterocyclic ring systems composed of 3 to 10 ring atoms, wherein 1, 2, 3, or 4 of the ring atoms are independently selected from nitrogen (N), oxygen (O), and sulfur (S), and the remainder are carbon atoms. The nitrogen atom may optionally be quaternized, and the nitrogen and sulfur atoms may optionally be oxidized (i.e., to NO, SO, or SO₂). When the total number of S and O atoms in the ring exceeds one, such heteroatoms are non-adjacent. The term includes monocyclic, bicyclic, and tricyclic heterocyclic systems, including spirocyclic, fused, and bridged heterocycles. The "3- to 10-membered heterocyclic group" may include, but is not limited to, 3-, 4-, 5-, 6-, 7-, 8-, 9-, or 10-membered rings. Specific examples include azetidinyl, oxetanyl, pyrrolidinyl (including 2- and 3-substituted forms), piperidinyl (including 2-, 3-, and 4-substituted), piperazinyl, hexahydrodiazine, morpholinyl, dioxanyl, azacycloheptyl, 1,4-diazacycloheptyl, cyclopentyl-fused-pyrrolidinyl, pyrrolidinyl-fused-pyrrolidinyl, cyclopropyl-spiropiperazinyl, cyclobutyl-spiroazetidinyl, azetidinyl-spiroazetidinyl, cyclobutyl-spiroazacyclopentyl, azetidinyl-spirocyclopentyl, azetidinyl-spiroazacyclopentyl, cyclobutyl-spiroazacyclohexyl, azetidinyl-spirocyclohexyl, azetidinyl-spiroazacyclohexyl, cyclopentyl-spiroazacyclopentyl, azacyclopentyl-spiroazacyclopentyl, 3,6-diazabicyclo[3.1.1]heptanyl, 3,8-diazabicyclo[3.2.1]octanyl, or 8-azabicyclo[3.2.1]octanyl, among others.

In the present invention, the term "cycloalkyl" refers to a saturated or partially unsaturated monocyclic or polycyclic carbocyclic ring system, wherein all ring atoms are carbon atoms. The term includes monocyclic, fused, spirocyclic, and bridged cycloalkyl groups. The cycloalkyl group may be unsubstituted or substituted, and, when substituted, substitution may occur at any available position. Preferred substituents include one or more selected from deuterium, halogen, hydroxy, amino, cyano, alkyl, alkoxy, haloalkyl, cycloalkyl, heterocycloalkyl, aryl, and heteroaryl.

In the present invention, the term "C3-C10 cycloalkyl" refers to a saturated or partially unsaturated monocyclic or polycyclic carbocyclic ring system having 3 to 10 carbon atoms. Such a system may include monocyclic, fused, spirocyclic, or bridged rings. The term "C3-C12 cycloalkyl" encompasses rings consisting of 3, 4, 5, 6, 7, 8, 9, 10, 11, or 12 ring atoms. Specific examples include, but are not limited to, cyclopropyl, cyclobutyl, cyclopentyl, cyclohexyl, cycloheptyl, cyclopentyl-fused-cyclopentyl, spirocyclobutyl, and bicyclo[1.1.1]pentyl.

In the present invention, the term "fused ring" refers to a polycyclic group formed by two or more ring structures that share two adjacent atoms.

In the present invention, the term "aromatic fused ring" refers to a fused ring system that possesses aromaticity. Aromaticity may be determined by methods known in the art, such as Hückel's rule, which considers a ring aromatic when it has 4n+2 π-conjugated electrons.

The term "bridged cycloalkyl" as used herein refers to polycyclic compounds sharing two or more carbon atoms.

In the present invention, the term "spiro" refers to a polycyclic group in which two rings share a single carbon atom (referred to as the spiro atom).

The term "substituted" as used herein means that at least one hydrogen atom is replaced by a non-hydrogen group, provided that normal valency is maintained and that the substitution results in a stable compound. As used herein, a cyclic double bond is a double bond formed between two adjacent ring atoms (eg, C=C, C=N, or N=N).

Where nitrogen atoms (e.g. amines) are present on compounds of the invention, these nitrogen atoms can be converted to N-oxides by treatment with oxidizing agents (e.g. mCPBA and/or hydrogen peroxide) to obtain other compounds of the invention . Accordingly, both shown and claimed nitrogen atoms are considered to cover both the shown nitrogen and its N-oxides to obtain the derivatives of the present invention.

When any variable occurs more than one time in any composition or formula of a compound, its definition on each occurrence is independent of its definition at every other occurrence. Thus, for example, if a group is shown to be substituted with 0-3 R, then said group may be optionally substituted with up to three R groups, and R at each occurrence is independently selected from the definition of R. Also, combinations of substituents and/or variables are permissible only if such combinations result in stable compounds.

The term "patient" as used herein refers to an organism being treated by the methods of the present invention. Such organisms preferably include, but are not limited to, mammals (e.g., murine, ape, monkey, equine, bovine, porcine, canine, feline, etc.) and most preferably refer to humans.

The term "effective amount" as used herein means an amount of a drug or agent (i.e., a compound of the invention) that will elicit a biological or medical response in a tissue, system, animal or human being sought, e.g., by a researcher or clinician. Furthermore, the term "therapeutically effective amount" means an amount which results in improved treatment, cure, prevention or alleviation of a disease, disorder or side effect, or a reduction in the rate of disease or disorder progression. An effective amount may be given in one or more administrations, applications or doses and is not intended to be limited by a particular formulation or route of administration. The term also includes within its scope amounts effective to enhance normal physiological function.

The term "treating" is used herein in its broadest sense and encompasses therapeutic and/or prophylactic treatment of a subject. In particular, "treating" includes any treatment that results in the alleviation, suppression, elimination and amelioration and/or prevention of a condition, disease, disorder, etc., such as alleviation, reduction, regulation, amelioration, elimination, prevention, prevention or amelioration of symptom. The therapeutic treatment includes alleviating, inhibiting or ameliorating the symptoms or condition of the disease; inhibiting the occurrence of complications; improving the underlying metabolic syndrome; inhibiting the occurrence of the disease or symptoms, such as controlling the development of the disease or condition; alleviating the disease or symptoms; Reduction of disease or symptoms; reduction of complications caused by disease or symptoms, or treatment of symptoms caused by disease or disorders. Preventative treatment includes prior treatment to prevent, block or delay, slow the onset or progression of a disease or disorder or reduce the severity of a disease or disorder.

Likewise, "therapeutic agent" also includes agents or reagents that have therapeutic and/or prophylactic treatment in a subject.

The term "pharmaceutically acceptable" is used herein to refer to those compounds, substances, compositions and/or dosage forms: within the scope of sound medical judgment, they are suitable for use in contact with human and animal tissues without excessive toxicity, irritation, allergic reactions, and/or other problems or complications, commensurate with a reasonable benefit/risk ratio.

The phrase "pharmaceutically acceptable carrier" as used herein means a pharmaceutical substance, composition or vehicle, such as a liquid or solid filler, diluent, excipient, manufacturing aid (e.g. lubricant, talc, magnesium stearate, calcium stearate, or zinc stearate, or stearic acid) or solvent-encapsulated substances involved in the carrying or transport of a subject compound from one organ or body part to another. Each carrier must be "acceptable" in the sense of being compatible with the other ingredients of the formulation and not injurious to the patient.

The term "pharmaceutical composition" means a composition comprising a compound of the present invention together with at least one other pharmaceutically acceptable carrier. "Pharmaceutically acceptable carrier" means a medium generally accepted in the art for delivering a biologically active agent to an animal, particularly a mammal, including (i.e.) an adjuvant, excipient or vehicle, such as a diluent , preservatives, fillers, flow regulators, disintegrants, wetting agents, emulsifiers, suspending agents, sweeteners, flavoring agents, fragrances, antibacterial agents, antifungal agents, lubricants and dispersants, which depends on the mode of administration and the nature of the dosage form.

### Specific pharmaceutical and medical terms

The term "acceptable", as used herein, means that a formulation ingredient or active ingredient does not have an undue adverse effect on health for the general purpose of treatment.

The term "cancer", as used herein, refers to an abnormal growth of cells that cannot be controlled and, under certain conditions, is capable of metastasizing (spreading). Cancers of this type include, but are not limited to, solid tumors (e.g., bladder, bowel, brain, chest, uterus, heart, kidney, lung, lymphoid tissue (lymphoma), ovary, pancreas or other endocrine organs (e.g., thyroid), prostate , skin (melanoma), or blood cancer (such as non-leukemic leukemia).

The term "administration in combination" or similar terms, as used herein, refers to the administration of several selected therapeutic agents to a patient, in the same or different modes of administration at the same or different times.

The term "enhancing" or "capable of enhancing", as used herein, means that a desired result is capable of being increased or prolonged, either in potency or duration. Thus, in relation to enhancing the therapeutic effect of a drug, the term "capable of potentiating" refers to the ability of the drug to increase or prolong its potency or duration in the system. As used herein, "potency value" refers to the ability to maximize the enhancement of another therapeutic drug in an ideal system.

The term "immune disease" refers to a disease or condition of an adverse or deleterious reaction to an endogenous or exogenous antigen. The result is usually dysfunction of the cells, or destruction thereof and dysfunction, or destruction of organs or tissues that may produce immune symptoms.

The terms "kit" and "product packaging" are synonymous.

The term "subject" or "patient" includes mammals and non-mammals. Mammals include, but are not limited to, mammals: humans, non-human primates such as orangutans, apes, and monkeys; agricultural animals such as cattle, horses, goats, sheep, and pigs; domestic animals such as rabbits and dogs; experimental animals include rodents, such as rats, mice and guinea pigs. Non-mammalian animals include, but are not limited to, birds, fish, and the like. In a preferred aspect, the selected mammal is a human.

As used herein, a certain compound or pharmaceutical composition, after administration, can improve a certain disease, symptom or condition, especially improve its severity, delay the onset, slow down the progression of the disease, or reduce the duration of the disease. Circumstances that may be attributable to or related to the administration, whether fixed or episodic, continuous or intermittent.

### Specific Examples

The present invention can be better understood with reference to the following specific examples, which are intended to illustrate rather than limit the present invention.

When the preparation route is not mentioned in the present invention, the relevant raw materials and intermediates are purchased from commercial reagents (for example, from Bidepharm, PharmaBlock etc.).

The abbreviations in the present invention have the following meanings:

| abbreviation | meaning | abbreviation | meaning |
|---|---|---|---|
| ACN | Acetonitrile | m-CPBA | m-Chloroperbenzoic acid |
| Boc | tert-Butyloxycarbonyl | min | minute |
| DBU | 1,8-Diazabicyclo[5.4.0]undec-7-ene | MeOH | Methanol |
| DCE | 1,2-Dichloroethane | MS | Mass spectrometry |
| DCM | Dichloromethane | NMP | N-Methylpyrrolidone |
| DIAD | Diisopropyl azodicarboxylate | Ruphos | 2-Dicyclohexylphosphino-2',6'-diisopropoxy-1,1'-biphenyl |
| DIEA/DIPEA | N,N-Diisopropylethylamine | Pd(dppf)Cl₂ | [1,1'-Bis(diphenylphosphino)ferrocene] palladium(II) dichloride |
| DMF | N,N-Dimethylfonnamide | S-phos | Dicyclohexyl(2',6'-dimethoxy-[1,1'-biphenyl]-2-yl)phosphine |
| DMP | Dess-Martin periodinane | STAB | Sodium triacetoxyborohydride |
| DMSO-d₆ | Deuterated dimethyl sulfoxide | TBAF | Tetrabutylammonium fluoride |
| DPPA | Diphenylphosphoryl azide | TBS | tert-Butyldimethylsilyl |
| DPPE | 1,2-Bis(diphenylphosphino)ethan e | TFA | Trifluoroacetic acid |
| h | Hour | TFAA | Trifluoroacetic anhydride |
| HPLC | High performance liquid chromatography | THF | Tetrahydrofuran |
| Prep-HPLC | Preparative HPLC | TLC | Thin layer chromatography |
| LiHMDS | Lithium Hexamethylsilylamide | | |

In the following examples, unless otherwise specified, the reaction temperature is room temperature (10-30°C).

The compound of the present invention is separated and purified by preparative TLC, silica gel column chromatography, Prep-HPLC and/or silica gel flash column chromatography (Flash column chromatography), and its structure is confirmed by ¹H NMR and/or MS. The reaction was monitored by TLC or LC-MS.

¹H-NMR spectra were recorded on a Bruker instrument at 500 MHz. Chemical shift values are expressed in parts per million, i.e., δ values. The following abbreviations are used for the multiplicity of NMR signals: s = singlet, brs = broad, d = doublet, t = triplet, m = multiplet. Coupling constants are listed as J values, measured in Hz. The LC-MS experimental conditions were as follows: Instrument: Thermo U3000, ALLtech ELSD, MSQ, UV detector combined with ELSD and MSD (elution ratio of 4:1). Column: Waters X-Bridge C-18, 3.5 µm, 4.6x50 mm; Column temperature: 30°C. Gradient [time (min)/solvent B in A (%)]: 0.00/5.0, 1.40/95, 2.80/95, 2.82/5, 3.00/5. (Solvent A = 0.01% trifluoroacetic acid in water; Solvent B = 0.01% trifluoroacetic acid in acetonitrile). UV detection: 214/254/280/300 nm; DAD detection: 210-350 nm; flow rate: 2 mL/min; MS: ESI, 100-1500 m/z.

Preparative HPLC usually uses alkaline method or acidic method (alkaline method mobile phase: acetonitrile/0.05% ammonium bicarbonate aqueous solution, acidic method mobile phase: acetonitrile/0.05% formic acid aqueous solution); instrument is Thermo U3000 AFC-3000; column: Globalsil C-18 12 nm, 250 x 20 mm, 10 µm, or equivalent; flow rate: 20 mL/min, for gradient elution separation.

### Example 1:

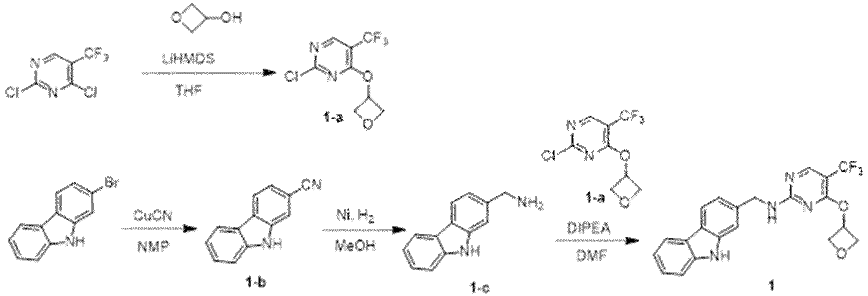

Dissolve 3-oxetanol (1.26 g, 17.0 mmol) and 2,4-dichloro-5-trifluoromethylpyrimidine (3.50 g, 16.1 mmol) in tetrahydrofuran (50 mL), and slowly add lithium bis(trimethylsilyl)amide (1 M in THF, 17.7 mL) dropwise at 0°C. After the mixture was stirred at 0°C for 3 hours, 50 mL of ammonium chloride solution was added to the reaction system, and the mixture was extracted twice with 100 mL of ethyl acetate. The combined organic phases were washed three times with water and once with saturated brine, dried over anhydrous sodium sulfate, filtered and concentrated. The residue was purified by column chromatography to obtain compound **1-a** (1.10 g, yield 26.9%).

3-Bromocarbazole (1 g, 4.06 mmol) was dissolved in N-methylpyrrolidone (5 mL) and cuprous cyanide (761 mg, 8.13 mmol) was added. The mixture was stirred at 170°C under microwave conditions for 5 hours. After the reaction solution was cooled to room temperature, 50 mL of ethyl acetate was added and the mixture was filtered through celite. The filtrate was washed three times with water and once with saturated brine, dried over anhydrous sodium sulfate, filtered and concentrated. The residue was purified by column chromatography to obtain compound **1-b** (500 mg, yield 64.0%). ESI-MS (m/z): 193.4 [M+H]⁺.

Compound **1-b** (500 mg, 2.60 mmol) was dissolved in methanol (10 mL), and Raney nickel (100 mg) was added. The mixture was stirred at room temperature under hydrogen atmosphere for 12 hours. The reaction solution was filtered through celite and concentrated. The residue was purified by flash column chromatography to obtain **1-c** (180 mg, yield 35.3%). ESI-MS (m/z): 197.5 [M+H]⁺.

Compound **1-c** (30 mg, 152 µmol) and compound **1-a** (38.9 mg, 152 µmol) were dissolved in N,N-dimethylformamide (2 mL), and N,N-diisopropylethylamine (59 mg, 458 µmol) was added. The mixture was stirred at 80°C for 2 hours. After the reaction solution was cooled to room temperature, it was purified by preparative liquid chromatography to obtain compound 1 (20 mg, yield 31.5%). ESI-MS (m/z): 415.4 [M+H]⁺; ¹H NMR (400 MHz, DMSO-*d*₆) δ 11.20 (s, 1H), 8.81 - 8.33 (m, 2H), 8.18 - 7.95 (m, 2H), 7.45 (d, *J =* 8.1 Hz, 1H), 7.40 - 7.31 (m, 2H), 7.19 - 7.05 (m, 2H), 5.73 - 5.46 (m, 1H), 4.95 - 4.41 (m, 6H).

### Example 2:

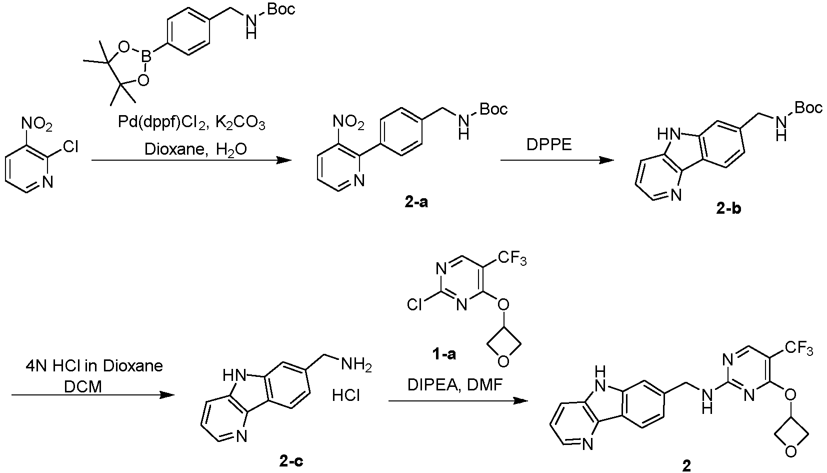

2-Chloro-3-nitropyridine (500 mg, 3.15 mmol) and tert-butyl (4-(4,4,5,5-tetramethyl-1,3,2-dioxaborolan-2-yl)benzyl)carbamate (1.05 g, 3.15 mmol) were added to 1,4-dioxane (5 mL) and water (0.5 mL), and 1,1'-bis(diphenylphosphino)ferrocenepalladium(II) dichloride (231 mg, 315 µmol) and potassium carbonate (872 mg, 6.31 mmol) were added. The mixture was stirred at 100°C for 16 hours. The reaction solution was cooled to room temperature, 30 mL of ethyl acetate was added, washed with water three times and saturated brine once, dried over anhydrous sodium sulfate, filtered and concentrated. The residue was purified by column chromatography to obtain compound **2-a** (600 mg, yield 57.8%). ESI-MS (m/z): 330.4 [M+H]⁺.

**2-a** (600 mg, 1.82 mmol) and 1,2-bis(diphenylphosphino)ethane (2.18 g, 5.47 mmol) were added to a flask. The mixture was reacted at 180°C for 0.5 hour. The mixture was cooled to room temperature and purified by column chromatography to obtain compound **2-b** (150 mg, yield 27.6%). ESI-MS (m/z): 298.3 [M+H]⁺.

Compound **2-b** (150 mg, 504 µmol) was dissolved in dichloromethane (10 mL), and dioxane hydrochloride solution (4 M, 2 mL) was added. The mixture was stirred at room temperature for 2 hours. The reaction solution was concentrated to obtain **2-c** (118 mg, yield 100%). ESI-MS (m/z): 198.4 [M+H]⁺.

Compound **2-c** (30 mg, 128 µmol) and compound **1-a** (32.7 mg, 128 µmol) were dissolved in N,N-dimethylformamide (2 mL), and N,N-diisopropylethylamine (49.8 mg, 385 µmol) was added. The mixture was stirred at 80°C for 2 hours. After the reaction solution was cooled to room temperature, it was purified by preparative liquid chromatography to obtain compound **2** (15 mg, yield 28.1%). ESI-MS (m/z): 416.4 [M+H]⁺; ¹H NMR (500 MHz, DMSO-*d*₆) δ 11.47 - 11.30 (m, 1H), 8.83 - 8.39 (m, 3H), 8.21 - 8.09 (m, 1H), 7.94 - 7.81 (m, 1H), 7.47 - 7.19 (m, 3H), 5.73 - 5.51 (m, 1H), 4.94 - 4.43 (m, 6H).

### Example 3:

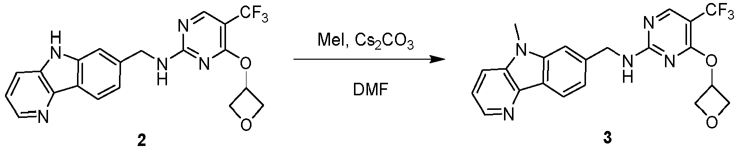

Compound 2 (20 mg, 48 µmol) was dissolved in N,N-dimethylformamide (2 mL), and cesium carbonate (31.4 mg, 96.3 µmol) and iodomethane (8.9 mg, 63 µmol) were added. The mixture was stirred at 50°C for 2 hours. After the reaction solution was cooled to room temperature, it was purified by preparative liquid chromatography to obtain compound **3** (5 mg, yield 24.2%). ESI-MS (m/z): 430.4 [M+H]⁺; ¹H NMR (500 MHz, DMSO-*d*₆) δ 8.78 - 8.35 (m, 3H), 8.22 - 8.10 (m, 1H), 8.01 (d, *J =* 8.3 Hz, 1H), 7.60 (s, 1H), 7.50 - 7.37 (m, 1H), 7.35 - 7.22 (m, 1H), 5.71 - 5.56 (m, 1H), 4.97 - 4.43 (m, 6H), 3.96 - 3.85 (m, 3H).

### Example 4:

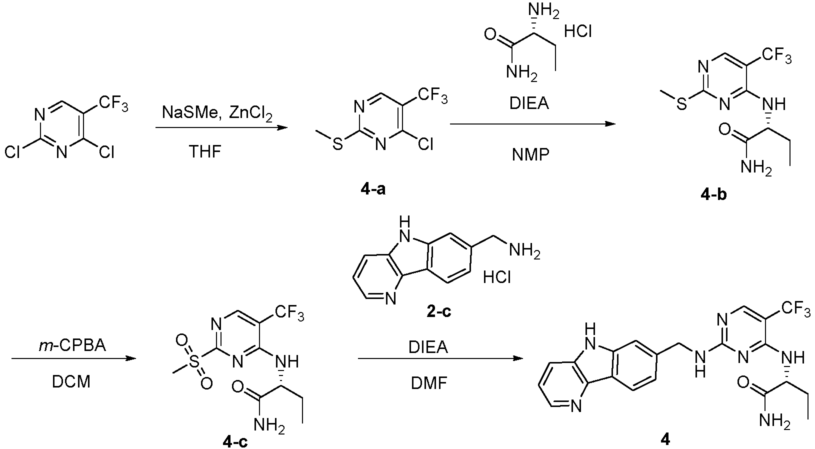

2,4-Dichloro-5-trifluoromethylpyrimidine (20 g, 92 mmol) and zinc chloride (16.3 g, 112 mmol) were dissolved in tetrahydrofuran (200 mL), and a 20% aqueous sodium thiomethoxide solution (48.5 g, 138 mmol) was added dropwise under ice-cooling. The mixture was stirred at 45°C for 16 hours. The reaction solution was cooled to room temperature, 300 mL of ethyl acetate was added, washed with water three times, washed once with saturated brine, dried over anhydrous sodium sulfate, filtered and concentrated. The residue was purified by column chromatography to give compound **4-a** (7.0 g, yield 33.2%).

Compound **4-a** (900 mg, 3.94 mmol) and (R)-2-aminobutanamide hydrochloride (813 mg, 7.97 mmol) were dissolved in N,N-dimethylformamide (10 mL), and N,N-diisopropylethylamine (1.53 g, 11.8 mmol) was added. The mixture was stirred at 70°C for 2 hours. The reaction solution was cooled to room temperature, 50 mL of ethyl acetate was added, washed with water three times and saturated brine once, dried over anhydrous sodium sulfate, filtered and concentrated. The residue was purified by column chromatography to give compound **4-b** (1.0 g, yield 86.3%). ESI-MS (m/z): 295.5 [M+H]⁺.

Compound 4-b (277 mg, 0.94 mmol) was dissolved in dichloromethane (10 mL), and m-chloroperbenzoic acid (325 mg, 1.88 mmol) was added. The mixture was stirred at room temperature for 2 hours. The reaction solution was concentrated, and the residue was purified by column chromatography to obtain compound **4-c** (150 mg, yield 48.9%). ESI-MS (m/z): 327.3 [M+H]⁺.

Compound **2-c** (30 mg, 128 µmol) and compound **4-c** (42 mg, 128 µmol) were dissolved in N,N-dimethylformamide (2 mL), and N,N-diisopropylethylamine (50 mg, 385 µmol) was added. The mixture was stirred at 80°C for 2 hours. After the reaction solution was cooled to room temperature, it was purified by preparative liquid chromatography to obtain compound **4** (20 mg, yield 35.1%). ESI-MS (m/z): 444.5 [M+H]⁺; ¹H NMR (500 MHz, DMSO-*d*₆) δ 8.80 - 8.40 (m, 4H), 8.30 - 8.11 (m, 1H), 7.84 (s, 1H), 7.75 - 7.60 (m, 2H), 7.48 - 7.31 (m, 2H), 4.83 - 4.72 (m, 2H), 4.60 (d, *J* = 6.5 Hz, 1H), 2.04 - 1.97 (m, 1H), 1.86 - 1.69 (m, 2H), 0.89 - 0.62 (m, 4H).

### Example 5:

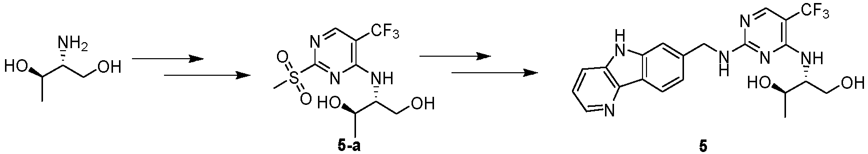

Using (2R,3R)-2-aminobutane-1,3-diol instead of (R)-2-aminobutanamide hydrochloride, compound **5-a** was obtained by referring to the synthesis of compound **4-c.** Compound **5** was obtained by replacing compound **4-c** with compound **5-a** and referring to the synthesis method of compound **4.** ESI-MS (m/z): 447.3 [M+H]⁺; ¹H NMR (500 MHz, DMSO-*d*₆) δ 12.74 - 12.32 (m, 1H), 8.86 - 8.68 (m, 2H), 8.60 - 8.45 (m, 2H), 8.34 - 8.11 (m, 1H), 7.93 - 7.83 (m, 1H), 7.78 - 7.01 (m, 3H), 4.82 - 4.74 (m, 2H), 4.12 - 3.97 (m, 2H), 0.95 - 0.81 (m, 3H).

### Example 6:

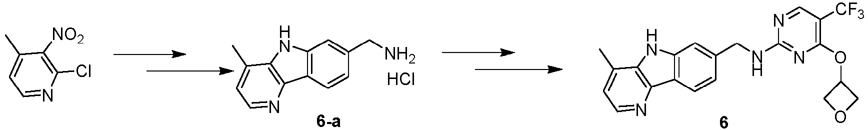

Using 2-chloro-4-methyl-3-nitropyridine instead of 2-chloro-3-nitropyridine, compound **6-a** was obtained by referring to the synthesis of compound **2-c.** Compound 6 was obtained by replacing compound **2-c** with compound **6-a** and referring to the synthesis method of compound 2. ESI-MS (m/z): 430.3 [M+H]+; ¹H NMR (500 MHz, DMSO-*d*₆) δ 11.36 (s, 1H), 8.82 - 8.41 (m, 2H), 8.39 - 8.29 (m, 1H), 8.16 - 8.07 (m, 1H), 7.51 - 7.41 (m, 1H), 7.23 - 7.17 (m, 2H), 5.70 - 5.53 (m, 1H), 5.35 - 4.89 (m, 1H), 4.78 - 4.57 (m, 4H), 4.45 - 4.41 (m, 1H), 2.57 (s, 3H).

### Example 7:

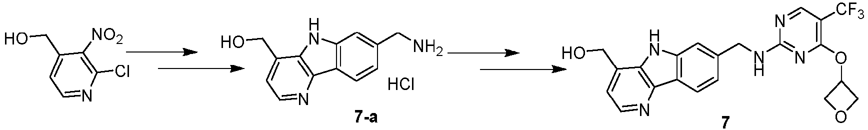

Using (2-chloro-3-nitropyridin-4-yl)methanol instead of 2-chloro-3-nitropyridine, compound **7-a** was obtained by referring to the synthesis of compound **2-c.** Compound **7** was obtained by replacing compound **2-c** with compound **7-a** and referring to the synthesis method of compound **2.** ESI-MS (m/z): 446.2 [M+H]⁺; ¹H NMR (500 MHz, DMSO-*d*₆) δ 11.24 (s, 1H), 8.80 - 8.37 (m, 3H), 8.16 - 8.01 (m, 1H), 7.51 - 7.15 (m, 3H), 5.68 - 5.47 (m, 2H), 4.92 - 4.67 (m, 5H), 4.65 - 4.40 (m, 3H).

### Example 8:

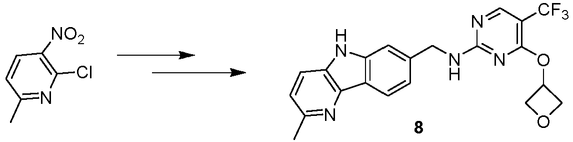

Using 2-chloro-6-methyl-3-nitropyridine instead of 2-chloro-3-nitropyridine, compound 8 was obtained by referring to the synthesis of compound **2.** ESI-MS (m/z): 430.3 [M+H]⁺; ¹H NMR (500 MHz, DMSO-*d*₆) δ 11.20 (s, 1H), 8.81 - 8.32 (m, 2H), 8.14 - 7.12 (m, 5H), 5.72 - 5.43 (m, 1H), 4.94 - 4.34 (m, 6H), 2.61 (s, 3H).

### Example 9:

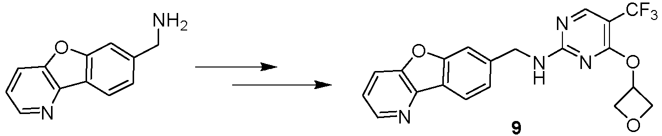

Compound **9** was obtained by replacing compound **1-c** with benzofuran[3,2-b]pyridine-7-methylamine and referring to the synthesis method of compound **1**. ESI-MS (m/z): 417.5 [M+H]⁺; ¹H NMR (500 MHz, DMSO-*d*₆) δ 8.81 - 8.49 (m, 2H), 8.48 - 8.39 (m, 1H), 8.22 - 8.09 (m, 2H), 7.70 (s, 1H), 7.59 - 7.51 (m, 1H), 7.51 - 7.43 (m, 1H), 5.70 - 5.52 (m, 1H), 4.95 - 4.35 (m, 6H).

### Example 10:

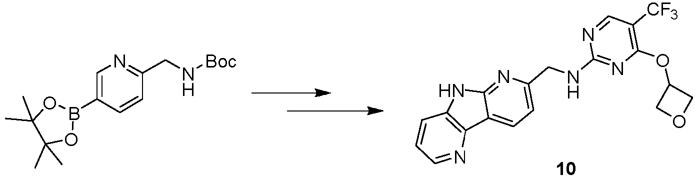

Compound **10** was obtained by replacing tert-butyl (4-(4,4,5,5-tetramethyl-1,3,2-dioxaborolan-2-yl)benzyl)carbamate with tert-butyl ((5-(4,4,5,5-tetramethyl-1,3,2-dioxaborolan-2-yl)pyridin-2-yl)methyl)carbamate. ESI-MS (m/z): 417.2 [M+H]⁺; ¹H NMR (500 MHz, DMSO-*d*₆) δ 11.97 (s, 1H), 8.78 - 8.40 (m, 4H), 7.95 - 7.86 (m, 1H), 7.49 - 7.42 (m, 1H), 7.27 - 7.21 (m, 1H), 5.76 - 5.36 (m, 1H), 5.35 - 4.65 (m, 3H), 4.64 - 4.53 (m, 2H), 4.32 - 4.29 (m, 1H).

### Example 11:

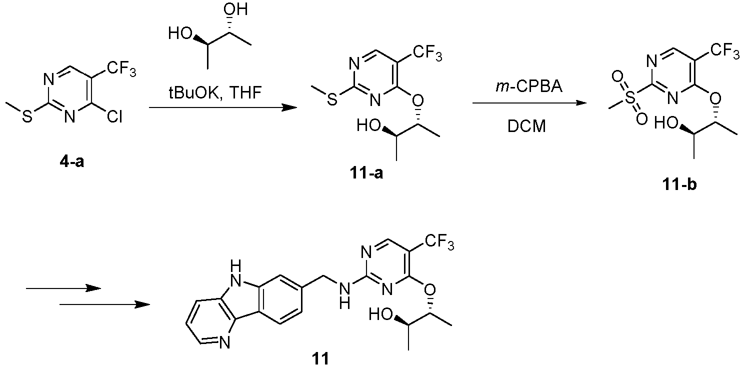

Compound **4-a** (1 g, 4.37 mmol) and (2R,3R)-2,3-butanediol (473 mg, 5.25 mmol) were dissolved in tetrahydrofuran (10 mL), and potassium tert-butoxide (589 mg, 5.25 mmol) was added. The mixture was stirred at 70°C for 2 hours. The reaction solution was cooled to room temperature, 50 mL of ethyl acetate was added, washed with water three times and saturated brine once, dried over anhydrous sodium sulfate, filtered and concentrated. The residue was purified by column chromatography to obtain compound **11-a** (400 mg, yield 32.4 %). ESI-MS (m/z): 283.1 [M+H]⁺.

Compound **11-a** (400 mg, 1.42 mmol) was dissolved in dichloromethane (10 mL), and m-chloroperbenzoic acid (318 mg, 1.84 mmol) was added. The mixture was stirred at room temperature for 2 hours. The reaction solution was concentrated, and the residue was purified by column chromatography to obtain compound **11-b** (250 mg, yield 56.1%). ESI-MS (m/z): 315.3 [M+H]⁺.

Compound **11** was obtained by replacing compound **4-c** with compound **11-b** and referring to the synthesis method of compound **4.** ESI-MS (m/z): 432.3 [M+H]⁺ ;¹H NMR (500 MHz, DMSO-*d*₆) δ 11.49 - 11.29 (m, 1H), 8.67 - 8.38 (m, 2H), 8.38 - 8.28 (m, 1H), 8.16 - 8.08 (m, 1H), 7.90 - 7.82 (m, 1H), 7.52 - 7.43 (m, 1H), 7.41 - 7.33 (m, 1H), 7.28 - 7.18 (m, 1H), 5.32 - 5.13 (m, 1H), 4.87 - 4.57 (m, 3H), 3.85 - 3.72 (m, 1H), 1.24 - 0.97 (m, 6H);

### Example 12:

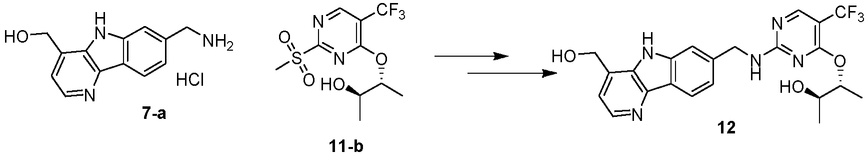

Compound **12** was obtained by replacing compound **2-c** with compound **7-a** and replacing compound **4-c** with compound **11-b.** ESI-MS (m/z): 462.2 [M+H]⁺; ¹H NMR (500 MHz, DMSO-*d*₆) δ 11.24 (s, 1H), 8.74 - 7.02 (m, 7H), 5.57 - 5.45 (m, 1H), 5.30 - 5.12 (m, 1H), 4.91 - 4.57 (m, 5H), 3.85 - 3.69 (m, 1H), 1.24 - 1.20 (m, 1H), 1.12 - 0.94 (m, 5H).

### Example 13:

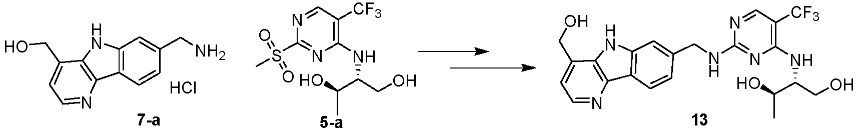

Compound **13** was obtained by replacing compound **2-c** with compound **7-a** and replacing compound **4-c** with compound **5-a,** and referring to the synthesis of compound **4.** ESI-MS (m/z): 477.0 [M+H]⁺; ¹H NMR (500 MHz, DMSO-*d*₆) δ 11.24 (s, 1H), 8.45 - 7.08 (m, 7H), 5.72 - 5.41 (m, 2H), 5.07 - 4.58 (m, 6H), 4.13 - 3.97 (m, 2H), 3.49 - 3.43 (m, 2H), 1.08 - 0.87 (m, 3H).

### Example 14:

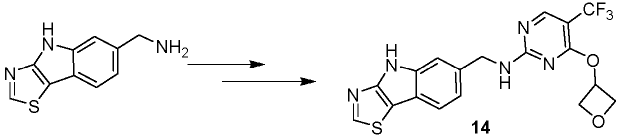

Compound **14** was obtained by replacing compound **1-c** with (4H-thiazolyl[4,5-b]indol-6-yl)methanamine and referring to the synthesis of compound **1.** ESI-MS (m/z): 422.2 [M+H]⁺; ¹H NMR (500 MHz, DMSO-*d*₆) δ 12.08 - 11.97 (m, 1H), 9.07 (s, 1H), 8.74 - 8.38 (m, 2H), 7.79 - 7.71 (m, 1H), 7.46 - 7.39 (m, 1H), 7.16 - 7.09 (m, 1H), 5.66 - 5.55 (m, 1H), 4.90 - 4.73 (m, 2H), 4.71 - 4.54 (m, 3H), 4.46 - 4.43 (m, 1H).

### Example 15:

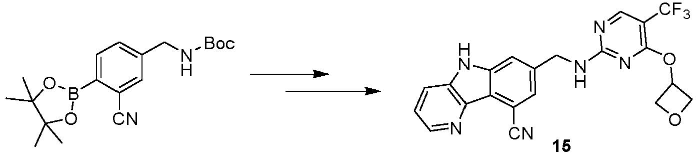

Compound **15** was obtained by replacing tert-butyl (4-(4,4,5,5-tetramethyl-1,3,2-dioxaborolan-2-yl)benzyl)carbamate with tert-butyl (3-cyano-4-(4,4,5,5-tetramethyl-1,3,2-dioxaborolan-2-yl)benzyl)carbamate, and referring to the synthesis of compound 2. ESI-MS (m/z): 441.2 [M+H]⁺; ¹H NMR (500 MHz, DMSO-*d*₆) δ 8.83 - 8.57 (m, 2H), 8.47 - 8.42 (m, 1H), 8.04 - 7.98 (m, 1H), 7.83 - 7.79 (m, 1H), 7.77 - 7.68 (m, 1H), 7.55 - 7.49 (m, 1H), 5.68 - 5.55 (m, 1H), 5.00 - 4.87 (m, 1H), 4.78 - 4.40 (m, 6H).

### Example 16:

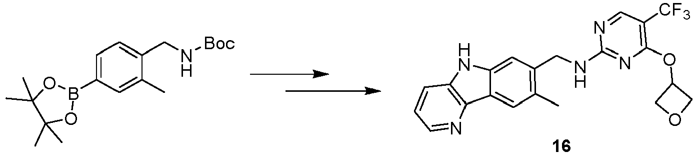

Compound 16 was obtained by replacing tert-butyl (4-(4,4,5,5-tetramethyl-1,3,2-dioxaborolan-2-yl)benzyl)carbamate with tert-butyl (2-methyl-4-(4,4,5,5-tetramethyl-1,3,2-dioxaborolan-2-yl)benzyl)carbamate and referring to the synthesis of compound **2.** ESI-MS (m/z): 430.2 [M+H]⁺; ¹H NMR (500 MHz, DMSO-*d*₆) δ 11.49 - 11.06 (m, 1H), 8.83 - 6.98 (m, 7H), 5.78 - 5.36 (m, 1H), 5.04 - 4.17 (m, 6H), 2.58 - 2.54 (m, 3H).

### Example 17:

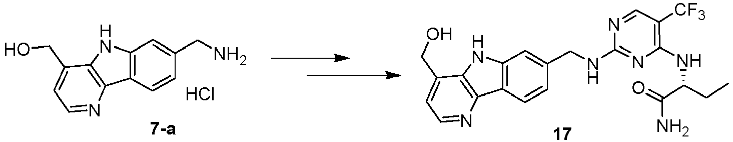

Compound **17** was obtained by replacing compound **2-c** with compound **7-a** and referring to the synthesis method of compound 4. ESI-MS (m/z): 474.2 [M+H]⁺; ¹H NMR (500 MHz, DMSO-*d*₆) δ 11.33 (s, 1H), 8.52 - 8.41 (m, 1H), 8.31 - 8.12 (m, 3H), 7.75 - 7.24 (m, 5H), 6.44 - 6.27 (m, 1H), 5.73 - 5.53 (m, 1H), 5.01 - 4.92 (m, 2H), 4.84 - 4.63 (m, 3H), 2.01 - 1.67 (m, 2H), 0.91 - 0.71 (m, 3H).

### Example 18:

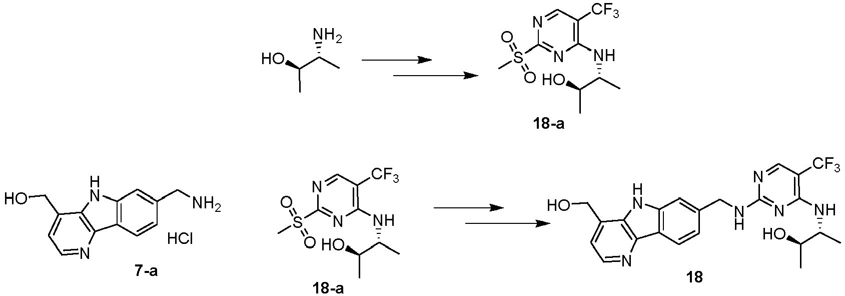

Using (2R,3R)-3-aminobutan-2-ol instead of (R)-2-aminobutanamide hydrochloride, compound **18-a** was obtained by referring to the synthesis of compound **4-c.** Compound **18** was obtained by replacing compound **4-c** with compound 18-a and replacing compound **2-c** with compound **7-a**, and referring to the synthesis of compound **4.** ESI-MS (m/z): 461.5 [M+H]⁺; ¹H NMR (500 MHz, DMSO-*d*₆) δ 11.25 (s, 1H), 8.39 (d, *J =* 4.7 Hz, 1H), 8.17 - 7.89 (m, 3H), 7.51 - 7.44 (m, 1H), 7.40 - 7.32 (m, 1H), 7.24 - 7.13 (m, 1H), 5.80 - 5.63 (m, 1H), 5.62 - 5.46 (m, 1H), 5.11 - 4.94 (m, 1H), 4.94 - 4.84 (m, 2H), 4.74 - 4.55 (m, 2H), 4.17 - 3.99 (m, 1H), 3.83 - 3.57 (m, 1H), 1.21 - 0.86 (m, 6H).

### Example 19:

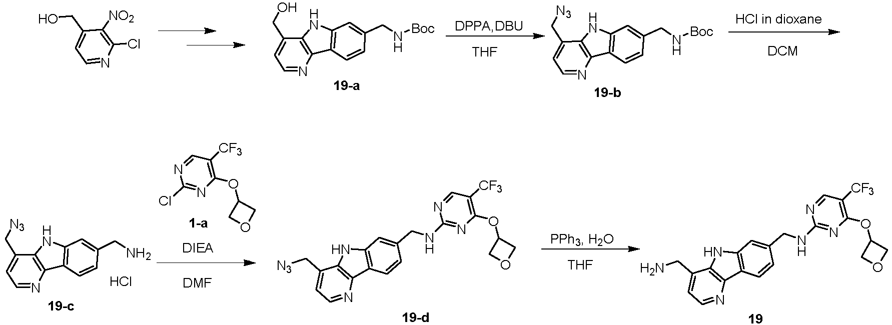

Using (2-chloro-3-nitropyridin-4-yl)methanol instead of 2-chloro-3-nitropyridine, compound **19-a** was obtained by referring to the synthesis of compound **2-b.**

Compound **19-a** (1.08 g, 3.29 mmol) was dissolved in tetrahydrofuran (20 mL), and 1,8-diazabicyclo[5.4.0]undec-7-ene (601 mg, 3.95 mmol) and diphenylphosphoryl azide (1.09 g, 3.95 mmol) were added. The mixture was stirred at room temperature for 5 hours. Add 50 mL of ethyl acetate, wash once with water, once with 5% dilute hydrochloric acid, once with saturated brine, dry over anhydrous sodium sulfate, filter, and concentrate. The residue was purified by column chromatography to obtain compound **19-b** (1.1 g, yield 94.9 %). ESI-MS (m/z): 353.4 [M+H]⁺. Compound **19-b** (280 mg, 795 µmol) was dissolved in dichloromethane (5 mL), and a dioxane hydrochloride solution (4 M, 2 mL) was added. The mixture was stirred at room temperature for 5 hours and concentrated to obtain compound **19-c** (229 mg, yield 99.9 %). ESI-MS (m/z): 253.7 [M+H]⁺.

Compound **19-c** (50 mg, 173 µmol) and compound **1-a** (44.1 mg, 173 µmol) were dissolved in N,N-dimethylformamide (5 mL), and N,N-diisopropylethylamine (112 mg, 865 mmol) was added. The mixture was stirred at room temperature for 2 hours. Water (10 mL) was added to the reaction solution, and the mixture was extracted three times with ethyl acetate (20 mL). The organic phases were combined, washed once with water and once with saturated brine, dried over anhydrous sodium sulfate, filtered, and concentrated. The residue was purified by flash column chromatography to obtain compound **19-d** (50 mg, yield 61.4 %). ESI-MS (m/z): 471.3 [M+H]⁺.

Compound **19-d** (50 mg, 106 µmol) was dissolved in tetrahydrofuran (20 mL), and triphenylphosphine (83.6 mg, 319 µmol) and water (0.1 mL) were added. The mixture was stirred at room temperature for 16 hours. The reaction solution was concentrated, and the residue was purified by preparative liquid chromatography to obtain compound **19** (10 mg, yield 21.1 %). ESI-MS (m/z): 445.4 [M+H]⁺; ¹H NMR (500 MHz, DMSO-*d*₆) δ 8.87 - 8.34 (m, 4H), 8.15 - 8.05 (m, 1H), 7.49 - 7.38 (m, 2H), 7.26 - 7.16 (m, 1H), 5.68 - 5.53 (m, 1H), 4.94 - 4.39 (m, 7H), 4.16 (s, 2H).

### Example 20:

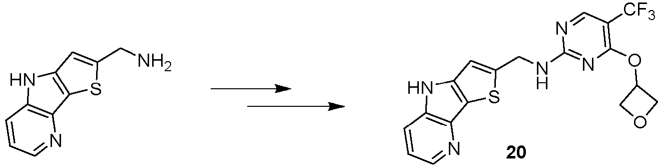

Compound **20** was obtained by replacing compound **1-c** with (4H-thieno[2',3':4,5]pyrrolo[3,2-b]pyridin-2-yl)methanamine and referring to the synthesis of compound 1. ESI-MS (m/z): 422.2 [M+H]⁺; ¹H NMR (500 MHz, DMSO-*d*₆) δ 11.56 - 11.47 (m, 1H), 8.79 - 8.52 (m, 1H), 8.52 - 8.41 (m, 1H), 8.37 - 8.28 (m, 1H), 7.94 - 7.76 (m, 1H), 7.22 - 7.15 (m, 2H), 5.76 - 5.60 (m, 1H), 4.95 - 4.79 (m, 3H), 4.76 - 4.73 (m, 1H), 4.60 - 4.52 (m, 2H).

### Example 21:

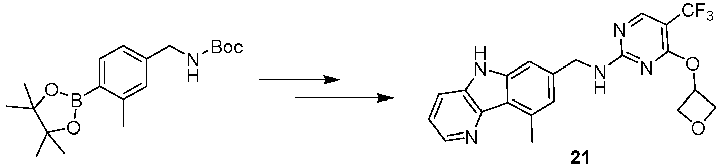

Compound **21** was obtained by replacing tert-butyl (4-(4,4,5,5-tetramethyl-1,3,2-dioxaborolan-2-yl)benzyl)carbamate with tert-butyl (3-methyl-4-(4,4,5,5-tetramethyl-1,3,2-dioxaborolan-2-yl)benzyl)carbamate and referring to the synthesis of compound 2. ESI-MS (m/z): 430.2 [M+H]⁺; ¹H NMR (500 MHz, DMSO-*d*₆) δ 11.40 - 11.30 (m, 1H), 8.76 - 8.39 (m, 3H), 7.88 - 7.80 (m, 1H), 7.37 - 7.31 (m, 1H), 7.30 - 7.24 (m, 1H), 7.04 - 6.95 (m, 1H), 5.69 - 5.55 (m, 1H), 4.92 - 4.71 (m, 2H), 4.69 - 4.42 (m, 4H), 2.98 - 2.91 (m, 3H).

### Example 22:

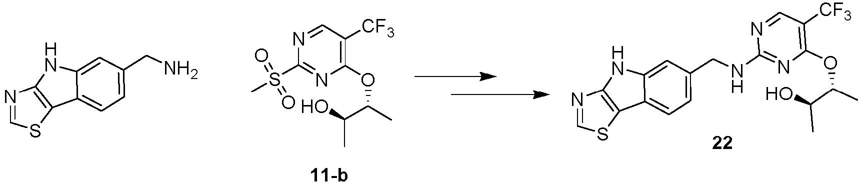

Compound 22 was obtained by replacing compound **2-c** with (4H-thiazo[4,5-b]indol-6-yl)methanamine and replacing compound **4-c** with compound **11-b.** The synthesis of compound **4** was referred to. ¹H NMR (500 MHz, DMSO-*d*₆) δ 12.08 - 11.98 (m, 1H), 9.07 (s, 1H), 8.61 - 8.37 (m, 1H), 8.36 - 8.27 (m, 1H), 7.80 - 7.71 (m, 1H), 7.51 - 7.41 (m, 1H), 7.18 - 7.10 (m, 1H), 5.28 - 5.16 (m, 1H), 4.83 - 4.74 (m, 1H), 4.72 - 4.54 (m, 2H), 3.82 - 3.73 (m, 1H), 1.22 - 0.98 (m, 6H); ESI-MS (m/z): 438.3 [M+H]⁺.

### Example 23:

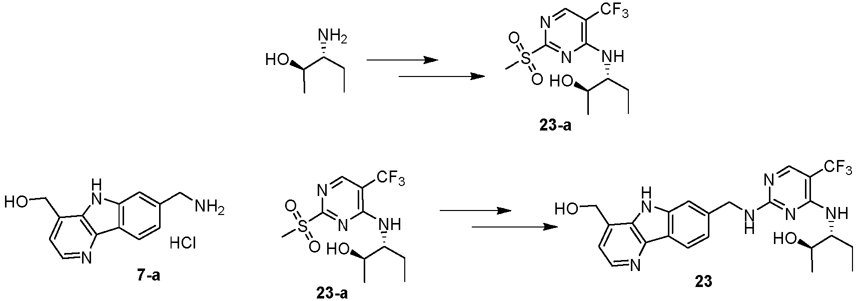

Using (2R,3R)-3-aminopentan-2-ol instead of (R)-2-aminobutanamide hydrochloride, compound **23-a** was obtained by referring to the synthesis of compound **4-c.** Compound **23** was obtained by replacing compound **4-c** with compound **23-a** and replacing compound **2-c** with compound **7-a,** and referring to the synthesis of compound **4.** ESI-MS (m/z): 475.3 [M+H]⁺; ¹H NMR (500 MHz, DMSO-*d*₆) δ 11.33 - 11.19 (m, 1H), 8.45 - 8.36 (m, 1H), 8.17 - 7.73 (m, 3H), 7.50 - 7.43 (m, 1H), 7.42 - 7.33 (m, 1H), 7.25 - 7.15 (m, 1H), 5.69 - 5.57 (m, 1H), 5.57 - 5.49 (m, 1H), 5.05 - 4.84 (m, 3H), 4.75 - 4.53 (m, 2H), 4.06 - 3.90 (m, 1H), 3.89 - 3.69 (m, 1H), 2.03 - 1.39 (m, 3H), 0.85 - 0.80 (m, 2H), 0.69 - 0.62 (m, 3H).

### Example 24:

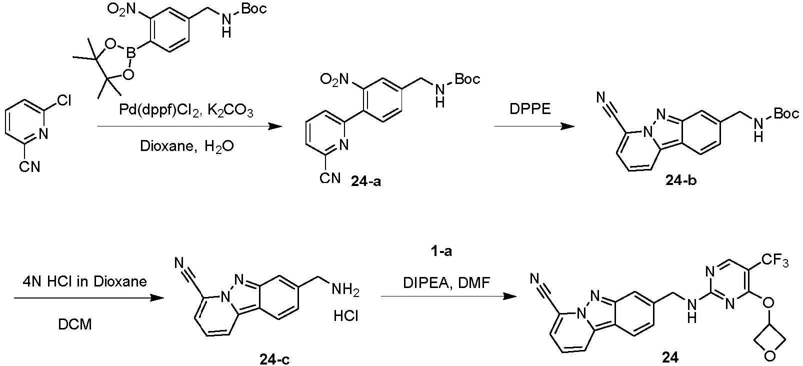

6-Chloropyridine-2-carbonitrile (1 g, 7.22 mmol) and tert-butyl (3-nitro-4-(4,4,5,5-tetramethyl-1,3,2-dioxaborolan-2-yl)benzyl)carbamate (3.00 g, 7.94 mmol) were dissolved in a mixture of 1,4-dioxane (5 mL) and water (0.5 mL). 1,1'-Bis(diphenylphosphino)ferrocenepalladium(II) chloride (528 mg, 722 µmol) and potassium carbonate (2.99 g, 21.7 mmol) were added. The mixture was stirred at 100°C for 16 hours. The reaction solution was cooled to room temperature, 80 mL of ethyl acetate was added, washed with water three times and saturated brine once, dried over anhydrous sodium sulfate, filtered and concentrated. The residue was purified by column chromatography to obtain compound **24-a** (2 g, yield 78.2%). ESI-MS (m/z): 355.3 [M+H]⁺.

Compound **24-a** (2 g, 5.64 mmol) and 1,2-bis(diphenylphosphino)ethane (6.75 g, 16.9 mmol) were added to a flask. The mixture was reacted at 180°C for 0.5 hour. The mixture was cooled to room temperature and purified by column chromatography to obtain compound **24-b** (200 mg, yield 11.0%). ESI-MS (m/z): 323.3 [M+H]⁺.

Compound **24-b** (200 mg, 620 µmol) was dissolved in dichloromethane (10 mL), and dioxane hydrochloride solution (4 M, 2 mL) was added. The mixture was stirred at room temperature for 2 hours. The reaction solution was concentrated to obtain **24-c** (160 mg, yield 100%). ESI-MS (m/z): 223.3 [M+H]⁺.

Compound **24-c** (30.5 mg, 118 µmol) and compound **1-a** (30 mg, 118 µmol) were dissolved in N,N-dimethylformamide (2 mL), and N,N-diisopropylethylamine (45.7 mg, 353 µmol) was added. The mixture was stirred at 80°C for 2 hours. After the reaction solution was cooled to room temperature, it was purified by preparative liquid chromatography to obtain compound **24** (12 mg, yield 23.1%). ESI-MS (m/z): 441.3 [M+H]⁺; ¹H NMR (500 MHz, DMSO-*d*₆) δ 8.85 - 8.51 (m, 2H), 8.45 - 8.11 (m, 3H), 7.83 - 7.24 (m, 3H), 5.71 - 5.45 (m, 1H), 4.93 - 4.65 (m, 4H), 4.63 - 4.35 (m, 2H).

### Example 25:

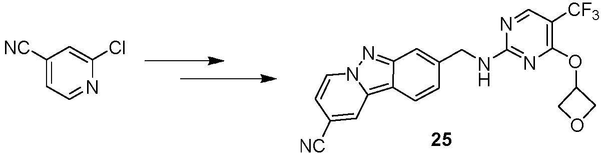

Compound **25** was obtained by replacing 6-chloropyridinecarbonitrile with 2-chloroisonicotinonitrile and referring to the synthesis method of compound **24.** ESI-MS (m/z): 441.3 [M+H]⁺; ¹H NMR (500 MHz, DMSO-*d*₆) δ 9.27 - 9.09 (m, 2H), 8.86 - 8.24 (m, 3H), 7.84 - 7.29 (m, 3H), 5.77 - 5.41 (m, 1H), 4.92 - 4.65 (m, 4H), 4.61 - 4.36 (m, 2H).

### Example 26:

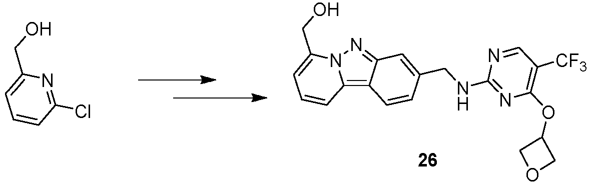

Compound **26** was obtained by replacing 6-chloropyridinecarbonitrile with 6-chloropyridine-2-methanol and referring to the synthesis method of compound **24.** ESI-MS (m/z): 446.1 [M+H]⁺; ¹H NMR (500 MHz, DMSO-*d*₆) δ 8.80 - 8.48 (m, 1H), 8.44 - 8.34 (m, 2H), 8.29 - 8.20 (m, 1H), 7.66 - 7.41 (m, 3H), 7.23 - 7.15 (m, 1H), 5.90-5.80 (m, 1H), 5.68 - 5.48 (m, 1H), 5.13 - 5.03 (m, 2H), 4.92 - 4.63 (m, 4H), 4.61 - 4.33 (m, 2H).

### Example 27:

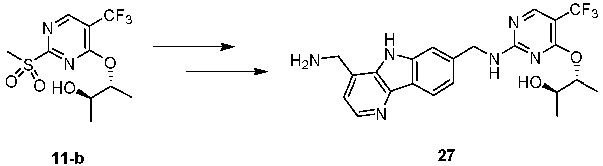

Compound 27 was obtained by replacing compound 1-a with compound 11-b and referring to the synthesis method of compound 19. ESI-MS (m/z): 461.3 [M+H]⁺; ¹H NMR (500 MHz, DMSO-*d*₆) δ 12.13 - 11.57 (m, 1H), 8.74 - 8.04 (m, 4H), 7.56 - 7.16 (m, 3H), 5.36 - 5.16 (m, 1H), 4.80 - 4.38 (m, 4H), 3.85 - 3.68 (m, 2H), 1.23 - 0.95 (m, 6H).

### Example 28:

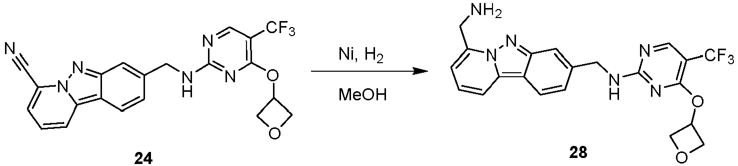

Compound 24 (10 mg, 23 µmol) was dissolved in methanol (2 mL) and Raney nickel (10 mg) was added. The mixture was stirred under hydrogen atmosphere for 16 hours. The reaction solution was filtered through celite and concentrated. The residue was purified by preparative liquid chromatography to obtain compound **28** (3 mg, yield 30%). ESI-MS (m/z): 445.3 (M+H)⁺; ¹H NMR (500 MHz, DMSO-*d*₆) δ 8.82 - 8.14 (m, 6H), 7.72 - 7.45 (m, 3H), 7.25 - 7.16 (m, 1H), 5.71 - 5.50 (m, 1H), 4.94 - 4.84 (m, 1H), 4.76 - 4.58 (m, 4H), 4.41 - 4.32 (m, 3H).

### Example 29:

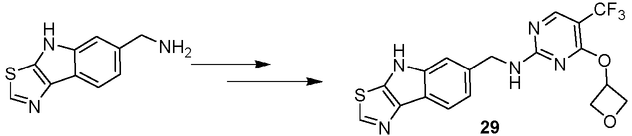

Compound 29 was obtained by replacing compound **1-c** with (4H-thiazolyl[5,4-b]indol-6-yl)methanamine and referring to the synthesis of compound **1.** ESI-MS (m/z): 422.2 [M+H]⁺; ¹H NMR (500 MHz, DMSO-*d*₆) δ 11.67 (s, 1H), 8.87 - 8.32 (m, 3H), 7.87 - 7.76 (m, 1H), 7.50 - 7.38 (m, 1H), 7.19 - 7.09 (m, 1H), 5.69 - 5.54 (m, 1H), 4.93 - 4.71 (m, 2H), 4.70 - 4.42 (m, 4H).

### Example 30:

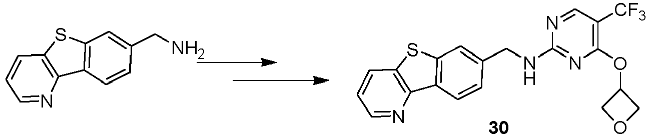

Compound 30 was obtained by replacing compound **1-c** with (benzo[4,5]thieno[3,2-b]pyridin-7-yl)methanamine and referring to the synthesis of compound 1. ESI-MS (m/z): 433.5 [M+H]⁺; ¹H NMR (500 MHz, DMSO-*d*₆) δ 8.81 - 8.70 (m, 2H), 8.54 - 8.49 (m, 1H), 8.47 - 8.41 (m, 1H), 8.40 - 8.34 (m, 1H), 8.06 - 7.98 (m, 1H), 7.57 - 7.49 (m, 2H), 5.71 - 5.53 (m, 1H), 4.92 - 4.37 (m, 6H).

### Example 31:

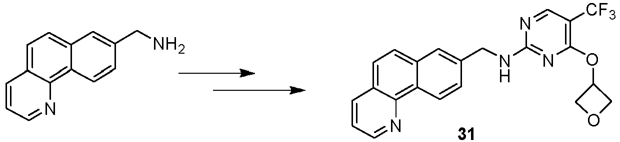

Compound **31** was obtained by replacing compound **1-c** with (benzo[h]quinolin-8-yl)methanamine and referring to the synthesis of compound **1.** ESI-MS (m/z): 427.5 [M+H]⁺; ¹H NMR (500 MHz, DMSO-*d*₆) δ 9.20 - 9.11 (m, 1H), 9.03 - 8.98 (m, 1H), 8.82 - 8.53 (m, 1H), 8.47 - 8.39 (m, 2H), 7.98 - 7.85 (m, 3H), 7.75 - 7.70 (m, 1H), 7.70 - 7.65 (m, 1H), 5.74 - 5.50 (m, 1H), 4.90 - 4.31 (m, 6H).

### Example 32:

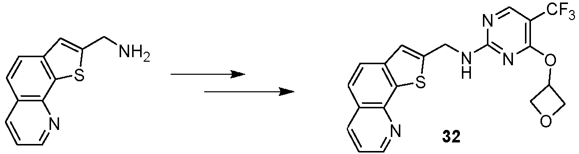

Compound 32 was obtained by replacing compound 1-c with (thieno[3,2-H]quinolin-2-yl)methanamine and referring to the synthesis of compound 1. ESI-MS (m/z): 433.4 [M+H]⁺; ¹H NMR (500 MHz, DMSO-*d*₆) δ 8.92 - 8.42 (m, 4H), 7.99 - 7.92 (m, 1H), 7.88 - 7.81 (m, 1H), 7.62 - 7.55 (m, 1H), 7.55 - 7.48 (m, 1H), 5.73 - 5.61 (m, 1H), 4.92 - 4.79 (m, 4H), 4.62 - 4.47 (m, 2H).

### Example 33:

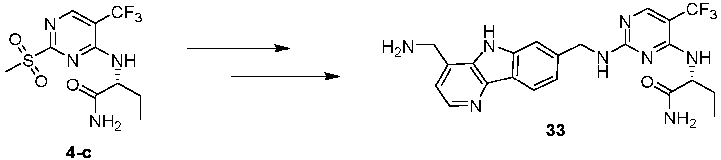

Compound 33 was obtained by replacing compound 1-a with compound 4-c and referring to the synthesis of compound 19. ESI-MS (m/z): 473.7 [M+H]⁺; ¹H NMR (400 MHz, DMSO-*d*₆) δ 8.39 (d, *J =* 4.8 Hz, 1H), 8.30 (s, 1H), 8.18 - 8.11 (m, 1H), 8.11 - 8.06 (m, 1H), 7.68 - 7.62 (m, 2H), 7.52 - 7.43 (m, 1H), 7.40 (d, *J =* 4.8 Hz, 1H), 7.36 - 7.28 (m, 1H), 7.26 - 7.15 (m, 1H), 6.26 (d, *J =* 6.9 Hz, 1H), 4.78 - 4.55 (m, 3H), 4.16 (s, 2H), 2.06 - 1.61 (m, 2H), 0.84 - 0.62 (m, 3H).

### Example 34:

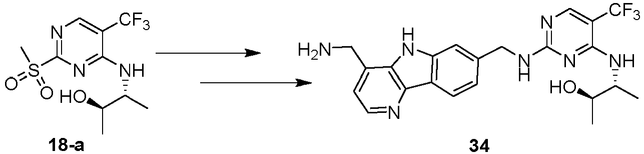

Compound **34** was obtained by replacing compound **1-a** with compound **18-a** and referring to the synthesis of compound **19.** ESI-MS (m/z): 460.6 [M+H]⁺; ¹H NMR (400 MHz, DMSO-*d*₆) δ 8.36 (d, *J =* 4.7 Hz, 1H), 8.10 (br.s, 1H), 8.08 - 8.02 (m, 2H), 7.45 (d, *J* = 5.2 Hz, 1H), 7.38 (d, *J =* 4.8 Hz, 1H), 7.23 - 7.11 (m, 1H), 5.75 - 5.65 (m, 1H), 5.04 (br.s, 1H), 4.73 - 4.52 (m, 2H), 4.15 - 3.96 (m, 3H), 3.78 - 3.57 (m, 1H), 1.48 - 0.78 (m, 6H).

### Example 35:

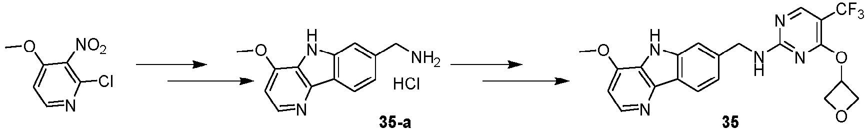

Using 2-chloro-4-methoxy-3-nitropyridine instead of 2-chloro-3-nitropyridine, compound **35-a** was obtained by referring to the synthesis of compound 2-c. Compound **35** was obtained by replacing compound **2-c** with compound **35-a** and referring to the synthesis method of compound **2.** ESI-MS (m/z): 446.5 [M+H]⁺; ¹H NMR (500 MHz, DMSO-*d*₆) δ 11.54 - 11.39 (m, 1H), 8.80 - 8.47 (m, 1H), 8.45 - 8.38 (m, 1H), 8.31 (d, *J* = 5.3 Hz, 1H), 8.09 - 8.03 (m, 1H), 7.43 (s, 1H), 7.18 (dd, *J =* 8.5, 4.6 Hz, 1H), 7.04 (d, *J =* 5.4 Hz, 1H), 5.68 - 5.54 (m, 1H), 4.93 - 4.40 (m, 6H), 4.04 (s, 3H).

### Example 36:

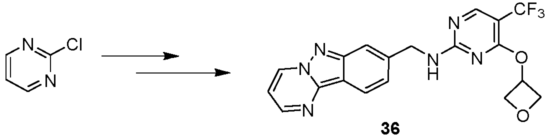

Compound **36** was obtained by replacing 6-chloropyrimidine with 2-chloropyrimidine and referring to the synthesis method of compound 24. ESI-MS (m/z): 417.6 [M+H]⁺; ¹H NMR (500 MHz, DMSO-*d*₆) δ 9.48 - 9.38 (m, 1H), 8.83 - 8.46 (m, 2H), 8.46 - 8.15 (m, 2H), 7.66 (s, 1H), 7.53 - 7.40 (m, 1H), 7.35 - 7.21 (m, 1H), 5.71 - 5.50 (m, 1H), 4.92 - 4.33 (m, 6H).

### Example 37:

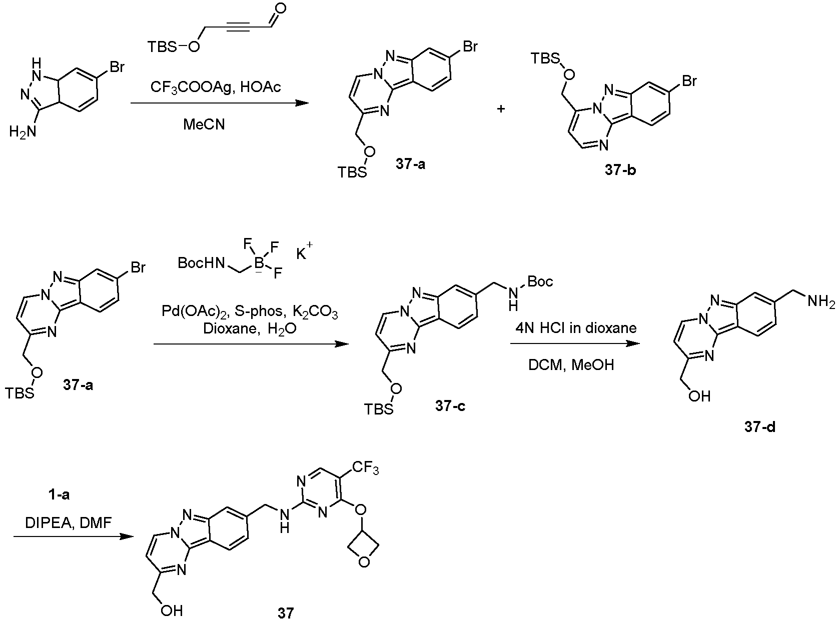

6-Bromo-1H-indazol-3-amine (2 g, 9.43 mmol) and 4-[tert-butyl(dimethyl)]silanyl]oxybut-2-ynal (6.2 g, 31.3 mmol) were dissolved in acetonitrile (6 mL), and glacial acetic acid (566 mg, 9.43 mmol) and silver trifluoroacetate (625 mg, 2.83 mmol) were added. The mixture was stirred at room temperature for 16 hours. Add 80 mL of ethyl acetate and 50 mL of water, filter through celite, separate the layers, wash the organic phase once with water and once with saturated brine, dry over anhydrous sodium sulfate, filter, and concentrate. The residue was purified by column chromatography to obtain compound **37-a** (700 mg, yield 18.9%) and compound **37-b** (700 mg, yield 18.9%). ESI-MS (m/z): 392.4 [M+H]⁺.

Compound 37-a (700 mg, 1.78 mmol) and potassium [(tert-butoxycarbonylamino)methyl]trifluoroborate (383 mg, 1.62 mmol) were dissolved in a mixture of 1,4-dioxane (6 mL) and water (1 mL), and S-phos (73.2 mg, 178 µmol), potassium carbonate (740 mg, 5.35 mmol) and palladium acetate (40 mg, 178 µmol) were added. The mixture was stirred at 90°C for 16 hours under nitrogen protection. The reaction solution was cooled to room temperature, 50 mL of ethyl acetate and 30 mL of water were added, and the mixture was filtered through celite. The organic phase was washed once with water and once with saturated brine, dried over anhydrous sodium sulfate, filtered, and concentrated. The residue was purified by column chromatography to obtain compound **37-c** (400 mg, yield 18.9%). ESI-MS (m/z): 443.4 [M+H]⁺.

Compound **37-c** (90 mg, 203 µmol) was dissolved in a mixture of methanol (1 mL) and dichloromethane (5 mL), and a dioxane hydrochloride solution (4 M, 1 mL) was added. The mixture was stirred at room temperature for 16 hours. The reaction solution was concentrated to obtain compound **37-d** (53 mg, yield 98.5%). ESI-MS (m/z): 229.4 (M+H)⁺.

Compound **37-d** (10 mg, 38 µmol) and compound **1-a** (9.62 mg, 38 µmol) were dissolved in N,N-dimethylformamide (2 mL), and N,N-diisopropylethylamine (24.4 mg, 189 µmol) was added. The mixture was stirred at 80°C for 2 hours. After the reaction solution was cooled to room temperature, it was purified by preparative liquid chromatography to obtain compound 37 (3.5 mg, yield 20.8%). ESI-MS (m/z): 447.5 [M+H]⁺; ¹H NMR (500 MHz, DMSO-*d*₆) δ 9.46 - 9.30 (m, 1H), 8.81 - 8.47 (m, 1H), 8.47 - 8.41 (m, 1H), 8.23 - 8.07 (m, 1H), 7.62 (s, 1H), 7.58 - 7.51 (m, 1H), 7.28 - 7.16 (m, 1H), 5.89 - 5.71 (m, 1H), 5.72 - 5.51 (m, 1H), 4.93 - 4.32 (m, 8H).

### Example 38:

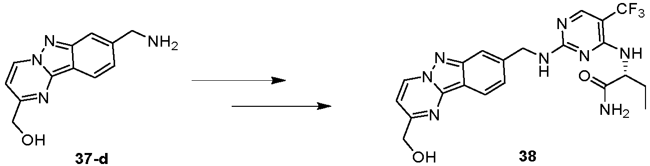

Compound **37-d** was used to replace compound **2-c,** and compound **38** was obtained by referring to the synthesis method of compound **4.** ESI-MS (m/z): 475.7 [M+H]⁺; ¹H NMR (500 MHz, DMSO-*d*₆) δ 9.42 - 9.30 (m, 1H), 8.20 - 7.86 (m, 3H), 7.73 - 7.56 (m, 2H), 7.56 - 7.47 (m, 1H), 7.41 - 7.18 (m, 2H), 6.34 - 6.17 (m, 1H), 5.86 - 5.74 (m, 1H), 4.85 - 4.54 (m, 5H), 1.86 - 1.60 (m, 2H), 0.85 - 0.58 (m, 3H).

### Example 39:

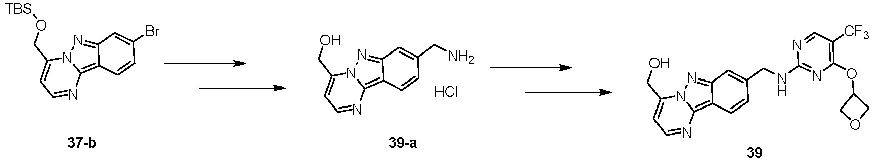

Compound **37-b** was used to replace compound **37-a,** and compound **39-a** was obtained by referring to the synthesis of compound **37-d.** Compound **39** was obtained by referring to the synthesis of compound **37.** ESI-MS (m/z): 447.6 [M+H]⁺; ¹H NMR (500 MHz, DMSO-*d*₆) δ 8.95 - 8.10 (m, 4H), 7.66 (s, 1H), 7.61 - 7.54 (m, 1H), 7.36 - 7.22 (m, 1H), 6.14 - 6.04 (m, 1H), 5.70 - 5.44 (m, 1H), 5.23 - 4.28 (m, 8H).

### Example 40:

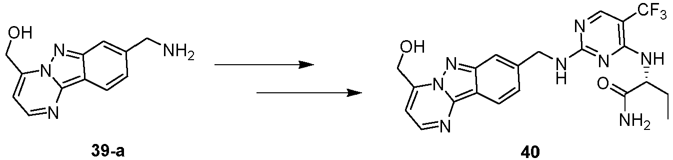

Compound **40** was obtained by replacing compound 2-c with compound **39-a** and referring to the synthesis method of compound **4.** ESI-MS (m/z): 475.6 [M+H]⁺; ¹H NMR (500 MHz, DMSO-*d*₆) δ 8.82 - 8.52 (m, 1H), 8.26 - 7.88 (m, 3H), 7.77 - 7.52 (m, 3H), 7.40 - 7.23 (m, 2H), 6.33 - 6.20 (m, 1H), 5.17 - 5.07 (m, 2H), 4.81 - 4.53 (m, 3H), 1.79 - 1.63 (m, 2H), 0.79 - 0.64 (m, 3H).

### Example 41:

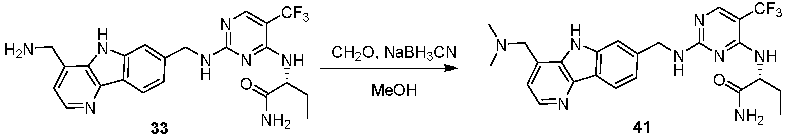

Compound **33** (5 mg, 11 µmol) was dissolved in methanol (2 mL), and aqueous formaldehyde solution (34%, 0.05 mL) was added. The mixture was stirred at room temperature for 1 hours. Sodium cyanoborohydride (1.33 mg, 21.17 µmol) was added. The mixture was stirred at room temperature overnight. The reaction solution was quenched with water (0.5 mL) and concentrated. The residue was purified by preparative liquid chromatography to obtain compound **41** (1 mg, yield 18.3%). ESI-MS (m/z): 501.5 [M+H]⁺; ¹H NMR (500 MHz, DMSO-*d*₆) δ 11.14 (s, 1H), 8.36 (d, *J =* 4.7 Hz, 1H), 8.17 - 8.04 (m, 3H), 7.68 - 7.46 (m, 2H), 7.37 - 7.12 (m, 3H), 6.32 - 6.21 (m, 1H), 4.77 - 4.49 (m, 3H), 3.74 (s, 2H), 2.23 (s, 6H)1.85 - 1.60 (m, 2H), 0.95 - 0.57 (m, 3H).

### Example 42:

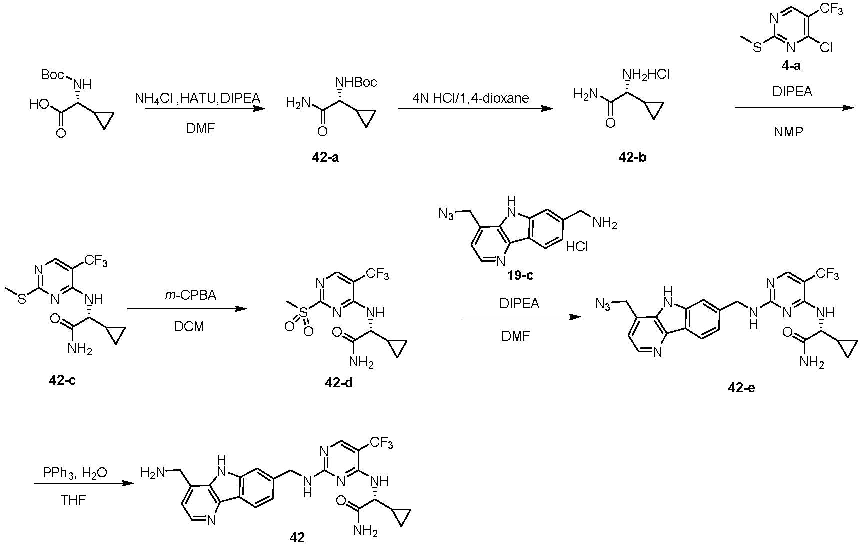

(R)-2-((tert-Butyloxycarbonyl)amino)-2-cyclopropylacetic acid (200 mg, 929 µmol), N,N-diisopropylethylamine (480 mg, 3.72 mmol) and ammonium chloride (149 mg, 2.79 mmol) were dissolved in N,N-dimethylformamide (5.0 mL) and HATU (530 mg, 1.39 mmol) was added. The mixture was stirred at room temperature for 2 hours. Water (10 mL) was added to the reaction solution, and the mixture was extracted three times with ethyl acetate (20 mL). The organic phases were combined, washed once with water and once with saturated brine, dried over anhydrous sodium sulfate, filtered, and concentrated. The residue was purified by flash column chromatography to obtain compound **42-a** (160 mg, yield 80.4%).

Compound **42-a** (160 mg, 746 µmol) was dissolved in dichloromethane (2 mL), and dioxane hydrochloride solution (4 M, 1 mL) was added. The reaction solution was stirred at room temperature for 1 hour. The reaction solution was concentrated to obtain compound **42-b** (112 mg, yield 99.6%).

Compound **42-b** (100 mg, 664 µmol) and compound **4-a** (152 mg, 664 µmol) were dissolved in N,N-dimethylformamide (5 mL), and N,N-diisopropylethylamine (257 mg, 1.99 mmol) was added. The mixture was stirred at 70°C for 2 hours. The reaction solution was cooled to room temperature, water (10 mL) was added, and the mixture was extracted three times with ethyl acetate (20 mL). The organic phases were combined, washed once with water and once with saturated brine, dried over anhydrous sodium sulfate, filtered, and concentrated. The residue was purified by flash column chromatography to obtain compound **42-c** (154 mg, yield 75.7%). ESI-MS (m/z): 307.3 [M+H]⁺.

Compound **42-c** (154 mg, 503 µmol) was dissolved in dichloromethane (10 mL), and m-chloroperbenzoic acid (174 mg, 1.01 mmol) was added. The mixture was stirred at room temperature for 2 hours. The reaction solution was concentrated, and the residue was purified by column chromatography to obtain compound **42-d** (140 mg, yield 82.3%). ESI-MS (m/z): 339.3 [M+H]⁺.

Compound **42-d** (96.1 mg, 284 µmol) and compound **19-c** (71.7 mg, 284 µmol) were dissolved in N,N-dimethylformamide (5 mL), and N,N-diisopropylethylamine (110 mg, 852 mmol) was added. The mixture was stirred at room temperature for 2 hours. Water (10 mL) was added to the reaction solution, and the mixture was extracted three times with ethyl acetate (20 mL). The organic phases were combined, washed once with water and once with saturated brine, dried over anhydrous sodium sulfate, filtered, and concentrated. The residue was purified by flash column chromatography to give compound 42-e (45 mg, yield 31.0%). ESI-MS (m/z): 511.5 [M+H]⁺.

Compound **42-e** (45 mg, 88 µmol) was dissolved in tetrahydrofuran (5 mL), and triphenylphosphine (69.4 mg, 264 µmol) and water (0.1 mL) were added. The mixture was stirred at room temperature for 16 hours. The reaction solution was concentrated, and the residue was purified by preparative liquid chromatography to give compound 42 (15 mg, yield 35.1 %). ESI-MS (m/z): 485.7 [M+H]⁺; ¹H NMR (500 MHz, DMSO-*d*₆) δ 8.39 (d, *J =* 4.7 Hz, 1H), 8.16 - 7.81 (m, 3H), 7.63 - 7.42 (m, 2H), 7.40 (d, *J =* 4.8 Hz, 1H), 7.33 - 7.13 (m, 2H), 6.27 - 6.13 (m, 1H), 4.82 - 4.51 (m, 2H), 4.32 - 4.10 (m, 3H), 1.31 - 0.91 (m, 1H), 0.64 - 0.09 (m, 4H).

### Example 43:

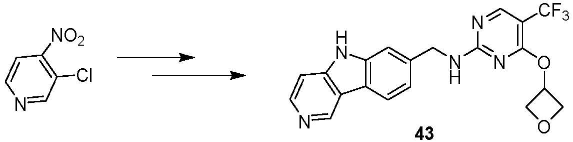

By replacing 2-chloro-3-nitropyridine with 3-chloro-4-nitropyridine, compound **43** was obtained by referring to the synthesis method of compound **2.** ESI-MS (m/z): 416.6 [M+H]⁺; ¹H NMR (500 MHz, DMSO-*d*₆) δ 11.65 (s, 1H), 9.28 (s, 1H), 8.79 - 8.45 (m, 1H), 8.41 - 8.36 (m, 1H), 8.24 - 8.11 (m, 2H), 7.49 - 7.41 (m, 2H), 7.28 - 7.18 (m, 1H), 5.67 - 5.51 (m, 1H), 4.93 - 4.84 (m, 1H), 4.75 - 4.68 (m, 2H), 4.66 - 4.56 (m, 2H), 4.44 - 4.41 (m, 1H).

### Example 44:

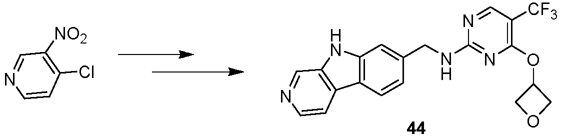

By replacing 2-chloro-3-nitropyridine with 4-chloro-3-nitropyridine, compound **44** was obtained by referring to the synthesis method of compound 2. ESI-MS (m/z): 416.6 [M+H]⁺; ¹H NMR (500 MHz, DMSO-*d*₆) δ 11.61 - 11.48 (m, 1H), 8.93 - 8.81 (m, 1H), 8.82 - 8.48 (m, 1H), 8.47 - 8.38 (m, 1H), 8.36 - 8.28 (m, 1H), 8.20 - 8.11 (m, 1H), 8.09 - 8.02 (m, 1H), 7.51 - 7.46 (m, 1H), 7.25 - 7.16 (m, 1H), 5.69 - 5.51 (m, 1H), 4.93 - 4.83 (m, 1H), 4.76 - 4.68 (m, 2H), 4.66 - 4.56 (m, 2H), 4.43 - 4.40 (m, 1H).

### Example 45:

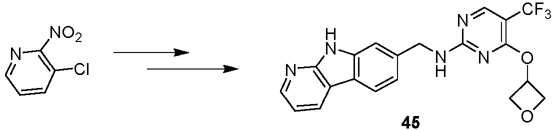

By replacing 2-chloro-3-nitropyridine with 3-chloro-2-nitropyridine, compound **45** was obtained by referring to the synthesis method of compound 2. ESI-MS (m/z): 416.6 [M+H]⁺; ¹H NMR (500 MHz, DMSO-*d*₆) δ 11.76 - 11.67 (m, 1H), 8.81 - 8.48 (m, 1H), 8.14 - 8.06 (m, 1H), 7.78 - 7.73 (m, 1H), 7.60 - 7.46 (m, 2H), 7.44 - 7.37 (m, 1H), 7.25 - 7.14 (m, 2H), 5.71 - 5.50 (m, 1H), 4.93 - 4.84 (m, 1H), 4.76 - 4.67 (m, 2H), 4.65 - 4.55 (m, 2H), 4.46 - 4.39 (m, 1H).

### Example 46:

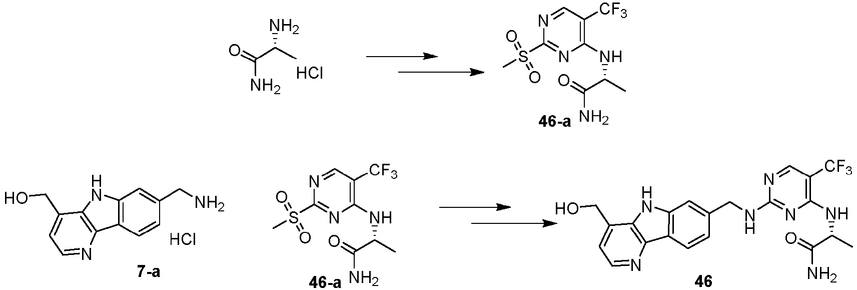

(R)-2-aminopropanamide hydrochloride was used instead of (R)-2-aminobutanamide hydrochloride, and compound **46-a** was obtained by referring to the synthesis of compound **4-c.** Compound **46** was obtained by replacing compound **4-c** with compound **46-a** and replacing compound **2-c** with compound **7-a,** and referring to the synthesis of compound **4.** ESI-MS (m/z): 460.7 [M+H]⁺; ¹H NMR (500 MHz, DMSO-*d*₆) δ 11.23 (s, 1H), 8.48 - 8.30 (m, 1H), 8.23 - 7.80 (m, 3H), 7.68 - 7.44 (m, 2H), 7.39 - 7.14 (m, 3H), 6.44 - 6.31 (m, 1H), 5.59 - 5.45 (m, 1H), 4.92 - 4.82 (m, 2H), 4.79 - 4.50 (m, 3H), 1.30 - 1.21 (m, 3H).

### Example 47:

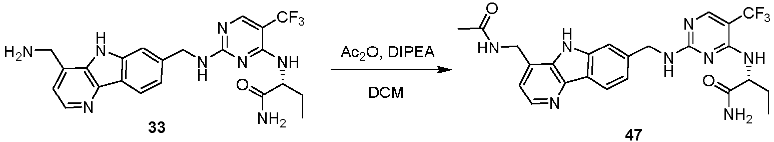

Compound **33** (5 mg, 11 µmol) was dissolved in dichloromethane (2 mL), and acetic anhydride (1.1 mg, 11 µmol) and N,N-diisopropylethylamine (2 mg, 16 µmol) were added. The mixture was stirred at room temperature overnight. The reaction solution was concentrated, and the residue was purified by preparative liquid chromatography to give compound **47** (3 mg, yield 55.1%). ESI-MS (m/z): 515.5 [M+H]⁺; ¹H NMR (500 MHz, DMSO-*d*₆) δ 11.38 (s, 1H), 8.63 - 8.51 (m, 1H), 8.37 (d, *J=* 4.8 Hz, 1H), 8.19 - 7.86 (m, 3H), 7.66 (s, 1H), 7.53 - 7.41 (m, 1H), 7.37 - 7.12 (m, 3H), 6.38 - 6.16 (m, 1H), 4.87 - 4.36 (m, 5H), 1.94 (s, 3H), 1.91 - 1.59 (m, 2H), 0.90 - 0.55 (m, 3H).

### Example 48:

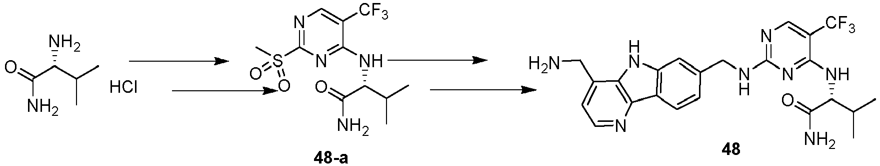

Using (R)-2-amino-3-methylbutanamide hydrochloride instead of (R)-2-aminobutanamide hydrochloride, refer to the synthesis of compound **4-c** to obtain compound **48-a,** using compound **48-a** instead of compound **1-a,** refer to the synthesis of compound **19** to obtain compound **48.** ESI-MS (m/z): 487.7 [M+H]⁺; ¹H NMR (500 MHz, DMSO-*d*₆) δ 8.37 (d, *J =* 4.7 Hz, 1H), 8.17 - 7.79 (m, 3H), 7.65 - 7.41 (m, 2H), 7.38 (d, *J* = 4.8 Hz, 1H), 7.33 - 7.12 (m, 2H), 6.20 - 5.81 (m, 1H), 4.83 - 4.43 (m, 3H), 4.06 (s, 2H), 2.10 - 1.95 (m, 1H), 0.96 - 0.58 (m, 6H).

### Example 49:

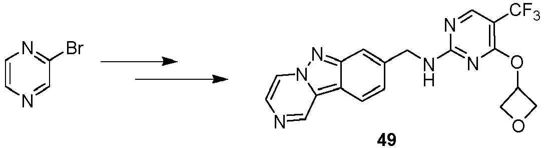

Compound **49** was obtained by replacing 6-chloropyridinecarbonitrile with 2-bromopyrazine and referring to the synthesis method of compound **24.** ESI-MS (m/z): 417.7 [M+H]⁺; ¹H NMR (500 MHz, DMSO-*d*₆) δ 9.80 (s, 1H), 9.11 - 9.01 (m, 1H), 8.83 - 8.49 (m, 1H), 8.44 - 8.36 (m, 2H), 8.32 (d, *J* = 4.6 Hz, 1H), 7.79 (s, 1H), 7.49 - 7.33 (m, 1H), 5.70 - 5.48 (m, 1H), 4.95 - 4.66 (m, 4H), 4.62 - 4.36 (m, 2H).

### Example 50:

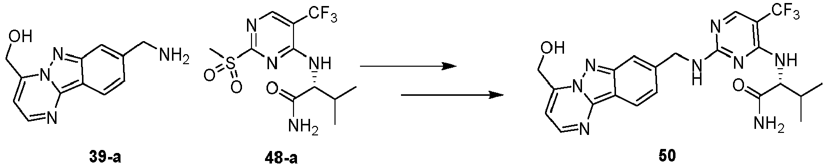

Compound **50** was obtained by replacing compound 2-c with compound **39-a** and replacing compound **4-c** with compound **48-a.** ESI-MS (m/z): 489.6 [M+H]⁺; ¹H NMR (500 MHz, DMSO-*d*₆) δ 8.77 (d, *J =* 4.4 Hz, 1H), 8.24 - 7.82 (m, 3H), 7.77 - 7.50 (m, 3H), 7.37 - 7.21 (m, 2H), 6.12 - 5.85 (m, 2H), 5.13 (d, *J =* 5.8 Hz, 2H), 4.84 - 4.50 (m, 3H), 2.06 - 1.94 (m, 1H), 0.98 - 0.63 (m, 6H).

### Example 51:

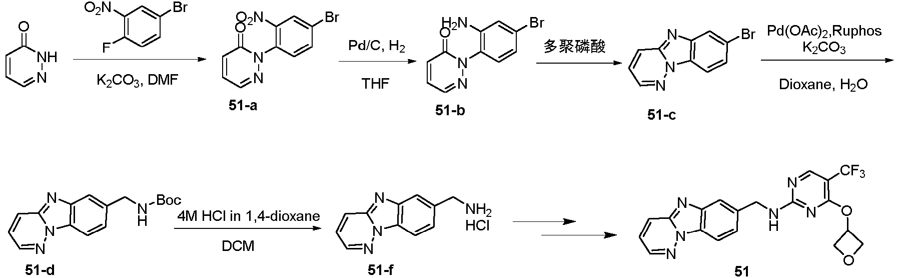

3-Pyridazinone (500 mg, 5.20 mmol) and 4-bromo-1-fluoro-2-nitrobenzene (1.14 g, 5.20 mmol) were dissolved in N,N-dimethylformamide (10 mL), and potassium carbonate (2.16 g, 15.6 mmol) was added. The reaction solution was stirred at 90°C for 16 hours. The reaction solution was cooled to room temperature, water (25 mL) was added and the mixture was extracted three times with ethyl acetate (20 mL). The organic phases were combined, washed once with water and once with saturated brine, dried over anhydrous sodium sulfate, filtered and concentrated. The residue was purified by flash column chromatography to obtain compound **51-a** (390 mg, yield 25.3%). ESI-MS (m/z): 296.1 [M+H]⁺.

Compound **51-a** (390 mg, 1.32 mmol) was dissolved in tetrahydrofuran (5 mL), and 10% palladium carbon (14.0 mg, 132 µmol) was added. The mixture was reacted under a hydrogen atmosphere at room temperature for 6 hours. The reaction solution was filtered through celite and concentrated to obtain compound **51-b** (110 mg, yield 31.4%). ESI-MS (m/z): 266.0 [M+H]⁺.

Compound **51-b** (110 mg, 413 µmol) was dissolved in polyphosphoric acid (1 g). The mixture was reacted at 150°C for 6 hours. The reaction solution was cooled to room temperature, added to water, and adjusted to pH>7 with 2N aqueous sodium hydroxide solution. The mixture was extracted three times with ethyl acetate (10 mL). The organic phases were combined, washed once with water and once with saturated brine, dried over anhydrous sodium sulfate, filtered and concentrated. The residue was purified by flash column chromatography to give compound **51-c** (56 mg, yield 54.6%). ESI-MS (m/z): 248.0 [M+H]⁺.

Compound **51-c** (56 mg, 225 µmol) and potassium [(tertbutoxycarbonylamino)methyl]trifluoroborate (69.6 mg, 293 µmol) were dissolved in a mixture of 1,4-dioxane (2 mL) and water (0.2 mL), and palladium acetate (3.8 mg, 22.6 µmol), Ruphos (21 mg, 45 µmol) and cesium carbonate (221 mg, 677 µmol) were added. The mixture was reacted at 90°C under nitrogen protection for 16 hours. The reaction solution was cooled to room temperature, water (10 mL) was added, and extraction was performed three times with ethyl acetate (10 mL). The organic phases were combined, washed once with water and once with saturated brine, dried over anhydrous sodium sulfate, filtered and concentrated. The residue was purified by flash column chromatography to give compound **51-d** (55 mg, yield 82.6%). ESI-MS (m/z): 299.2 [M+H]⁺.

Compound 51-d (55 mg, 184 µmol) was dissolved in dichloromethane (2 mL), and dioxane hydrochloride solution (4 M, 0.5 mL) was added. The reaction solution was stirred at room temperature for 2 hour. The reaction solution was concentrated to obtain compound **51-f** (43 mg, yield 99.4%). ESI-MS (m/z): 199.2 [M+H]⁺.

Compound **51** was obtained by replacing compound **1-c** with compound **51-f** and referring to the synthesis method of compound **1.** ESI-MS (m/z): 417.4 [M+H]⁺; ¹H NMR (500 MHz, DMSO-*d*₆) δ 8.80 - 8.49 (m, 2H), 8.45 - 8.38 (m, 1H), 8.26 - 8.20 (m, 1H), 8.17 - 8.10 (m, 1H), 7.81 (s, 1H), 7.54 - 7.49 (m, 1H), 7.49 - 7.45 (m, 1H), 5.70 - 5.53 (m, 1H), 5.39 - 4.80 (m, 1H), 4.78 - 4.71 (m, 2H), 4.70 - 4.66 (m, 1H), 4.61 - 4.38 (m, 2H).

### Example 52:

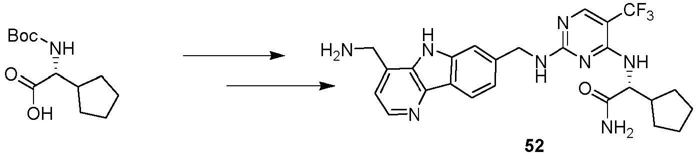

Compound **52** was obtained by replacing (R)-2-((tert-butoxycarbonyl)amino)-2-cyclopentylacetic acid with (R)-2-((tert-butoxycarbonyl)amino)-2-cyclopropylacetic acid and referring to the synthesis of Compound **42.** ESI-MS (m/z): 513.7 [M+H]⁺; ¹H NMR (500 MHz, DMSO-*d*₆) δ 8.44 - 8.32 (m, 1H), 8.18 - 7.99 (m, 3H), 7.59 - 7.42 (m, 2H), 7.38 (d, *J =* 4.8 Hz, 1H), 7.25 - 7.12 (m, 2H), 6.27 - 5.84 (m, 1H), 4.99 - 4.39 (m, 4H), 4.07 (s, 2H), 1.72 - 0.87 (m, 9H).

### Example 53:

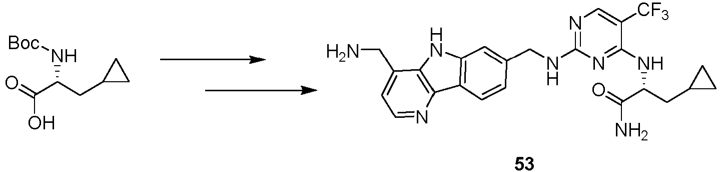

Compound 53 was obtained by replacing (R)-2-((tert-butoxycarbonyl)amino)-2-cyclopropylacetic acid with (R)-2-((tert-butoxycarbonyl)amino)-3-cyclopropylpropionic acid and referring to the synthesis of Compound **42.** ESI-MS (m/z): 499.7 [M+H]⁺; ¹H NMR (500 MHz, DMSO-*d*₆) δ 8.46 - 8.28 (m, 1H), 8.21 - 7.86 (m, 3H), 7.58 (s, 1H), 7.46 (s, 1H), 7.38 (d, *J =* 4.8 Hz, 1H), 7.32 - 7.13 (m, 2H), 6.38 - 6.20 (m, 1H), 4.83 - 4.46 (m, 3H), 4.09 (s, 2H), 1.86 - 1.45 (m, 2H), 0.71 - 0.05 (m, 4H), -0.18 - -0.29 (m, 1H).

### Example 54:

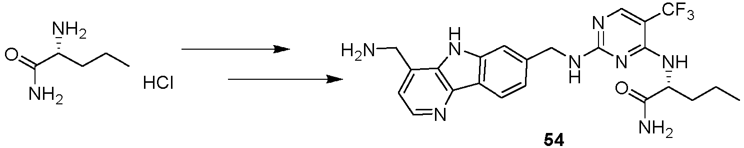

Compound **54** was obtained by replacing compound **42-b** with (R)-2-aminopentanamide hydrochloride and referring to the synthesis of compound **42.** ESI-MS (m/z): 487.6 [M+H]⁺; ¹H NMR (500 MHz, DMSO-*d*₆) δ 8.45 - 8.28 (m, 1H), 8.24 - 7.80 (m, 3H), 7.64 - 7.43 (m, 2H), 7.38 (d, *J =* 4.7 Hz, 1H), 7.32 - 7.07 (m, 2H), 6.33 - 6.16 (m, 1H), 4.88 - 4.40 (m, 3H), 4.09 (s, 2H), 1.99 - 0.77 (m, 7H).

### Example 55:

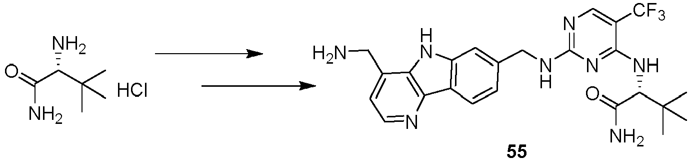

Compound **55** was obtained by replacing compound **42-b** with (R)-2-amino-3,3-dimethylbutanamide hydrochloride and referring to the synthesis of compound **42.** ESI-MS (m/z): 501.4 [M+H]⁺; ¹H NMR (500 MHz, DMSO-*d*₆) δ 8.37 (d, *J =* 4.8 Hz, 1H), 8.20 - 7.55 (m, 4H), 7.51 - 7.41 (m, 1H), 7.38 (d, *J =* 4.7 Hz, 1H), 7.32 - 7.08 (m, 2H), 5.98 - 5.64 (m, 1H), 5.00 - 4.42 (m, 3H), 4.07 (s, 2H), 1.06 - 0.70 (m, 9H).

### Example 56:

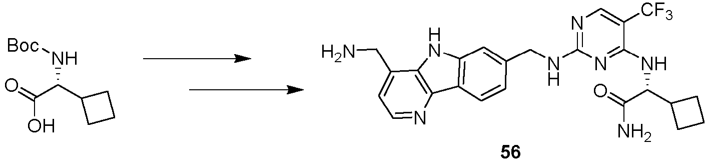

Compound **56** was obtained by replacing (R)-2-((tert-butoxycarbonyl)amino)-2-cyclobutylacetic acid with (R)-2-((tert-butoxycarbonyl)amino)-2-cyclopropylacetic acid and referring to the synthesis of Compound **42.** ESI-MS (m/z): 499.3 [M+H]⁺; ¹H NMR (500 MHz, DMSO-*d*₆) δ 8.42 - 8.26 (m, 1H), 8.19 - 7.79 (m, 3H), 7.65 - 7.34 (m, 3H), 7.30 - 7.08 (m, 2H), 6.21 - 5.95 (m, 1H), 4.90 - 3.86 (m, 5H), 2.80 - 2.54 (m, 1H), 2.03 - 1.39 (m, 6H).

### Example 57:

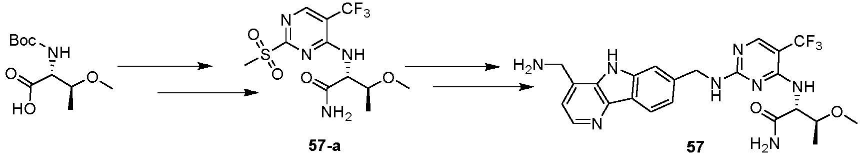

Compound **57-a** was obtained by replacing (R)-2-((tert-butoxycarbonyl)amino)-2-cyclopropylacetic acid with (2R,3S)-2-((tert-butoxycarbonyl)amino)-3-methoxybutanoic acid and referring to the synthesis of compound **42-d.** Compound **57** was obtained by replacing compound **42-d** with compound **57-a** and referring to the synthesis of compound **42.** ESI-MS (m/z): 503.3 [M+H]⁺; ¹H NMR (500 MHz, DMSO-*d*₆) δ 8.41 - 8.37 (m, 1H), 8.21 - 7.91 (m, 3H), 7.48 - 7.30 (m, 4H), 7.24 - 7.11 (m, 1H), 6.04 - 5.93 (m, 1H), 4.77 - 4.60 (m, 3H), 4.18 - 4.11 (m, 2H), 3.82 - 3.77 (m, 1H), 3.17 - 3.13 (m, 3H), 1.10 - 0.91 (m, 3H).

### Example 58:

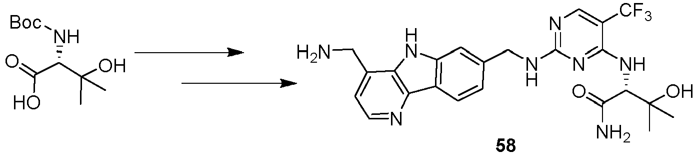

Compound **58** was obtained by referring to the synthesis of Compound **42,** using (R)-2-((tert-butoxycarbonyl)amino)-3-hydroxy-3-methylbutanoic acid instead of (R)-2-((tert-butoxycarbonyl)amino)-2-cyclopropylacetic acid. ESI-MS (m/z): 503.3 [M+H]⁺; ¹H NMR (500 MHz, DMSO-*d*₆) δ 8.44 - 8.20 (m, 1H), 8.18 - 7.91 (m, 3H), 7.85 - 6.73 (m, 6H), 6.17 - 5.95 (m, 1H), 5.18 - 3.80 (m, 6H), 1.33 - 0.97 (m, 6H).

### Example 59:

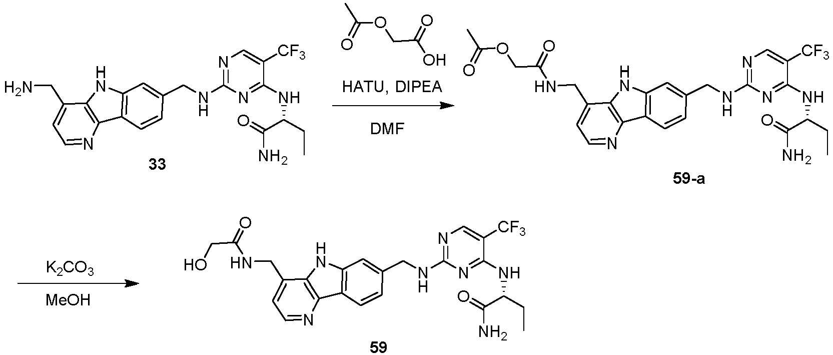

Compound 33 (10 mg, 21 µmol) was dissolved in dichloromethane (2 mL), and acetoxyacetic acid (3 mg, 25 µmol), N,N-diisopropylethylamine (8.2 mg, 64 µmol) and HATU (12 mg, 32 µmol) were added. The mixture was stirred at room temperature overnight. Water (5 mL) was added to the reaction solution, and extraction was performed three times with ethyl acetate (10 mL). The organic phases were combined, washed once with water and once with saturated brine, dried over anhydrous sodium sulfate, filtered, and concentrated to obtain a crude product, Compound **59-a** (12 mg). ESI-MS (m/z): 573.4 [M+H]⁺.

The crude product compound 59-a (12 mg) was dissolved in methanol (2 mL), and potassium carbonate (8.7 mg, 63 µmol) was added. The mixture was stirred at room temperature for 4 hours. The reaction solution was concentrated, and the residue was purified by preparative liquid chromatography to give compound **59** (3 mg, yield 27.0%). ESI-MS (m/z): 531.0 [M+H]⁺; ¹H NMR (500 MHz, DMSO-*d*₆) δ 11.33 (s, 1H), 8.59 - 8.49 (m, 1H), 8.36 (d, *J* = 4.8 Hz, 1H), 8.18 - 7.86 (m, 3H), 7.68 - 7.56 (m, 1H), 7.52 - 7.41 (m, 1H), 7.36 - 7.13 (m, 3H), 6.36 - 6.07 (m, 1H), 5.62 (t, *J =* 5.8 Hz, 1H), 4.81 - 4.45 (m, 5H), 3.91 (d, *J =* 5.6 Hz, 2H), 1.97 - 1.56 (m, 2H), 0.88 - 0.58 (m, 3H).

### Example 60:

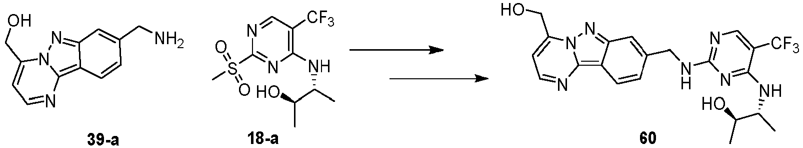

Compound **60** was obtained by replacing compound **2-c** with compound **39-a** and replacing compound **4-c** with compound **18-a,** and referring to the synthesis of compound **4.** ESI-MS (m/z): 462.5 [M+H]⁺; ¹H NMR (500 MHz, DMSO-*d*₆) δ 8.79 - 8.75 (m, 1H), 8.22 - 8.18 (m, 1H), 8.16 - 7.93 (m, 2H), 7.71 - 7.62 (m, 1H), 7.56 - 7.53 (m, 1H), 7.33 - 7.25 (m, 1H), 6.14 - 6.05 (m, 1H), 5.78 - 5.65 (m, 1H), 5.16 - 5.10 (m, 2H), 5.07 - 4.94 (m, 1H), 4.73 - 4.57 (m, 2H), 4.16 - 3.99 (m, 1H), 3.77 - 3.60 (m, 1H), 1.08 - 0.88 (m, 6H).

### Example 62:

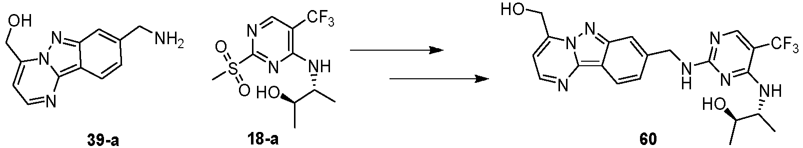

Compound **62-a** was obtained by replacing compound **37-a** with compound **37-b** and referring to the synthesis of compound **37-c.**

Compound **62-a** (500 mg, 1.13 mmol) was dissolved in tetrahydrofuran (5 mL), and tetrabutylammonium fluoride tetrahydrofuran solution (1 M, 2.26 mL) was added under ice cooling. The mixture was reacted in an ice bath for 10 minutes. Water (10 mL) was added to the reaction solution, and extraction was performed three times with ethyl acetate (20 mL). The organic phases were combined, washed three times with water and once with saturated brine, dried over anhydrous sodium sulfate, filtered and concentrated. The residue was purified by flash column chromatography to give compound **62-b** (325 mg, yield 87.6%). ESI-MS (m/z): 329.5 [M+H]⁺.

Compound **62-b** (100 mg, 304 µmol), phthalimide (49.3 mg, 335 µmol) and triphenylphosphine (160 mg, 609 µmol) were dissolved in tetrahydrofuran (10 mL), and diisopropyl azodicarboxylate (123 mg, 609 µmol) was added under ice-cooling. The mixture was stirred at 60°C for 6 hours. Water (10 mL) was added to the reaction solution, and extraction was performed three times with ethyl acetate (20 mL). The organic phases were combined, washed three times with water and once with saturated brine, dried over anhydrous sodium sulfate, filtered and concentrated. The residue was purified by flash column chromatography to give compound **62-c** (113 mg, yield 81.1%). ESI-MS (m/z): 458.3 [M+H]⁺.

Compound 62-c (60 mg, 131 µmol) was dissolved in dichloromethane (2 mL), and dioxane hydrochloride solution (4 M, 0.5 mL) was added. The mixture was stirred at room temperature for 1 hours. The reaction solution was concentrated to obtain compound **62-d** (45 mg, yield 85%). ESI-MS (m/z): 358.4 [M+H]⁺.

Compound **62-d** (45 mg, 111 µmol) and compound **4-c** (43 mg, 140 µmol) were dissolved in N,N-dimethylformamide (2 mL), and N,N-diisopropylethylamine (59 mg, 458 µmol) was added. The mixture was stirred at room temperature overnight. Water (10 mL) was added to the reaction solution, and extraction was performed three times with ethyl acetate (20 mL). The organic phases were combined, washed three times with water and once with saturated brine, dried over anhydrous sodium sulfate, filtered and concentrated. The residue was purified by flash column chromatography to give compound **62-f** (60 mg, yield 89%). ESI-MS (m/z): 604.2 [M+H]⁺.

Compound **62-f** (60 mg, 99 µmol) was dissolved in 95% ethanol (5 mL), and 80% hydrazine hydrate (0.5 mL) was added. The mixture was stirred at 80°C for 6 hours. The reaction solution was cooled to room temperature, filtered, and the filtrate was concentrated. The residue was purified by preparative liquid chromatography to give compound **62** (2 mg, yield 4.2%). ESI-MS (m/z): 474.5 [M+H]⁺; ¹H NMR (500 MHz, DMSO-*d*₆) δ 8.95 - 8.71 (m, 1H), 8.26 - 7.71 (m, 4H), 7.71 - 7.55 (m, 2H), 7.41 - 7.22 (m, 2H), 6.34 - 6.16 (m, 1H), 4.81 - 4.55 (m, 3H), 4.42 - 4.27 (m, 2H), 2.00 - 1.60 (m, 2H), 0.83 - 0.63 (m, 3H).

### Example 63:

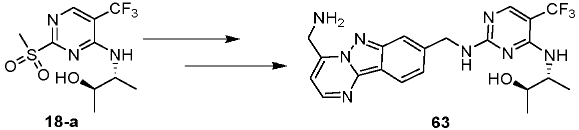

Compound **63** was obtained by replacing compound **4-c** with compound **18-a** and referring to the synthesis method of compound 62. ESI-MS (m/z): 461.3 [M+H]⁺; ¹H NMR (500 MHz, DMSO-*d*₆) δ 8.75 (d, *J =* 4.4 Hz, 1H), 8.18 (d, *J =* 8.4 Hz, 1H), 8.15 - 8.07 (m, 1H), 8.07 - 7.91 (m, 1H), 7.73 - 7.63 (m, 1H), 7.60 (d, *J =* 4.4 Hz, 1H), 7.32 - 7.22 (m, 1H), 5.76 - 5.63 (m, 1H), 5.10 - 4.90 (m, 1H), 4.78 - 4.55 (m, 2H), 4.33 (s, 2H), 4.17 - 3.96 (m, 1H), 3.79 - 3.58 (m, 1H), 1.46 - 0.71 (m, 6H).

### Example 67:

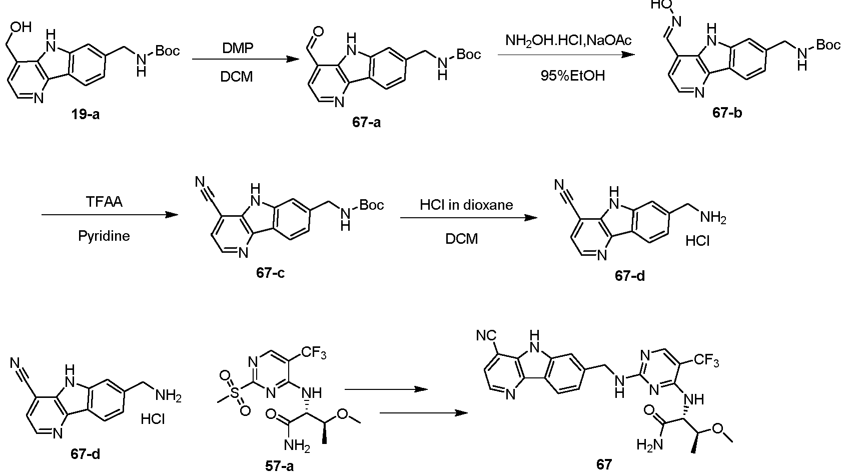

Compound 19-a (1 g, 3.05 mmol) was dissolved in dichloromethane (30 mL), and DMP (1.43 g, 3.36 mmol) was added under ice-cooling. The mixture was slowly warmed to room temperature and stirred at room temperature overnight. Saturated sodium bicarbonate (20 mL) was added to the reaction solution, dichloromethane was removed under reduced pressure, and the residue was extracted three times with ethyl acetate (30 mL). The organic phases were combined, washed once with water and once with saturated brine, dried over anhydrous sodium sulfate, filtered, and concentrated to obtain a crude product, compound **67-a** (1 g). ESI-MS (m/z): 326.8 [M+H]⁺.

The crude product compound **67-a** (1 g) was dissolved in 95% ethanol (20 mL), and sodium acetate (756 mg, 9.22 mmol) and hydroxylamine hydrochloride (320 mg, 4.61 mmol) were added. The mixture was stirred at room temperature for 4 hours. The reaction mixture was concentrated, and water (30 mL) was added to the residue, followed by extraction with ethyl acetate (30 mL) three times. The organic phases were combined, washed once with water and once with saturated brine, dried over anhydrous sodium sulfate, filtered, and concentrated to give a crude product, compound **67-b** (1.05 g). ESI-MS (m/z): 341.7 [M+H]⁺.

The crude product compound **67-b** (1.05 g) was dissolved in pyridine (10 mL), and trifluoroacetic anhydride (2.57 g, 12.22 mmol) was slowly added under ice-cooling. The mixture was slowly warmed to room temperature and stirred at room temperature for 4 hours. Methanol (5 mL) was added to the reaction solution, and the mixture was stirred at room temperature for 1 hour. The reaction mixture was concentrated, and water (30 mL) was added to the residue, followed by extraction with ethyl acetate (30 mL) three times. The organic phases were combined, washed once with saturated citric acid solution and once with saturated brine, dried over anhydrous sodium sulfate, filtered and concentrated. The residue was purified by flash column chromatography to obtain compound **67-c** (400 mg, yield 40.6%). ESI-MS (m/z): 323.7 [M+H]⁺.

Compound **67-c** (250 mg, 775 µmol) was dissolved in dichloromethane (5 mL), and dioxane hydrochloride solution (4 M, 1 mL) was added. The mixture was stirred at room temperature for 2 hours. The reaction solution was concentrated to obtain compound **67-d** (229 mg, yield 100%). ESI-MS (m/z): 223.5 [M+H]⁺.

Compound **67** was obtained by replacing compound **2-c** with compound **67-d** and replacing compound **4-c** with compound **57-a,** and referring to the synthesis of compound **4.** ESI-MS (m/z): 499.4 [M+H]⁺; ¹H NMR (500 MHz, DMSO-*d*₆) δ 12.49 - 12.28 (m, 1H), 8.67 - 8.46 (m, 1H), 8.28 - 7.96 (m, 3H), 7.83 - 7.77 (m, 1H), 7.58 - 7.50 (m, 1H), 7.45 - 7.28 (m, 3H), 6.05 - 5.94 (m, 1H), 4.80 - 4.59 (m, 3H), 3.95 - 3.73 (m, 1H), 3.31 - 3.12 (m, 3H), 1.13 - 0.88 (m, 3H).

### Example 68:

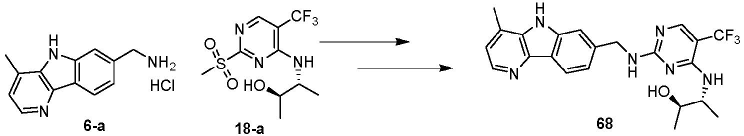

Compound 68 was obtained by replacing compound 2-c with compound 6-a and replacing compound 4-c with compound 18-a, and referring to the synthesis of compound 4. ESI-MS (m/z): 445.5 [M+H]⁺; ¹H NMR (500 MHz, DMSO-*d*₆) δ 11.34 (s, 1H), 8.31 - 8.27 (m, 1H), 8.14 - 7.90 (m, 3H), 7.47 - 7.40 (m, 1H), 7.22 - 7.14 (m, 2H), 5.83 - 5.66 (m, 1H), 5.12 - 4.93 (m, 1H), 4.75 - 4.50 (m, 2H), 4.15 - 3.97 (m, 1H), 3.81 - 3.59 (m, 1H), 2.56 - 2.55 (m, 3H), 1.17 - 0.86 (m, 6H).

### Example 69:

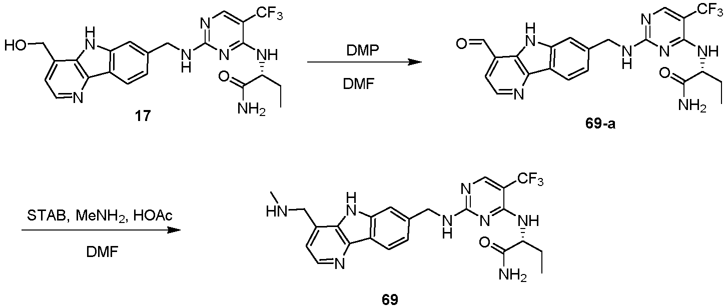

Compound 17 (20 mg, 42 µmol) was dissolved in N,N-dimethylformamide (2 mL) and DMP (20 mg, 46 µmol) was added. The mixture was stirred at room temperature overnight. Water (5 mL) was added to the reaction solution, and extraction was performed three times with ethyl acetate (10 mL). The organic phases were combined, washed once with water and once with saturated brine, dried over anhydrous sodium sulfate, filtered, and concentrated to obtain a crude product, Compound **69-a** (20 mg). ESI-MS (m/z): 472.7 [M+H]⁺.

The crude product compound **69-a** (8 mg) was dissolved in N,N-dimethylformamide (2 mL), and methylamine tetrahydrofuran solution (2 M, 34 µL) and glacial acetic acid (1 mg, 17 µmol) were added. The mixture was stirred at room temperature for 1 hour, sodium triacetoxyborohydride (18 mg, 85 µmol) was added, and the reaction was continued at room temperature overnight. The reaction solution was quenched by adding water (0.1 mL), and the mixture was purified by preparative liquid chromatography to give compound **69** (1.5 mg, yield 18.2%). ESI-MS (m/z): 487.5 [M+H]⁺; ¹H NMR (500 MHz, DMSO-*d*₆) δ 11.55 - 11.16 (m, 1H), 8.41 - 8.36 (m, 1H), 8.19 - 7.88 (m, 4H), 7.69 - 7.60 (m, 1H), 7.53 - 7.45 (m, 1H), 7.40 - 7.29 (m, 2H), 7.25 - 7.14 (m, 1H), 6.32 - 6.22 (m, 1H), 4.76 - 4.55 (m, 3H), 4.14 - 4.02 (m, 2H), 2.40 - 2.38 (m, 3H), 0.83 - 0.76 (m, 2H), 0.69 - 0.62 (m, 3H).

### Example 70:

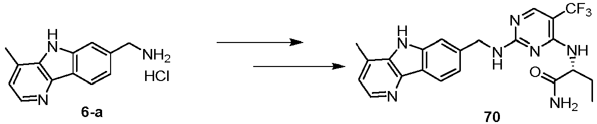

Compound **70** was obtained by replacing compound **2-c** with compound **6-a** and referring to the synthesis method of compound **4.** ESI-MS (m/z): 458.5 [M+H]⁺; ¹H NMR (500 MHz, DMSO-*d*₆) δ 11.36 (s, 1H), 8.30 (d, *J =* 4.7 Hz, 1H), 8.20 - 7.84 (m, 3H), 7.73 - 7.58 (m, 1H), 7.49 - 7.42 (m, 1H), 7.40 - 7.30 (m, 1H), 7.26 - 7.14 (m, 2H), 6.37 - 6.18 (m, 1H), 4.83 - 4.54 (m, 3H), 2.56 (s, 3H), 1.91 - 1.61 (m, 2H), 0.85 - 0.56 (m, 3H).

### Example 71:

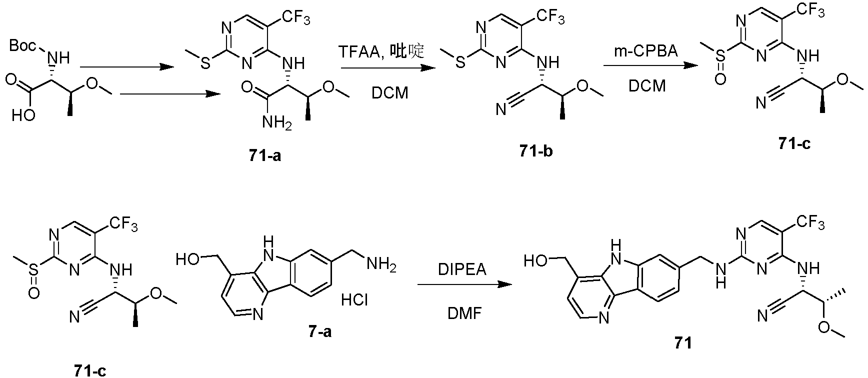

Using (2R,3S)-2-((tert-butoxycarbonyl)amino)-3-methoxybutyric acid instead of (R)-2-((tert-butoxycarbonyl)amino)-2-cyclopropylacetic acid, compound **71-a** was obtained by referring to the synthesis of compound **42-c.**

Compound **71-a** (100 mg, 308 µmol) was dissolved in dichloromethane (2 mL), and trifluoroacetic anhydride (194 mg, 925 µmol) and pyridine (244 mg, 3.08 mmol) were added under ice-cooling. The mixture was slowly warmed to room temperature and stirred for 2 hours. Water (5 mL) was added to the reaction solution, and extraction was performed three times with ethyl acetate (10 mL). The organic phases were combined, washed once with water and once with saturated brine, dried over anhydrous sodium sulfate, filtered, and concentrated to obtain a crude product, Compound **71-b** (94 mg). ESI-MS (m/z): 307.4 [M+H]⁺.

The crude product compound **71-b** (94 mg) was dissolved in dichloromethane (5 mL), and m-chloroperbenzoic acid (80 mg, 462 µmol) was added under ice cooling. The mixture was stirred at room temperature for 2 hours. Saturated sodium bicarbonate (5 mL) was added to the reaction solution, and the mixture was extracted three times with dichloromethane (10 mL). The organic phases were combined, washed once with water and once with saturated brine, dried over anhydrous sodium sulfate, filtered, and concentrated to obtain a crude product, Compound **71-c** (99 mg). ESI-MS (m/z): 323.5 [M+H]⁺.

Compound **7-a** (35.3 mg, 134 µmol) and crude compound **1-a** (50 mg) were dissolved in N,N-dimethylformamide (2 mL), and N,N-diisopropylethylamine (200 mg, 1.55 mmol) was added. The mixture was stirred at 50°C for 2 hours. After the reaction solution was cooled to room temperature, it was purified by preparative liquid chromatography to obtain compound **71** (12 mg, yield 24.7%). ESI-MS (m/z): 486.5 [M+H]⁺; ¹H NMR (500 MHz, DMSO-*d*₆) δ 11.24 (s, 1H), 8.46 - 8.22 (m, 2H), 8.22 - 8.17 (m, 1H), 8.11 - 8.06 (m, 1H), 7.49 - 7.43 (m, 1H), 7.38 - 7.32 (m, 1H), 7.25 - 7.14 (m, 2H), 5.56 - 5.50 (m, 1H), 5.39 - 4.93 (m, 1H), 4.91 - 4.83 (m, 2H), 4.79 - 4.64 (m, 2H), 3.95 - 3.50 (m, 1H), 3.25 - 2.89 (m, 3H), 1.24 - 0.94 (m, 3H).

### Example 72:

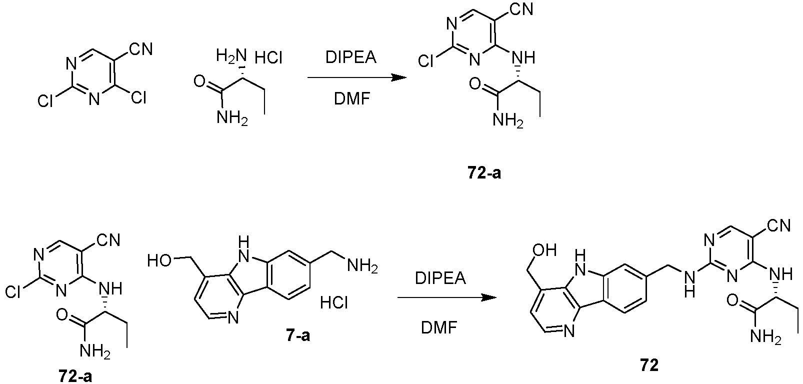

2,4-Dichloro-5-cyanopyrimidine (500 mg, 2.87 mmol) was dissolved in N,N-dimethylformamide (5 mL), and (R)-2-aminobutanamide hydrochloride (440 mg, 4.31 mmol) and N,N-diisopropylethylamine (1.11 g, 8.62 mmol) were added. The mixture was stirred at room temperature for 2 hours. Water (20 mL) was added to the reaction solution, and extraction was performed three times with ethyl acetate (20 mL). The organic phases were combined, washed once with water and once with saturated brine, dried over anhydrous sodium sulfate, filtered and concentrated. The residue was purified by column chromatography to give compound **72-a** (151 mg, yield 21.9%). ESI-MS (m/z): 240.4 [M+H]⁺.

Compound **7-a** (30 mg, 132 µmol) and compound **72-a** (31.64 mg, 132 µmol) were dissolved in N,N-dimethylformamide (2 mL), and N,N-diisopropylethylamine (200 mg, 1.55 mmol) was added. The mixture was stirred at 50°C for 2 hours. After the reaction solution was cooled to room temperature, it was purified by preparative liquid chromatography to obtain compound **72** (10 mg, yield 17.6%). ESI-MS (m/z): 431.5 [M+H]⁺; ¹H NMR (500 MHz, DMSO-*d*₆) δ 11.25 (s, 1H), 8.39 (d, *J =* 5.0 Hz, 1H), 8.37 - 8.12 (m, 2H), 8.11 - 8.05 (m, 1H), 7.56 - 7.41 (m, 2H), 7.36 (d, *J =* 4.7 Hz, 1H), 7.25 - 7.13 (m, 2H), 6.97 - 6.79 (m, 1H), 5.56 (s, 1H), 4.88 (s, 2H), 4.74 - 4.56 (m, 2H), 4.51 - 4.42 (m, 1H), 1.87 - 1.60 (m, 2H), 0.90 - 0.66 (m, 3H).

### Example 73:

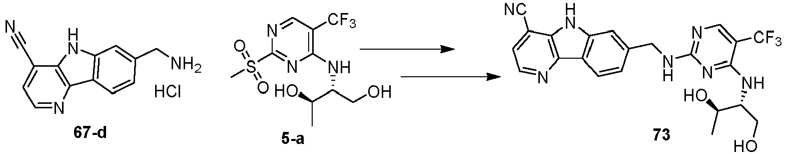

Compound **73** was obtained by replacing compound **2-c** with compound **67-d** and replacing compound **4-c** with compound **5-a** and referring to the synthesis of compound **4.** ESI-MS (m/z): 472.4 [M+H]⁺; ¹H NMR (500 MHz, DMSO-*d*₆) δ 12.62 - 12.17 (m, 1H), 8.60 - 8.53 (m, 1H), 8.20 - 7.95 (m, 3H), 7.82 - 7.47 (m, 2H), 7.35 - 7.27 (m, 1H), 5.79 - 5.65 (m, 2H), 5.12 - 4.93 (m, 2H), 4.82 - 4.74 (m, 2H), 4.12 - 3.97 (m, 3H), 0.95 - 0.81 (m, 3H).

### Example 74:

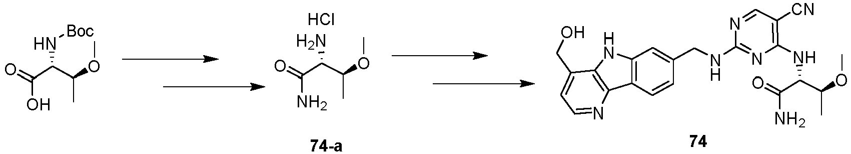

Using (2R,3S)-2-((tert-butoxycarbonyl)amino)-3-methoxybutanoic acid instead of (R)-2-((tert-butoxycarbonyl)amino)-2-cyclopropylacetic acid, compound **74-a** was obtained by referring to the synthesis of compound **42-b.** Compound **74** was obtained by replacing (R)-2-aminobutanamide hydrochloride with compound **74-a** and referring to the synthesis method of compound **72.** ESI-MS (m/z): 461.5 [M+H]⁺; ¹H NMR (500 MHz, DMSO-*d*₆) δ 11.32 - 11.20 (m, 1H), 8.49 - 8.21 (m, 3H), 8.10 - 8.05 (m, 1H), 7.57 - 7.42 (m, 2H), 7.38 - 7.34 (m, 1H), 7.31 - 7.27 (m, 1H), 7.22 - 7.13 (m, 1H), 6.36 - 6.20 (m, 1H), 5.69 - 5.48 (m, 1H), 4.96 - 4.83 (m, 2H), 4.75 - 4.51 (m, 3H), 3.98 - 3.75 (m, 1H), 3.23 - 3.11 (m, 3H), 1.12 - 0.95 (m, 3H).

### Example 75:

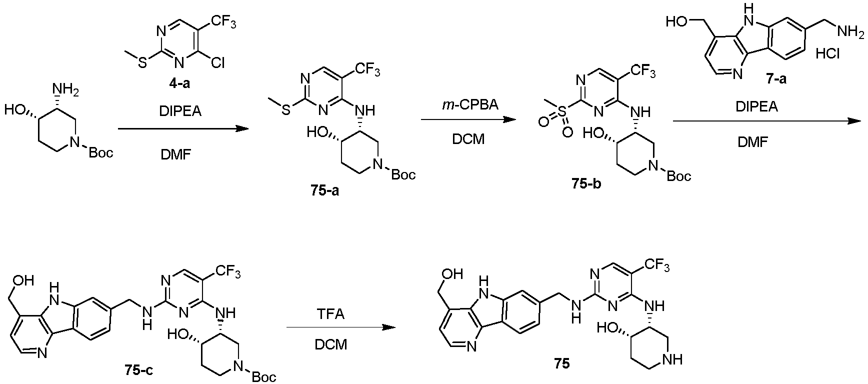

Compound **4-a** (105 mg, 459 µmol) was dissolved in N,N-dimethylformamide (3 mL), and (3R,4S)-tert-butyl 3-amino-4-hydroxypiperidine-1-carboxylate (99 mg, 459 µmol) and N,N-diisopropylethylamine (178 mg, 1.38 mmol) were added. The mixture was stirred at 50°C overnight. Water (10 mL) was added to the reaction solution, and extraction was performed three times with ethyl acetate (10 mL). The organic phases were combined, washed once with water and once with saturated brine, dried over anhydrous sodium sulfate, filtered and concentrated. The residue was purified by column chromatography to give compound **75-a** (180 mg, yield 95.9%). ESI-MS (m/z): 409.3 .[M+H]⁺.

Compound **75-a** (154 mg, 503 µmol) was dissolved in dichloromethane (5 mL), and m-chloroperbenzoic acid ((152 mg, 881 µmol)) was added. The mixture was stirred at room temperature for 2 hours. Saturated sodium bicarbonate (10 mL) was added to the reaction solution, and the mixture was extracted three times with ethyl acetate (10 mL). The organic phases were combined, washed once with water and once with saturated brine, dried over anhydrous sodium sulfate, filtered, and concentrated to obtain a crude product, Compound **75-b** (190 mg). ESI-MS (m/z): 441.5 [M+H]⁺.

Compound **7-a** (50 mg, 220 µmol) and crude compound 75-b (97 mg) were dissolved in N,N-dimethylformamide (3 mL), and N,N-diisopropylethylamine (85.3 mg, 660 µmol) was added. The mixture was stirred at room temperature overnight. The reaction solution was purified by column chromatography to obtain compound **75-c** (95 mg, yield 73.5%). ESI-MS (m/z): 588.4 [M+H]⁺.

Compound **75-c** (95 mg, 161 µmol) was dissolved in dichloromethane (2 mL), and trifluoroacetic acid (2 mL) was added. The mixture was stirred at room temperature for 1 hours. The reaction solution was concentrated, and the residue was purified by preparative liquid chromatography to give compound **75** (35 mg, yield 44.4%). ESI-MS (m/z): 488.7 [M+H]⁺; ¹H NMR (500 MHz, DMSO-*d*₆) δ 11.25 (s, 1H), 8.42 - 8.35 (m, 1H), 8.19 - 7.95 (m, 3H), 7.53 - 7.42 (m, 1H), 7.39 - 7.32 (m, 1H), 7.25 - 7.15 (m, 1H), 5.81 - 5.67 (m, 1H), 5.59 - 5.48 (m, 1H), 5.13 - 4.81 (m, 3H), 4.71 - 4.56 (m, 2H), 4.19 - 4.00 (m, 1H), 3.89 - 3.73 (m, 1H), 2.86 - 2.66 (m, 2H), 2.58 - 2.51 (m, 2H), 1.64 - 1.51 (m, 2H).

### Example 76:

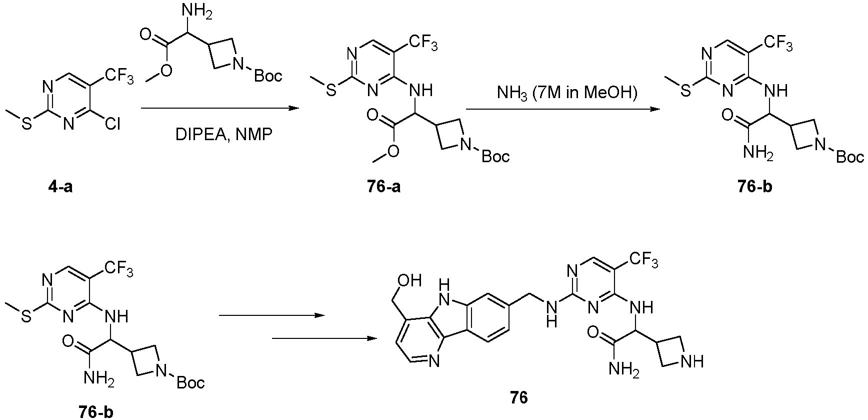

Compound **4-a** (855 mg, 3.74 mmol) and tert-butyl 3-(1-amino-2-methoxy-2-oxoethyl)azetidine-1-carboxylate (914 mg, 3.74 mmol) were dissolved in N,N-dimethylformamide (3 mL), and N,N-diisopropylethylamine (1.45 g, 11.2 mmol) was added. The mixture was stirred at 80°C for 4 hours. The reaction solution was cooled to room temperature, water (20 mL) was added, and extraction was performed three times with ethyl acetate (20 mL). The organic phases were combined, washed once with water and once with saturated brine, dried over anhydrous sodium sulfate, filtered and concentrated. The residue was purified by column chromatography to give compound 76-a (330 mg, yield 20.2%). ESI-MS (m/z): 437.0 [M+H]⁺.

Compound **76-a** (330 mg, 756 µmol) was dissolved in ammonia methanol solution (7 M, 5 mL). The mixture was stirred at 60°C overnight. The reaction solution was concentrated, and the residue was purified by reverse phase column chromatography to obtain compound **76-b** (240 mg, yield 75.3%). ESI-MS (m/z): 422.0 [M+H]⁺.

Compound **76** was obtained by replacing compound **75-a** with compound **76-b** and referring to the synthesis method of compound **75.** ESI-MS (m/z): 501.5 [M+H]⁺; ¹H NMR (500 MHz, DMSO-*d*₆) δ 11.78 - 11.18 (m, 1H), 8.43 - 8.34 (m, 1H), 8.27 - 8.14 (m, 1H), 8.14 - 7.94 (m, 2H), 7.67 - 7.44 (m, 2H), 7.42 - 7.21 (m, 3H), 6.83 - 6.31 (m, 1H), 5.88 - 5.33 (m, 1H), 4.91 - 4.85 (m, 2H), 4.82 - 4.64 (m, 2H), 4.66 - 4.48 (m, 1H), 3.82 - 3.47 (m, 4H), 3.19 - 2.92 (m, 1H), 1.51 - 1.28 (m, 1H).

### Example 77:

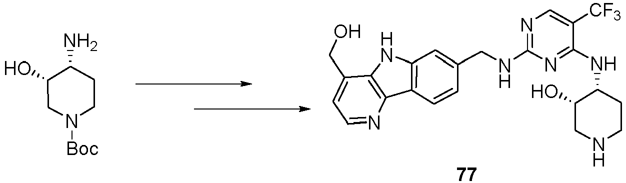

Compound **77** was obtained by replacing (3R,4S)-3-amino-4-hydroxypiperidine-1-carboxylic acid tert-butyl (3S,4R)-4-amino-3-hydroxypiperidine-1-carboxylate with tert-butyl (3S,4R)-4-amino-3-hydroxypiperidine-1-carboxylate and referring to the synthesis of Compound **75.** ESI-MS (m/z): 488.5 [M+H]⁺; ¹H NMR (500 MHz, DMSO-*d*₆) δ 11.25 (s, 1H), 8.45 - 8.32 (m, 1H), 8.30 - 7.90 (m, 3H), 7.45 (s, 1H), 7.42 - 7.31 (m, 1H), 7.28 - 7.11 (m, 1H), 5.74 - 5.48 (m, 2H), 5.12 - 4.93 (m, 1H), 4.87 (s, 2H), 4.72 - 4.52 (m, 2H), 4.19 - 3.88 (m, 1H), 3.88 - 3.40 (m, 2H), 2.93 - 2.53 (m, 4H), 1.76 - 1.23 (m, 2H).

### Example 78:

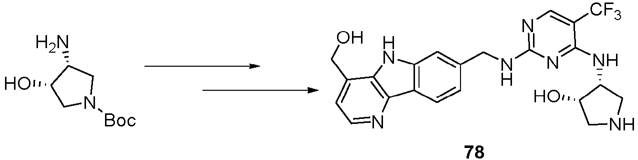

Compound 77 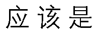 78 was obtained by replacing (3R,4S)-3-amino-4-hydroxypiperidine-1-carboxylic acid tert-butyl ester with (3R,4S)-3-amino-4-hydroxypyrrolidine-1-carboxylic acid tert-butyl ester and referring to the synthesis of Compound 75. ESI-MS (m/z): 474.4 [M+H]⁺; ¹H NMR (500 MHz, DMSO-*d*₆) δ 11.71 - 11.21 (m, 1H), 8.41 - 8.36 (m, 1H), 8.24 - 7.93 (m, 3H), 7.56 - 7.45 (m, 1H), 7.37 - 7.31 (m, 1H), 7.23 - 7.15 (m, 1H), 6.18 - 5.95 (m, 1H), 5.87 - 5.45 (m, 2H), 4.90 - 4.85 (m, 2H), 4.72 - 4.56 (m, 2H), 4.23 - 3.80 (m, 2H), 3.57 - 3.44 (m, 1H), 3.24 - 3.01 (m, 2H), 2.71 - 2.55 (m, 1H), 2.49 - 2.28 (m, 1H).

### Example 79:

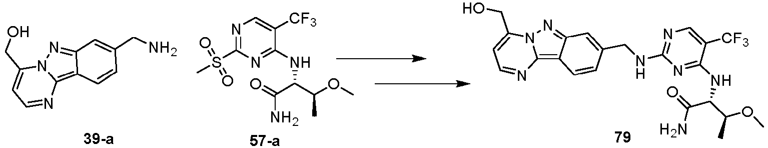

Compound **79** was obtained by replacing compound **2-c** with compound **39-a** and replacing compound **4-c** with compound **57-a,** and referring to the synthesis of compound 4. ESI-MS (m/z): 505.5 [M+H]⁺; ¹H NMR (500 MHz, DMSO-*d*₆) δ 8.81 - 8.75 (m, 1H), 8.25 - 7.96 (m, 3H), 7.75 - 7.62 (m, 1H), 7.58 - 7.53 (m, 1H), 7.45 - 7.26 (m, 3H), 6.19 - 5.91 (m, 2H), 5.19 - 5.07 (m, 2H), 4.82 - 4.60 (m, 3H), 3.96 - 3.75 (m, 1H), 3.20 - 3.10 (m, 3H), 1.11 - 0.91 (m, 3H).

### Example 80:

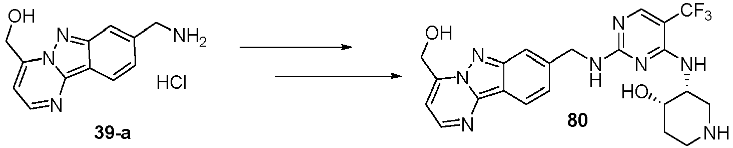

Compound **80** was obtained by replacing compound **7-a** with compound **39-a** and referring to the synthesis of compound **75.** ESI-MS (m/z): 489.2 [M+H]⁺; ¹H NMR (500 MHz, DMSO-*d*₆) δ 8.77 (d, *J =* 4.4 Hz, 1H), 8.22 - 7.96 (m, 3H), 7.76 - 7.61 (m, 1H), 7.56 - 7.53 (m, 1H), 7.34 - 7.24 (m, 1H), 6.08 (s, 1H), 5.80 - 5.67 (m, 1H), 5.15 - 4.99 (m, 3H), 4.73 - 4.55 (m, 2H), 4.20 - 3.98 (m, 1H), 3.89 - 3.71 (m, 1H), 2.85 - 2.50 (m, 4H), 1.63 - 1.50 (m, 2H).

### Example 81:

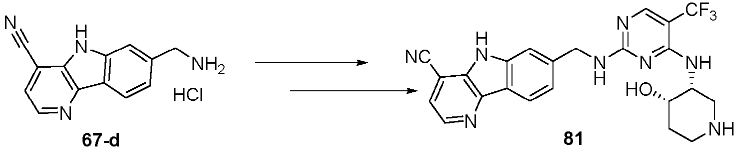

Compound **81** was obtained by replacing compound **7-a** with compound **67-d** and referring to the synthesis of compound 75. ESI-MS (m/z): 483.7 [M+H]⁺; ¹H NMR (500 MHz, DMSO-*d*₆) δ 12.37 (s, 1H), 8.56 (d, *J =* 5.0 Hz, 1H), 8.25 - 7.95 (m, 3H), 7.79 (d, *J =* 5.0 Hz, 1H), 7.63 - 7.46 (m, 1H), 7.40 - 7.26 (m, 1H), 5.82 - 5.68 (m, 1H), 5.15 - 4.90 (m, 1H), 4.75 - 4.56 (m, 2H), 4.23 - 3.93 (m, 1H), 3.86 - 3.72 (m, 1H), 2.88 - 2.52 (m, 4H), 1.65 - 1.49 (m, 2H).

### Example 82:

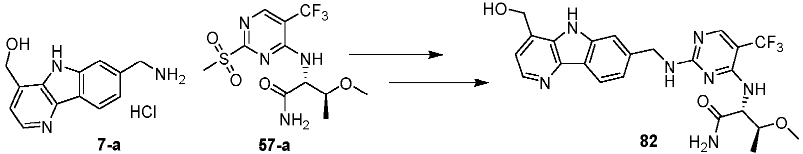

Compound **82** was obtained by replacing compound **2-c** with compound **7-a** and replacing compound **4-c** with compound **57-a,** and referring to the synthesis of compound **4.** ESI-MS (m/z): 504.2 [M+H]⁺; ¹H NMR (500 MHz, DMSO-*d*₆) δ 11.23 (s, 1H), 8.39 (d, *J =* 4.8 Hz, 1H), 8.25 - 7.89 (m, 3H), 7.51 - 7.30 (m, 4H), 7.25 - 7.12 (m, 1H), 6.05 - 5.88 (m, 1H), 5.54 (t, *J =* 5.5 Hz, 1H), 4.88 (d, *J =* 5.4 Hz, 2H), 4.76 - 4.48 (m, 3H), 3.95 - 3.66 (m, 1H), 3.33 - 3.14 (m, 3H), 1.10 - 0.84 (m, 3H).

### Example 83:

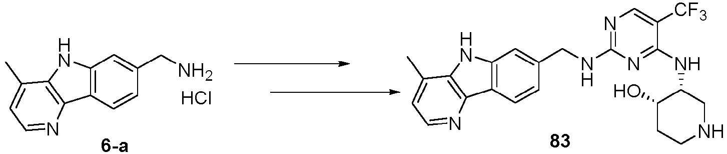

Compound **83** was obtained by replacing compound **7-a** with compound **6-a** and referring to the synthesis of compound 75. ESI-MS (m/z): 472.4 [M+H]⁺; ¹H NMR (500 MHz, DMSO-*d*₆) δ 11.48 - 11.28 (m, 1H), 8.30 (d, *J =* 4.7 Hz, 1H), 8.25 - 7.92 (m, 3H), 7.53 - 7.42 (m, 1H), 7.28 - 7.15 (m, 2H), 5.86 - 5.75 (m, 1H), 5.32 - 5.14 (m, 1H), 4.75 - 4.60 (m, 2H), 4.24 - 4.15 (m, 1H), 3.91 - 3.80 (m, 1H), 2.93 - 2.66 (m, 4H), 2.60 - 2.55 (m, 3H), 1.75 - 1.58 (m, 2H).

### Example 84:

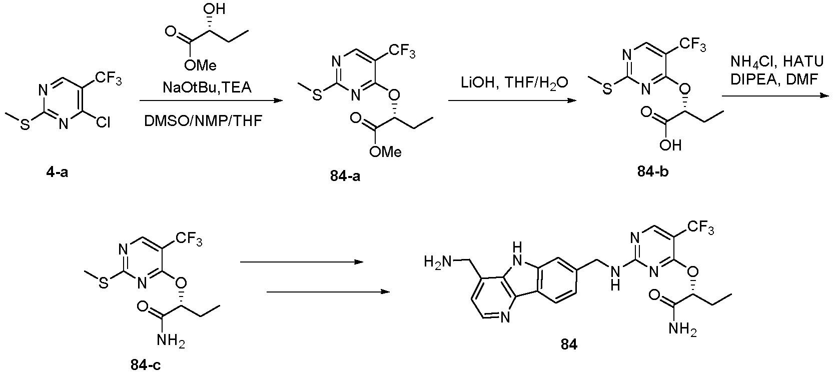

Compound **4-a** (500 mg, 2.19 mmol) and triethylamine (332 mg, 3.28 mmol) were dissolved in N-methylpyrrolidone, and a solution of (R)-2-hydroxybutyric acid methyl ester (310 mg, 2.62 mmol) in tetrahydrofuran (2 mL) and a solution of sodium tertbutoxide (315 mg, 3.28 mmol) in dimethyl sulfoxide (2 mL) were added under ice cooling. The mixture was reacted at room temperature overnight. Water (20 mL) was added to the reaction solution, and extraction was performed three times with ethyl acetate (20 mL). The organic phases were combined, washed three times with water and once with saturated brine, dried over anhydrous sodium sulfate, filtered and concentrated. The residue was purified by flash column chromatography to give compound **84-a** (434 mg, yield 64.0%). ESI-MS (m/z): 311.3 [M+H]⁺.

Compound **84-a** (360 mg, 1.16 mmol) was dissolved in tetrahydrofuran (3 mL) and water (3 mL), and lithium hydroxide monohydrate (97 mg, 2.32 mmol) was added. The mixture was stirred at room temperature for 5 hours. The pH of the reaction solution was adjusted to 4 with dilute hydrochloric acid, tetrahydrofuran was removed under reduced pressure, and the mixture was extracted three times with ethyl acetate (10 mL). The organic phases were combined, washed three times with water and once with saturated brine, dried over anhydrous sodium sulfate, filtered, and concentrated to give compound **84-b** (343 mg, yield 99.8%). ESI-MS (m/z): 297.1 [M+H]⁺.

Compound **84-b** (50 mg, 168 µmol) was dissolved in N,N-dimethylformamide (3 mL), and ammonium chloride (18 mg, 337 µmol), HATU (96 mg, 253 µmol) and N,N-diisopropylethylamine (65 mg, 506 µmol) were added. The mixture was stirred at room temperature overnight. Water (5 mL) was added to the reaction solution, and extraction was performed three times with ethyl acetate (5 mL). The organic phases were combined, washed three times with water and once with saturated brine, dried over anhydrous sodium sulfate, filtered, and concentrated to obtain compound **84-c** (49 mg, yield 98.3%). ESI-MS (m/z): 296.6 [M+H]⁺.

Compound **84** was obtained by replacing compound **42-c** with compound **84-c** and referring to the synthesis of compound **42.** ESI-MS (m/z): 474.3 [M+H]⁺; ¹H NMR (500 MHz, DMSO-*d*₆) δ 8.57 - 8.26 (m, 3H), 8.04 - 7.98 (m, 1H), 7.47 - 7.37 (m, 1H), 7.35 - 7.19 (m, 2H), 7.17 - 7.02 (m, 2H), 5.17 - 5.00 (m, 1H), 4.74 - 4.51 (m, 2H), 4.05 - 3.96 (m, 2H), 1.85 - 1.69 (m, 2H), 0.89 - 0.81 (m, 3H).

### Example 85:

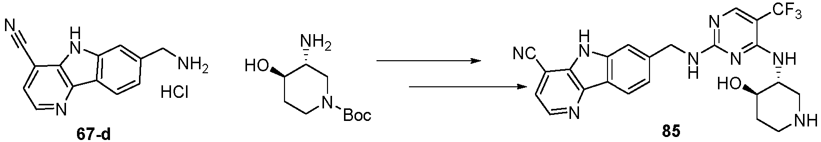

Compound **85** was obtained by replacing compound **7-a** with compound **67-d** and replacing (3R,4R)-3-amino-4-hydroxypiperidine-1-carboxylic acid tert-butyl ester with (3R,4S)-3-amino-4-hydroxypiperidine-1-carboxylic acid tert-butyl ester and referring to the synthesis of compound **85.** ESI-MS (m/z): 483.7 [M+H]⁺; ¹H NMR (500 MHz, DMSO-*d*₆) δ 12.40 (s, 1H), 8.59 - 8.52 (m, 1H), 8.20 - 8.08 (m, 2H), 8.06 - 7.93 (m, 1H), 7.81 - 7.77 (m, 1H), 7.61 - 7.52 (m, 1H), 7.40 - 7.29 (m, 1H), 6.06 - 5.95 (m, 1H), 4.82 - 4.58 (m, 3H), 3.99 - 3.73 (m, 1H), 3.64 - 3.55 (m, 1H), 3.23 - 3.00 (m, 1H), 2.85 (s, 1H), 2.48 - 2.31 (m, 1H), 2.28 - 1.68 (m, 2H), 1.52 - 1.26 (m, 2H).

### Example 86:

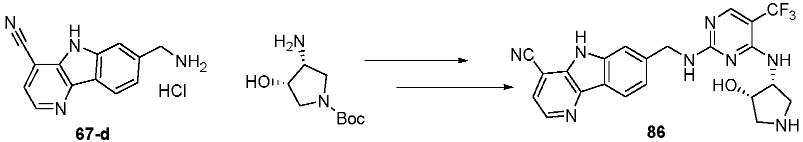

Compound **67-d** was used instead of compound **7-a,** and (3R,4S)-3-amino-4-hydroxypyrrolidine-1-carboxylic acid tert-butyl ester was used instead of (3R,4S)-3-amino-4-hydroxypiperidine-1-carboxylic acid tert-butyl ester. The synthesis of compound **75** was referred to to obtain compound **86.** ESI-MS (m/z): 469.1 [M+H]⁺; ¹H NMR (500 MHz, DMSO-*d*₆) δ 12.45 (s, 1H), 8.61 - 8.53 (m, 1H), 8.19 - 8.06 (m, 2H), 7.80 (d, *J =* 5.0 Hz, 1H), 7.59 - 7.52 (m, 1H), 7.36 - 7.19 (m, 2H), 6.22 - 5.96 (m, 1H), 5.79 - 5.43 (m, 1H), 4.76 - 4.64 (m, 2H), 4.28 - 3.99 (m, 2H), 3.14 - 2.99 (m, 3H), 2.70 - 2.57 (m, 1H), 2.40 - 2.28 (m, 1H).

### Example 87:

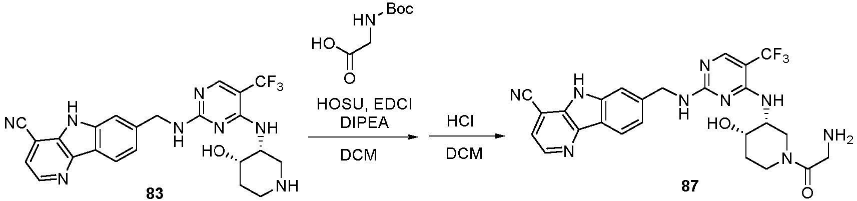

N-(tert-butyloxycarbonyl)glycine (3.6 mg, 21 µmol) and N-hydroxysuccinimide (2.9 mg, 25 µmol) were dissolved in dichloromethane (2 mL) and 1-(3-dimethylaminopropyl)-3-ethylcarbodiimide hydrochloride (6 mg, 31 µmol) was added. The mixture was stirred at room temperature for 0.5 h, and compound **83** (10 mg, 21 µmol) and N,N-diisopropylethylamine (2.7 mg, 21 mmol) were added. Water (5 mL) was added to the reaction solution, and the mixture was extracted three times with dichloromethane (10 mL). The organic phases were combined and concentrated. The residue was dissolved in dichloromethane (2 mL), dioxane hydrochloride solution (4 M, 1 mL) was added, and the mixture was stirred at room temperature for 1 hour. The reaction solution was concentrated, and the residue was purified by preparative liquid chromatography to give compound **87** (3 mg, yield 22.6%). ESI-MS (m/z): 540.4 [M+H]⁺; ¹H NMR (500 MHz, DMSO-*d*₆) δ 8.53 - 8.45 (m, 1H), 8.25 - 7.98 (m, 3H), 7.75 - 7.68 (m, 1H), 7.53 - 7.43 (m, 1H), 7.29 - 7.20 (m, 1H), 5.72 - 5.57 (m, 1H), 5.45 - 5.27 (m, 1H), 4.82 - 4.04 (m, 3H), 3.96 - 3.37 (m, 5H), 3.12 - 2.69 (m, 2H), 1.63 - 1.44 (m, 2H).

### Example 88:

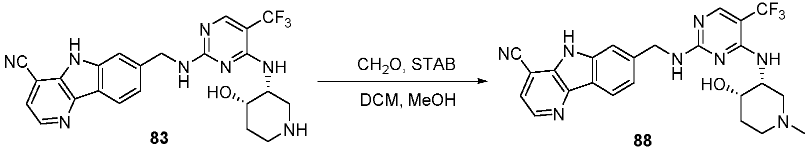

Compound **83** (450 mg, 867 µmol) was dissolved in dichloromethane (10 mL) and methanol (3 mL), and aqueous formaldehyde solution (34%, 213 µL) and glacial acetic acid (52 mg, 867 µmol) were added. The mixture was stirred at room temperature for 10 minutes, sodium triacetoxyborohydride (919 mg, 4.34 mmol) was added, and then stirred at room temperature overnight. Saturated sodium bicarbonate (10 mL) was added to the reaction solution, and the mixture was extracted three times with ethyl acetate (30 mL). The organic phases were combined and concentrated. The residue was purified by preparative liquid chromatography to obtain compound **88** (200 mg, yield 46.5%). ESI-MS (m/z): 497.5 [M+H]⁺; ¹H NMR (500 MHz, DMSO-*d*₆) δ 12.40 (s, 1H), 8.63 - 8.54 (m, 1H), 8.27 - 8.07 (m, 3H), 7.85 - 7.77 (m, 1H), 7.55 (s, 1H), 7.39 - 7.27 (m, 1H), 5.81 - 5.67 (m, 1H), 5.16 - 4.55 (m, 3H), 4.32 - 4.07 (m, 1H), 3.84 - 3.63 (m, 1H), 3.29 - 3.18 (m, 2H), 2.36 - 1.87 (m, 5H), 1.74 - 1.41 (m, 2H).

### Example 89:

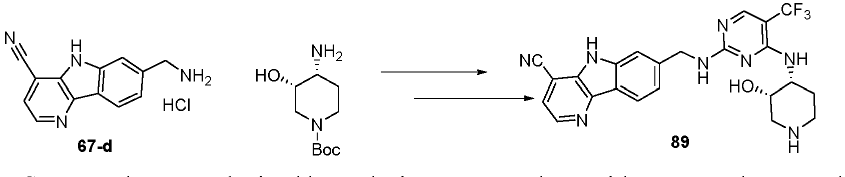

Compound **89** was obtained by replacing compound **7-a** with compound **67-d** and replacing (3R,4S)-3-amino-4-hydroxypiperidine-1-carboxylic acid tert-butyl ester with (3S,4R)-4-amino-3-hydroxypiperidine-1-carboxylic acid tert-butyl ester and referring to the synthesis of compound **89.** ESI-MS (m/z): 483.5 [M+H]⁺; ¹H NMR (500 MHz, DMSO-*d*₆) δ 12.40 (s, 1H), 8.58 (d, *J =* 5.0 Hz, 1H), 8.32 - 8.00 (m, 3H), 7.81 (d, *J =* 4.9 Hz, 1H), 7.54 (s, 1H), 7.37 - 7.29 (m, 1H), 5.77 - 5.62 (m, 1H), 5.36 - 5.10 (m, 1H), 4.76 - 4.55 (m, 2H), 4.20 - 3.89 (m, 1H), 3.78 - 3.48 (m, 1H), 2.97 - 2.55 (m, 3H), 2.51 - 2.37 (m, 2H), 1.71 - 1.29 (m, 2H).

### Example 90:

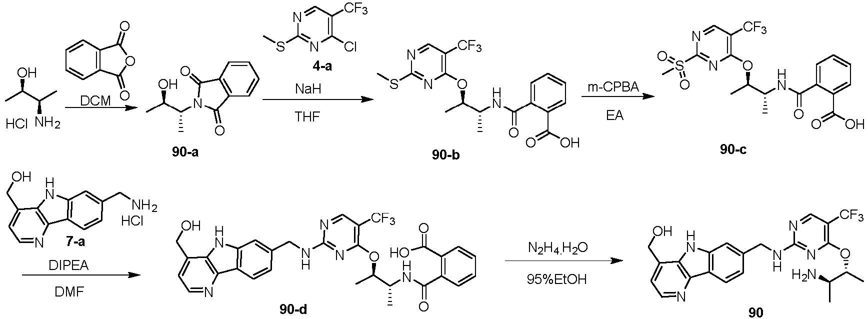

(2R,3R)-3-aminobutan-2-ol hydrochloride (1.27 g, 10.1 mmol) and phthalic anhydride (1 g, 6.8 mmol) were added to a glass bottle, and the mixture was heated to 150°C and reacted for 5 hours. The mixture was cooled to room temperature, and dichloromethane (30 mL) was added. The mixture was washed twice with water and once with saturated brine, dried over anhydrous sodium sulfate, filtered and concentrated. The residue was purified by column chromatography to give compound **90-a** (126 mg, yield 8.5%).

Compound **90-a** (70 mg, 319 µmol) was dissolved in tetrahydrofuran (2 mL), sodium hydride (60% in oil, 15 mg, 639 µmol) was added under ice-cooling, the mixture was stirred under ice-cooling for 15 minutes, and compound **4-a** (88 mg, 383 µmol) was added. The reaction solution was heated to 50°C and the reaction was continued for 5 hours. The reaction solution was cooled to room temperature, saturated ammonium chloride solution (10 mL) was added, and extraction was performed three times with ethyl acetate (10 mL). The organic phases were combined, washed once with water and once with saturated brine, dried over anhydrous sodium sulfate, filtered and concentrated. The residue was purified by preparative liquid chromatography to give compound **90-b** (30 mg, yield 21.9%). ESI-MS (m/z): 430.6 [M+H]⁺.

Compound **90-b** (30 mg, 70 µmol) was dissolved in ethyl acetate (2 mL), and m-chloroperbenzoic acid (24 mg, 139 µmol) was added. The mixture was stirred at room temperature for 2 hours. Ethyl acetate (5 mL) was added to the reaction solution and filtered. The residue was washed twice with ethyl acetate (5 mL). The organic phases were combined and concentrated to obtain the crude product Compound **90-c** (30 mg). ESI-MS (m/z): 462.3 [M+H]⁺.

Compound **90-c** (30 mg, 65 µmol) and compound **4-a** (17 mg, 65 µmol) were dissolved in N,N-dimethylformamide (2 mL), and N,N-diisopropylethylamine (25 mg, 195 µmol) was added. The mixture was stirred at room temperature overnight. The reaction solution was concentrated to obtain the crude product Compound **90-d** (32 mg). ESI-MS (m/z): 609.8 [M+H]⁺.

The crude product compound **90-d** (32 mg) was dissolved in ethanol (5 mL), and 80% hydrazine hydrate (0.1 mL) was added. The mixture was reacted at 80°C for 2 hours. The reaction solution was cooled to room temperature, filtered, and the filtrate was concentrated. The residue was purified by preparative liquid chromatography to obtain compound **90** (3 mg, yield 13 %). ESI-MS (m/z): 461.5 [M+H]⁺; ¹H NMR (500 MHz, DMSO-*d*₆) δ 11.29 - 11.10 (m, 1H), 8.34 - 8.30 (m, 1H), 8.06 - 7.81 (m, 3H), 7.49 - 7.44 (m, 1H), 7.31 - 7.27 (m, 1H), 7.17 - 7.12 (m, 1H), 5.55 - 5.39 (m, 1H), 4.86 - 4.74 (m, 2H), 4.68 - 4.35 (m, 2H), 4.09 - 3.52 (m, 1H), 2.06 - 1.84 (m, 1H), 1.23 - 0.73 (m, 6H).

### Example 91:

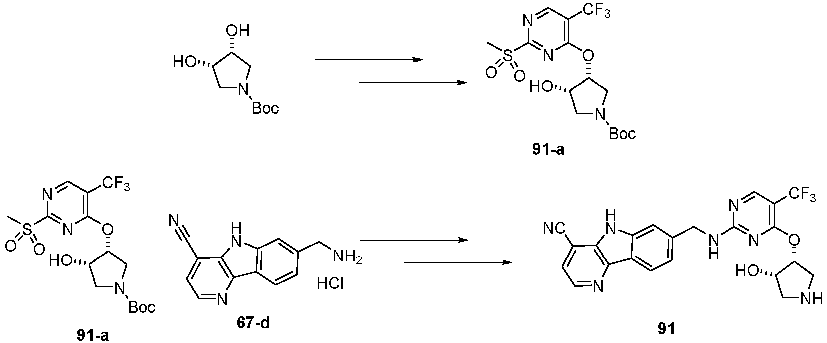

Compound **91-a** was obtained by replacing (2R,3R)-2,3-butanediol with tert-butyl cis-3,4-dihydroxypyrrolidine-1-carboxylate and referring to the synthesis method of compound **11-b.** Compound **91** was obtained by replacing compound **7-a** with compound **67-d** and replacing compound **75-b** with compound **91-a.** ESI-MS (m/z): 470.3 [M+H]⁺; ¹H NMR (500 MHz, DMSO-*d*₆) δ 12.40 (s, 1H), 8.77 - 8.40 (m, 2H), 8.38 - 8.29 (m, 1H), 8.22 - 8.16 (m, 1H), 7.84 - 7.78 (m, 1H), 7.61 - 7.54 (m, 1H), 7.39 - 7.29 (m, 1H), 5.36 - 5.09 (m, 1H), 4.85 - 4.54 (m, 3H), 4.28 - 4.01 (m, 1H), 3.24 - 3.19 (m, 1H), 3.11 - 2.80 (m, 2H), 2.71 - 2.55 (m, 2H).

### Example 92:

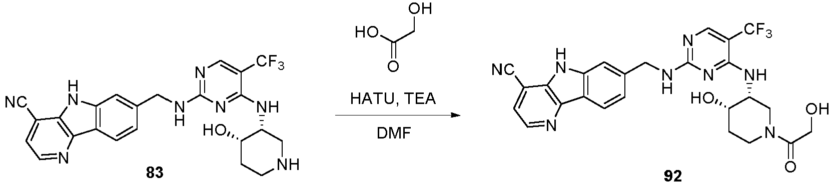

Compound **83** (20 mg, 41 µmol) and 2-hydroxyacetic acid (3 mg, 41 µmol) were dissolved in N,N-dimethylformamide (2 mL), and HATU (17 mg, 45 µmol) and triethylamine (8.4 mg, 82 µmol) were added. The mixture was stirred at room temperature for 2 hours. Water (0.1 mL) was added to the reaction solution, and the product was directly purified by preparative liquid chromatography to obtain compound **92** (6.5 mg, yield 29%). ESI-MS (m/z): 541.3 [M+H]⁺; ¹H NMR (500 MHz, DMSO-*d*₆) δ 12.46 - 12.31 (m, 1H), 8.61 - 8.55 (m, 1H), 8.33 - 8.07 (m, 3H), 7.83 - 7.79 (m, 1H), 7.60 - 7.53 (m, 1H), 7.37 - 7.29 (m, 1H), 5.80 - 5.40 (m, 2H), 4.89 - 4.44 (m, 3H), 4.40 - 3.38 (m, 6H), 3.27 - 2.80 (m, 2H), 1.68 - 1.44 (m, 2H).

### Example 93:

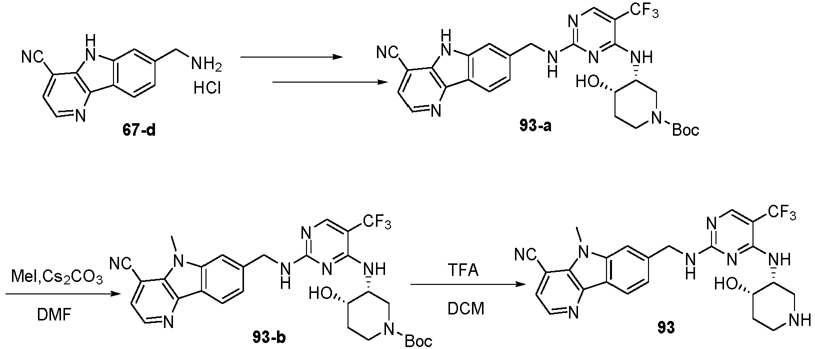

Compound **93-a** was obtained by replacing compound **7-a** with compound **67-d** and referring to the synthesis of compound **75-c.**

Compound **93-a** (20 mg, 34 µmol) was dissolved in N,N-dimethylformamide (2 mL), and iodomethane (7 mg, 51 µmol) and cesium carbonate (26 mg, 80 µmol) were added. The mixture was stirred at room temperature for 2 hours. Water (5 mL) was added to the reaction solution, and the mixture was extracted three times with ethyl acetate (5 mL). The organic phases were combined, washed once with water and once with saturated brine, dried over anhydrous sodium sulfate, filtered and concentrated. The residue was purified by preparative thin layer chromatography to give compound **93-b** (12 mg, yield 59%). ESI-MS (m/z): 597.3 [M+H]⁺.

Compound **93-b** (12 mg, 20 µmol) was dissolved in dichloromethane (2 mL), and trifluoroacetic acid (1 mL) was added. The mixture was stirred at room temperature for 2 hours. The reaction solution was concentrated, and the residue was purified by preparative liquid chromatography to give compound **93** (5.5 mg, yield 55.1%). ESI-MS (m/z): 497.5 [M+H]⁺; ¹H NMR (500 MHz, DMSO-*d*₆) δ 8.60 - 8.54 (m, 1H), 8.26 - 8.09 (m, 2H), 8.08 - 7.95 (m, 1H), 7.86 - 7.80 (m, 1H), 7.78 - 7.69 (m, 1H), 7.45 - 7.34 (m, 1H), 5.85 - 5.69 (m, 1H), 5.14 - 4.95 (m, 1H), 4.81 - 4.59 (m, 2H), 4.19 - 4.02 (m, 4H), 3.87 - 3.75 (m, 1H), 2.86 - 2.52 (m, 4H), 2.05 - 1.44 (m, 3H).

### Example 94:

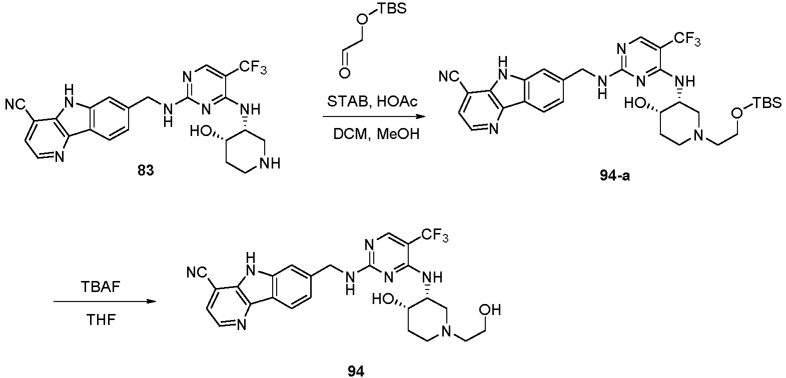

Compound **83** (20 mg, 41 µmol) and 2-(tert-butyldimethylsilyloxy)acetaldehyde (7.2 mg, 41 µmol) were dissolved in dichloromethane (1 mL) and methanol (1 mL), and glacial acetic acid (2.4 mg, 41 µmol) was added. The mixture was stirred at room temperature for 30 minutes, sodium cyanoborohydride (5.2 mg, 82 µmol) was added, and the mixture was stirred at room temperature overnight. Water (5 mL) was added to the reaction solution, and the mixture was extracted three times with ethyl acetate (5 mL). The organic phases were combined, washed once with water and once with saturated brine, dried over anhydrous sodium sulfate, filtered and concentrated. The residue was purified by preparative thin layer chromatography to give compound **94-a** (20 mg, yield 75.3%). ESI-MS (m/z): 641.3 [M+H]⁺.

Compound **94-a** (20 mg, 31 µmol) was dissolved in tetrahydrofuran (2 mL), and 1 N tetrabutylammonium fluoride tetrahydrofuran solution (0.1 mL) was added. The mixture was stirred at room temperature for 2 hours. The reaction solution was concentrated, and the residue was purified by preparative liquid chromatography to give compound **94** (5 mg, yield 30.4 %). ESI-MS (m/z): 527.3 [M+H]⁺; ¹H NMR (500 MHz, DMSO-*d*₆) δ 8.53 - 8.00 (m, 4H), 7.72 - 7.42 (m, 2H), 7.25 - 7.14 (m, 1H), 5.82 - 5.71 (m, 1H), 5.22 - 4.98 (m, 1H), 4.81 - 4.45 (m, 2H), 4.33 - 4.09 (m, 1H), 3.85 - 3.66 (m, 1H), 3.59 - 3.44 (m, 3H), 2.82 - 2.67 (m, 1H), 2.60 - 2.53 (m, 1H), 2.47 - 2.36 (m, 2H), 2.33 - 1.95 (m, 3H), 1.76 - 1.61 (m, 2H).

### Example 95:

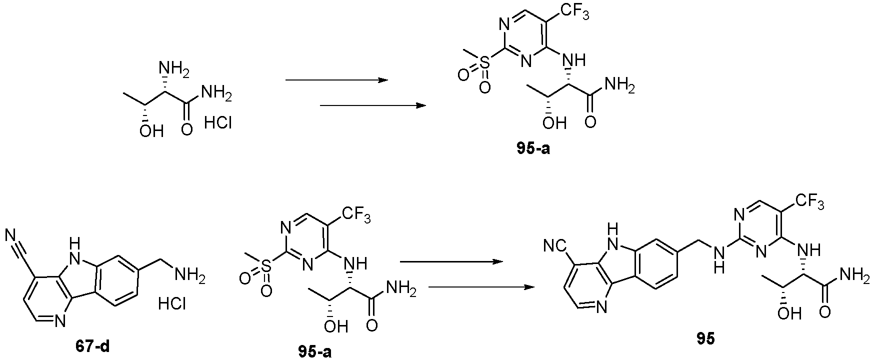

Using (2S,3R)-2-amino-3-hydroxybutanamide hydrochloride instead of (R)-2-aminobutanamide hydrochloride, compound **95-a** was obtained by referring to the synthesis of compound **4-c.** Compound **95** was obtained by replacing compound **2-c** with compound **67-d** and replacing compound **4-c** with compound **95-a** and referring to the synthesis of compound **4.** ESI-MS (m/z): 485.3 [M+H]⁺; ¹H NMR (500 MHz, DMSO-*d*₆) δ 12.31 (s, 1H), 8.49 (d, *J =* 5.0 Hz, 1H), 8.16 - 7.84 (m, 3H), 7.72 (d, *J =* 4.9 Hz, 1H), 7.54 - 7.43 (m, 1H), 7.35 - 7.20 (m, 2H), 7.12 (s, 1H), 6.01 (d, *J =* 7.6 Hz, 1H), 5.27 - 5.05 (m, 1H), 4.74 - 4.52 (m, 2H), 4.46 - 4.35 (m, 1H), 4.21 - 4.03 (m, 1H), 1.08 - 0.84 (m, 3H).

### Example 96:

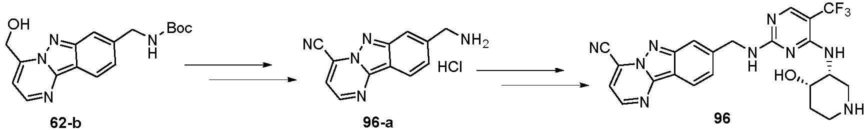

Compound **96-a** was obtained by replacing compound **19-a** with compound **62-b** and referring to the synthesis of compound **67-d.** Compound **96** was obtained by replacing compound **7-a** with compound **96-a** and referring to the synthesis of compound **75.** ESI-MS (m/z): 484.5[M+H]⁺; ¹H NMR (500 MHz, DMSO-*d*₆) δ 8.77 (d, *J* = 4.4 Hz, 1H), 8.24 - 8.20 (m, 1H), 8.18 (d, *J =* 4.4 Hz, 1H), 8.15 - 7.94 (m, 2H), 7.82 - 7.68 (m, 1H), 7.43 - 7.33 (m, 1H), 5.76 - 5.61 (m, 1H), 5.04 - 4.90 (m, 1H), 4.71 - 4.56 (m, 2H), 4.16 - 3.89 (m, 1H), 3.84 - 3.60 (m, 1H), 2.76 - 2.44 (m, 5H), 1.60 - 1.45 (m, 2H).

### Example 97:

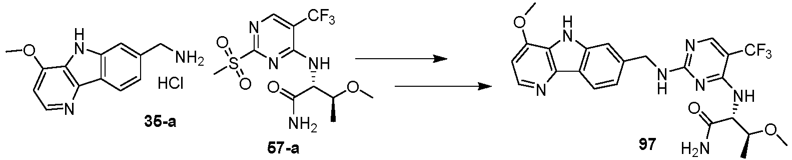

Compound **35-a** was used to replace compound **2-c,** and compound **57-a** was used to replace compound **4-c.** Compound **97** was obtained by referring to the synthesis of compound 4. ESI-MS (m/z): 504.4 [M+H]⁺; ¹H NMR (500 MHz, DMSO-*d*₆) δ 11.46 (s, 1H), 8.34 - 8.29 (m, 1H), 8.21 - 7.90 (m, 3H), 7.46 - 7.35 (m, 3H), 7.23 - 7.14 (m, 1H), 7.08 - 7.02 (m, 1H), 6.04 - 5.93 (m, 1H), 4.76 - 4.54 (m, 3H), 4.04 (s, 3H), 3.92 - 3.76 (m, 1H), 3.17 (s, 3H), 1.07 - 0.92 (m, 3H).

### Example 98:

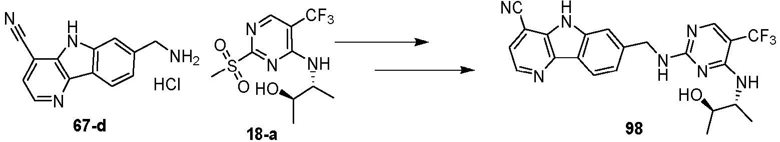

Compound **98** was obtained by replacing compound **2-c** with compound **67-d** and replacing compound **4-c** with compound **18-a** and referring to the synthesis of compound **4.** ESI-MS (m/z): 456.5 [M+H]⁺; ¹H NMR (500 MHz, DMSO-*d*₆) δ 12.62 - 12.17 (m, 1H), 8.60 - 8.53 (m, 1H), 8.20 - 7.95 (m, 3H), 7.82 - 7.47 (m, 2H), 7.35 - 7.27 (m, 1H), 5.79 - 5.65 (m, 1H), 5.12 - 4.93 (m, 1H), 4.80 - 4.55 (m, 2H), 4.19 - 3.92 (m, 1H), 3.83 - 3.57 (m, 1H), 1.16 - 0.85 (m, 6H).

### Example 99:

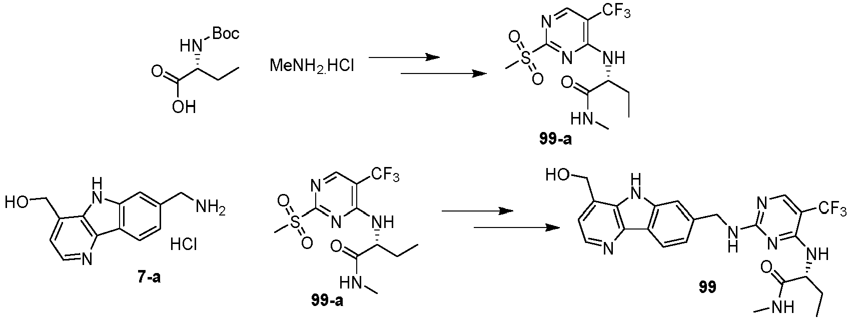

Compound **99-a** was obtained by replacing (R)-2-((tertbutoxycarbonyl)amino)butyric acid with (R)-2-((tert-butoxycarbonyl)amino)-2-cyclopropylacetic acid and replacing ammonium chloride with methylamine hydrochloride, and referring to the synthesis of compound 42-d. Compound **99** was obtained by replacing compound **4-c** with compound **99-a** and replacing compound **2-c** with compound **7-a,** and referring to the synthesis of compound **4.** ESI-MS (m/z): 488.7 [M+H]⁺;¹H NMR (500 MHz, DMSO-*d*₆) δ 11.24 (s, 1H), 8.45 - 8.34 (m, 1H), 8.19 - 7.86 (m, 4H), 7.52 - 7.42 (m, 1H), 7.38 - 7.33 (m, 1H), 7.24 - 7.14 (m, 1H), 6.32 - 6.21 (m, 1H), 5.54 (t, *J =* 5.5 Hz, 1H), 4.87 (d, *J =* 5.3 Hz, 2H), 4.72 - 4.52 (m, 3H), 2.67 - 2.52 (m, 3H), 1.94 - 1.56 (m, 2H), 0.83 - 0.63 (m, 3H).

### Example 100:

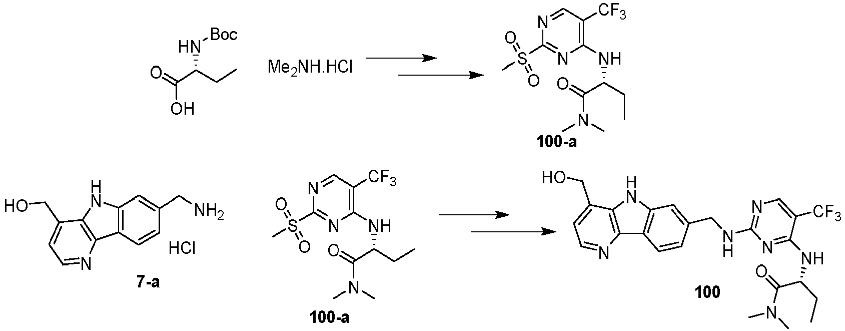

Compound 100-a was obtained by using (R)-2-((tertbutoxycarbonyl)amino)butyric acid instead of (R)-2-((tert-butoxycarbonyl)amino)-2-cyclopropylacetic acid, and dimethylamine hydrochloride instead of ammonium chloride, in accordance with the synthesis procedure of compound 42-d.Compound **100** was obtained by replacing compound **4-c** with compound **100-a** and replacing compound **2-c** with compound **7-a,** and referring to the synthesis of compound **4.** ESI-MS (m/z): 502.1 [M+H]⁺; ¹H NMR (500 MHz, DMSO-*d*₆) δ 11.24 (s, 1H), 8.41 - 8.37 (m, 1H), 8.22 - 7.92 (m, 3H), 7.46 (s, 1H), 7.37 - 7.32 (m, 1H), 7.21 - 7.16 (m, 1H), 6.33 - 6.24 (m, 1H), 5.53 (t, *J =* 5.6 Hz, 1H), 5.16 - 4.85 (m, 3H), 4.73 - 4.59 (m, 2H), 3.12 - 2.65 (m, 6H), 1.75 - 1.44 (m, 2H), 0.87 - 0.61 (m, 3H).

### Example 101:

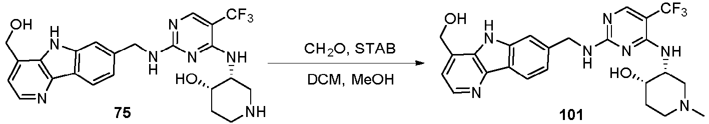

Compound 75 (15 mg, 30 µmol) was dissolved in dichloromethane (2 mL) and methanol (0.5 mL), and aqueous formaldehyde (34%, 15 µL) and glacial acetic acid (2 mg, 43 µmol) were added. The mixture was stirred at room temperature for 10 minutes, sodium triacetoxyborohydride (33 mg, 154 µmol) was added, and then stirred at room temperature overnight. Saturated aqueous sodium bicarbonate solution (5 mL) was added to the reaction solution, and the mixture was extracted three times with ethyl acetate (15 mL). The organic phases were combined and concentrated. The residue was purified by preparative liquid chromatography to obtain compound **101** (5.2 mg, yield 34.1%). ESI-MS (m/z): 502.5 [M+H]⁺; ¹H NMR (500 MHz, DMSO-*d*₆) δ 11.21 (s, 1H), 8.38 (d, *J =* 4.8 Hz, 1H), 8.24 - 7.98 (m, 3H), 7.48 - 7.42 (m, 1H), 7.35 (d, *J =* 4.7 Hz, 1H), 7.21 - 7.14 (m, 1H), 5.92 - 5.68 (m, 1H), 5.56 - 5.48 (m, 1H), 5.32 - 4.97 (m, 3H), 4.93 - 4.80 (m, 2H), 4.78 - 4.50 (m, 2H), 4.40 - 4.01 (m, 1H), 3.84 - 3.58 (m, 1H), 2.33 - 2.09 (m, 3H), 2.04 - 1.80 (m, 2H), 1.77 - 1.49 (m, 2H).

### Test Example

### Bioactivity assay for HGC27 (human gastric cancer cell) cell growth inhibition

This detection method is used to evaluate the biological activity of the compounds of the present invention at the cellular level.

HGC27 cells (purchased from the Chinese Academy of Sciences) were harvested, resuspended in complete medium and adjusted to a cell density of 0.2 x 10⁶ cells per ml. The cell suspension was added to a 96-well plate at a volume of 100 µL per well and cultured overnight in a 37°C, 5% CO₂ incubator. Prepare the test compound and add it to the cell plate so that the highest concentration of the compound is 10 µM. Set 8 concentration points according to 3-fold dilution. At the same time, 100% inhibition control wells were set, that is, wells with no cells but only an equal volume of complete culture medium; and 0% inhibition control wells, that is, wells with cells added with 0.1% DMSO. The cell plate was cultured at 37°C and 5% CO₂ for 24 hours. Remove the cell culture plate and add 25 µl CellTiter-Glo^{®} Luminescent Cell Viability Reagent (promega cat#G7573) to each well. Incubate in the dark for 10 minutes and then transfer 100 µL to a white plate and use Molecular Devices SpectraMax i3 to detect chemiluminescence. The inhibition rate was calculated using the following formula for data processing: compound inhibition rate = (0% inhibition control well signal - compound treatment well signal) / (0% inhibition control well signal - 100% inhibition control well signal) * 100%. The calculated data were fitted with four parameters using GraphPad Prism software and the corresponding IC₅₀ was calculated.

| Compound No. | HGC27 CTG IC₅₀ (nM) | Compound No. | HGC27 CTG IC₅₀ (nM) |
|---|---|---|---|
| **2** | 139 | **52** | 39 |
| **3** | 342 | **53** | 29 |
| **4** | 238 | **54** | 32 |
| **5** | 30 | **55** | 26 |
| **6** | 145 | **56** | 25 |
| **7** | 43 | **57** | 14 |
| **8** | 308 | **58** | 114 |
| **9** | 292 | **60** | 13 |
| **10** | 225 | **62** | 70 |
| **11** | 62 | **63** | 20 |
| **12** | 25 | **67** | 14 |
| **13** | 41 | **68** | 36 |
| **14** | 75 | **70** | 112 |
| **16** | 520 | **71** | 68 |
| **17** | 68 | **73** | 6 |
| **18** | 8 | **74** | 128 |
| **19** | 62 | **75** | 38 |
| **20** | 454 | **77** | 151 |
| **22** | 63 | **78** | 244 |
| **23** | 13 | **79** | 38 |
| **24** | 80 | **80** | 41 |
| **26** | 387 | **81** | 2 |
| **27** | 21 | **82** | 28 |
| **28** | 313 | **83** | 8 |
| **29** | 374 | **84** | 409 |
| **33** | 32 | **85** | 17 |
| **34** | 7 | **86** | 6 |
| **35** | 120 | **87** | 12 |
| **36** | 133 | **88** | 2 |
| **39** | 54 | **89** | 2 |
| **40** | 121 | **90** | 4.9 |
| **42** | 20 | **91** | 14 |
| **45** | 89 | **92** | 48 |
| **46** | 79 | **93** | 17 |
| **47** | 266 | **94** | 9 |
| **48** | 53 | **95** | 3 |
| **49** | 510 | **96** | 110 |
| **50** | 257 | **97** | 43 |
| **51** | 145 | **98** | 7 |

It can be seen from the above results that the compounds of the present invention have good growth inhibitory activity on HGC27 tumor cells.

### Bioactivity detection of NCI-N87 (human gastric cancer cell) cell growth inhibition

This detection method is used to evaluate the biological activity of the compounds described in this invention at the cellular level.

NCI-N87 cells (purchased from the Chinese Academy of Sciences) were harvested, resuspended in complete medium and adjusted to a cell density of 0.1 x 10⁶ cells per ml. The cell suspension was added to a 96-well plate at a volume of 100 µL per well and cultured overnight in a 37°C, 5% CO2 incubator. Prepare the test compound and add it to the cell plate so that the highest concentration of the compound is 10 µM. Set 8 concentration points according to 3-fold dilution. At the same time, 100% inhibition control wells were set, that is, wells with no cells but only an equal volume of complete culture medium; and 0% inhibition control wells, that is, wells with cells added with 0.1% DMSO. The cell plate was cultured at 37°C and 5% CO2 for 72 hours. Remove the cell culture plate and add 25 µl CellTiter-Glo Luminescent Cell Viability Reagent (promega cat#G7573) to each well. Incubate in the dark for 10 minutes and then transfer 100 µL to a white plate and use Molecular Devices SpectraMax i3 to detect chemiluminescence. The inhibition rate was calculated using the following formula for data processing: compound inhibition rate = (0% inhibition control well signal - compound treatment well signal) / (0% inhibition control well signal - 100% inhibition control well signal) * 100%. The calculated data were fitted with four parameters using GraphPad Prism software and the corresponding IC50 was calculated.

| Compound No. | N87 CTG IC₅₀ (nM) | Compound No. | N87 CTG IC₅₀ (nM) |
|---|---|---|---|
| **2** | 26 | **73** | 3 |
| **12** | 7.5 | **74** | 75 |
| **13** | 13 | **75** | 22 |
| **17** | 3.1 | **77** | 61 |
| **18** | <1 | **78** | 120 |
| **33** | 10 | **79** | 9 |
| **34** | 1.7 | **80** | 5.4 |
| **39** | 9 | **81** | 0.8 |
| **40** | 7 | **82** | 6 |
| **42** | 13 | **83** | 8 |
| **48** | 20 | **84** | 85 |
| **52** | 32 | **85** | 8.5 |
| **53** | 48 | **86** | 4 |
| **54** | 32 | **87** | 12 |
| **55** | 19 | **88** | 2 |
| **56** | 30 | **89** | 2.9 |
| **57** | 11 | **90** | 6 |
| **58** | 58 | **91** | 5.3 |
| **60** | 1.8 | **93** | 4.1 |
| **62** | 48 | **94** | 3.7 |
| **63** | 15 | **95** | 8 |
| **67** | 2.5 | **97** | 6.6 |
| **68** | 1 | **98** | 2 |
| **70** | 13 | **99** | 117 |
| **71** | 8.6 | **100** | 112 |

It can be seen from the above results that the compounds of the present invention have good growth inhibitory activity on NCI-N87 tumor cells.

### Western blot analysis of Cyclin K (CCNK) degradation

HEK293 cells (ATCC, cat #CRL-1573) were harvested and the cell density was adjusted to 1 x 106 cells per ml. 1 ml of cell suspension was plated into each well of a 6-well plate and cultured overnight. The compound stock solution was taken and diluted with DMSO to the appropriate concentration. The diluted compounds were added to the cell wells at a ratio of 1:1000 using culture medium to ensure that the DMSO concentration in each well was 0.1%. Negative control wells were also set up, which contained complete medium containing 0.1% DMSO. After treatment with the compounds at the concentrations indicated in the figures and at different times, total cell protein was extracted using RIPA cell lysis buffer (Beyotime, cat#P0013B) containing PMSF. After protein quantification using the BCA protein quantification kit (Thermo Fisher, cat#A53225), each sample was loaded with 40 µg for subsequent SDS-PAGE and western blot experiments. The specific conditions are as follows: after running the gel at a constant voltage of 120 V for 90 minutes, transfer the membrane at a constant current of 320 mA for 60 minutes. Incubate the antibodies according to the recommended dilution ratio and incubation time. The antibody information used in the experiment is as follows: Anti-GAPDH antibody (abcam, cat#ab9485), Anti-Cyclin K antibody (abcam, cat#ab85854), Goat anti-Rabbit IgG (H+L) Cross-Adsorbed Secondary Antibody, HRP (invitrogen, cat #G-21234). ECL luminescent solution (Thermo Fisher, cat#32209) was used for color development. The final results were analyzed using a gel imaging system.

According to the above detection method, some compounds described in the present invention were evaluated.

Figure 1 shows that compounds 12, 17, and 18 can significantly induce the degradation of Cyclin K compared with the control after treating HEK293 cells for 6 h at different concentrations.

It can be seen from the above results that the compounds of the present invention can effectively degrade Cyclin K.

## Claims

1. A compound having the structure of formula (I), or a stereoisomer, tautomer, solvate, pharmaceutically acceptable salt, metabolite, isotope derivative, N-oxide, or prodrug thereof: 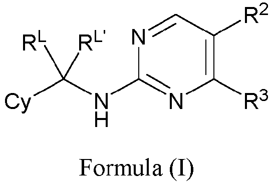
wherein, Cy represents an 11- to 14-membered tricyclic fused aromatic ring, and Cy represents 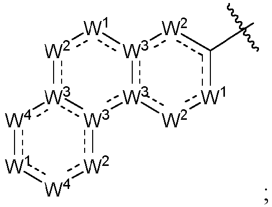
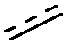 each independently represents a single bond or a double bond;
W¹ each independently represents CR, N, or a bond;
W² each independently represents CR⁰, N, NR^{a}, S, or O;
W³ each independently represents C or N, provided that no more than two W³ groups are simultaneously N;
W⁴ each independently represents CR¹, N, NR^{a}, S, or O
R, R⁰, and R¹ each independently represent hydrogen, halogen, nitro, cyano, -R^{a}, - OR^{a}, -SR^{a}, -NR^{a}R^{b}, -C(O)R^{a}, -C(O)OR^{a}, -C(O)NR^{a}R^{b}, -NR^{a}C(O)R^{b}, -S(O)₂R^{a}, - S(O)R^{a}, -S(O)₂NR^{a}R^{b}, -P(O)R^{a}R^{b}, a C₁-C₆ alkyl group, a C₂-C₆ alkenyl group, or a C₂-C₆ alkynyl group, wherein the alkyl, alkenyl, or alkynyl group may each independently may be optionally substituted with 0 to 3 substituents selected from -OR^{a}, -SR^{a}, - NR^{a}R^{b}, -NR^{a}C(O)R^{b}, -C(O)R^{a}, -C(O)OR^{a}, -C(O)NR^{a}R^{b}, -S(O)₂R^{a}, -S(O)R^{a}, - S(O)₂NR^{a}R^{b}, and -P(O)R^{a}R^{b}; wherein in -NR^{a}C(O)R^{b}, R^{b} is optionally substituted with 0, 1, or 2 substituents selected from -(C₀-C₃ alkylene)OR^{a}, -(C₀-C₃ alkylene)SR^{a}, and -(C₀-C₃ alkylene)NR^{a}R^{b};
R^{L} and R^{L'} each independently represent hydrogen, fluorine, a C₁-C₆ alkyl group, or a C₃-C₆ cycloalkyl group, and R^{L} and R^{L}' may together with the carbon atom to which they are attached form a 3- to 6-membered ring;
R² represents a halogen, -R^{a}, -OR^{a}, -SR^{a}, a nitro group, a cyano group, -NR^{a}R^{b}, - NR^{a}C(O)R^{b}, -C(O)R^{a}, -C(O)OR^{a}, -C(O)NR^{a}R^{b}, -S(O)₂R^{a}, -S(O)R^{a}, -S(O)₂NR^{a}R^{b}, - P(O)R^{a}R^{b}, a (C₂-C₆) alkenyl group, or a (C₂-C₆) alkynyl group;
R³ represents a C₁-C₆ alkyl group, a C₁-C₆ alkenyl group, a C₁-C₆ alkynyl group, a C₃-C₁₀ cycloalkyl group, a 3- to 10-membered heterocycloalkyl group, a C₆-C₁₀ aryl group, a 5- to 10-membered heteroaryl group, -NR^{M}R^{N}, -NHR^{M}, -OR^{M}, or -SR^{M};
when R³ represents a C₁-C₆ alkyl group, a C₁-C₆ alkenyl group, a C₁-C₆ alkynyl group, a C₃-C₁₀ cycloalkyl group, or a 3- to 10-membered heterocycloalkyl group, it may optionally be substituted with 0, 1, 2, or 3 substituents independently selected from oxo, nitro, halogen, cyano, -R^{a}, -(C₀-C₆ alkylene)-OR^{a}, -(C₀-C₆ alkylene)-SR^{a}, -(C₀-C₆ alkylene)-NR^{a}R^{b}, -(C₀-C₆ alkylene)-NR^{a}C(O)R^{b}, -(C₀-C₆ alkylene)-C(O)R^{a}, - (C₀-C₆ alkylene)-C(O)OR^{a}, -(C₀-C₆ alkylene)-C(O)NR^{a}R^{b}, -(C₀-C₆ alkylene)-S(O)₂R^{a}, -(C₀-C₆ alkylene)-S(O)R^{a}, -(C₀-C₆ alkylene)-S(O)₂NR^{a}R^{b}, or -(C₀-C₆ alkylene)-P(O)R^{a}R^{b};
when R³ represents a C₆-C₁₀ aryl group or a 5- to 10-membered heteroaryl group, it may optionally be substituted with 0, 1, 2, or 3 substituents independently selected from nitro, halogen, cyano, -R^{a}, -(C₀-C₆ alkylene)-OR^{a}, -(C₀-C₆ alkylene)-SR^{a}, -(C₀-C₆ alkylene)-NR^{a}R^{b}, -(C₀-C₆ alkylene)-NR^{a}C(O)R^{b}, -(C₀-C₆ alkylene)-C(O)R^{a}, - (C₀-C₆ alkylene)-C(O)OR^{a}, -(C₀-C₆ alkylene)-C(O)NR^{a}R^{b}, -(C₀-C₆ alkylene)-S(O)₂R^{a}, -(C₀-C₆ alkylene)-S(O)R^{a}, -(C₀-C₆ alkylene)-S(O)₂NR^{a}R^{b}, or -(C₀-C₆ alkylene)-P(O)R^{a}R^{b};
when R³ represents -NR^{M}R^{N}, -NHR^{M}, -OR^{M}, or -SR^{M}, R^{M} and R^{N} each independently represent a C₁-C₆ alkyl group, a -(C₀-C₆ alkylene)(C₃-C₁₀ cycloalkyl) group, a -(C₀-C₆ alkylene)(3- to 10-membered heterocycloalkyl) group, a -(C₀-C₆ alkylene)(C₆-C₁₀ aryl) group, or a -(C₀-C₆ alkylene)(5- to 10-membered heteroaryl) group; R^{M} and R^{N} may each independently be optionally substituted with 0, 1, 2, or 3 substituents selected from oxo, nitro, halogen, cyano, -R^{a}, a 4- to 8-membered heterocycloalkyl group, -(C₀-C₆ alkylene)-OR^{a}, -(C₀-C₆ alkylene)-SR^{a}, -(C₀-C₆ alkylene)-NR^{a}R^{b}, -(C₀-C₆ alkylene)-NR^{a}C(O)R^{b}, -(C₀-C₆ alkylene)-C(O)R^{a}, -(C₀-C₆ alkylene)-C(O)OR^{a}, -(C₀-C₆ alkylene)-C(O)NR^{a}R^{b}, -(C₀-C₆ alkylene)-S(O)₂R^{a}, - (C₀-C₆ alkylene)-S(O)R^{a}, -(C₀-C₆ alkylene)-S(O)₂NR^{a}R^{b}, or -(C₀-C₆ alkylene)-P(O)R^{a}R^{b}; wherein when R^{M} or R^{N} represents a -(C₀-C₆ alkylene)(3- to 10-membered heterocycloalkyl) group containing a nitrogen atom, and the substituent is positioned on the nitrogen atom, the carbon atom adjacent to the nitrogen atom in the substituent may be further substituted with an oxo group;
wherein, R^{a} and R^{b} each independently represent a hydrogen atom, a C₁-C₆ alkyl group, or a C₃-C₈ cycloalkyl group, wherein the alkyl group or cycloalkyl group may each independently be optionally substituted with 0, 1, 2, or 3 halogen atoms.

2. The compound, or a stereoisomer, tautomer, solvate, pharmaceutically acceptable salt, metabolite, isotope derivative, N-oxide, or prodrug thereof according to claim 1, **characterized in that** Cy represents: 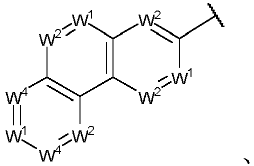 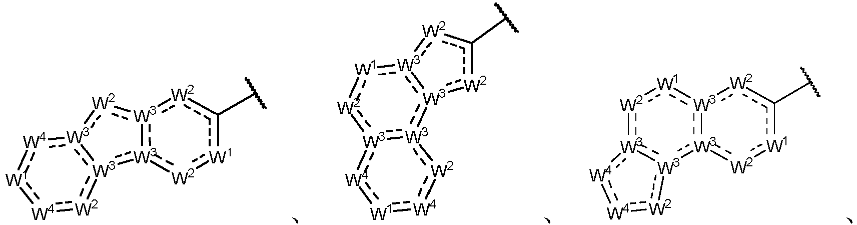 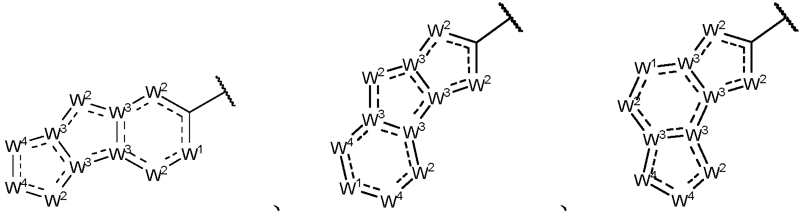 or 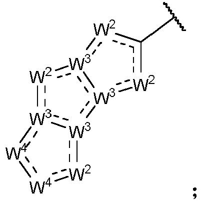 wherein each W¹ is independently selected from CR or N; and W², W³, and W⁴ are as defined above with respect to Formula (I).

3. The compound, or a stereoisomer, tautomer, solvate, pharmaceutically acceptable salt, metabolite, isotope derivative, N-oxide, or prodrug thereof according to any one of the preceding claims, **characterized in that** Cy represents: 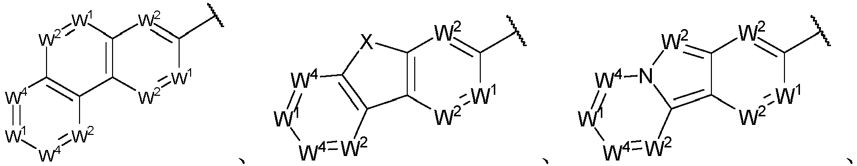 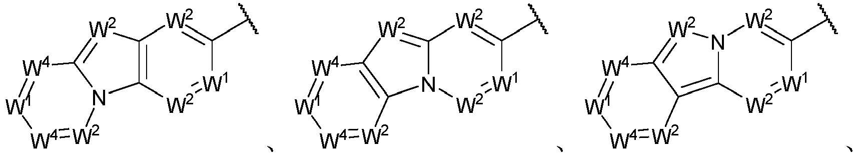 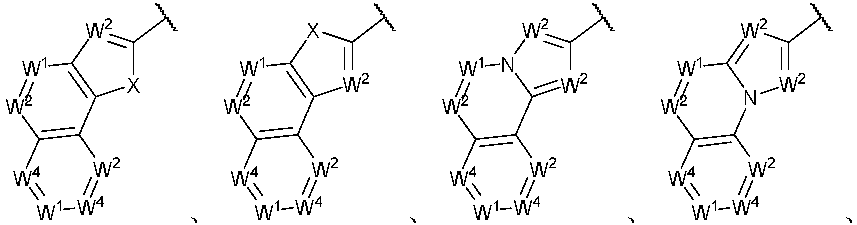 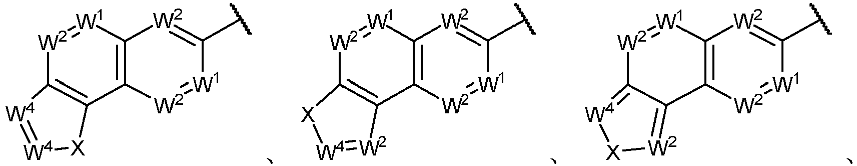 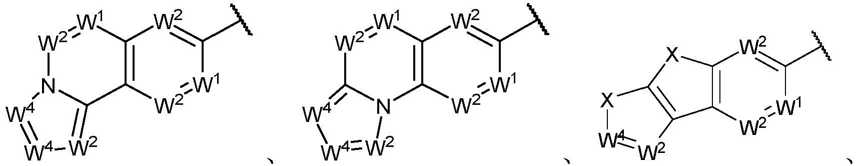 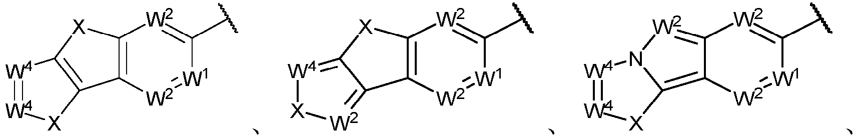 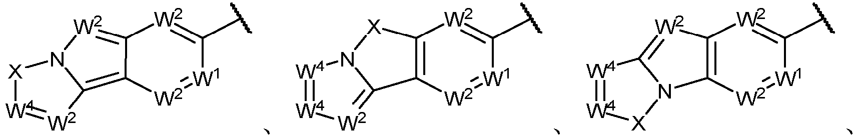 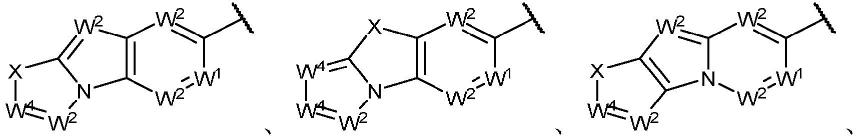 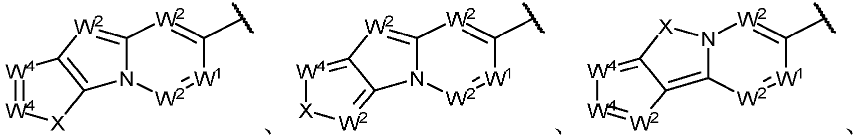 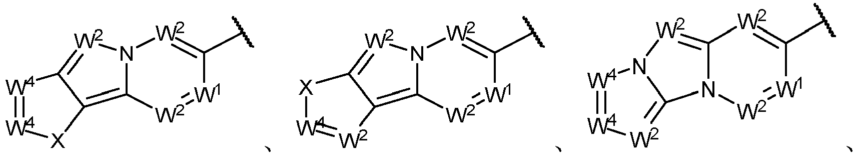 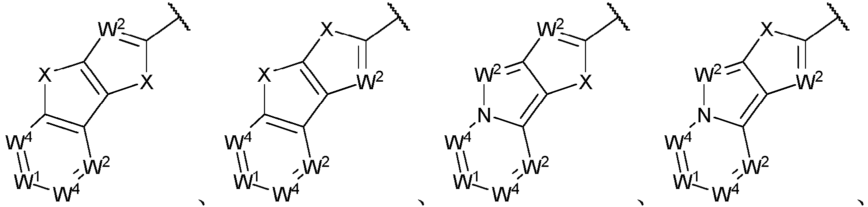 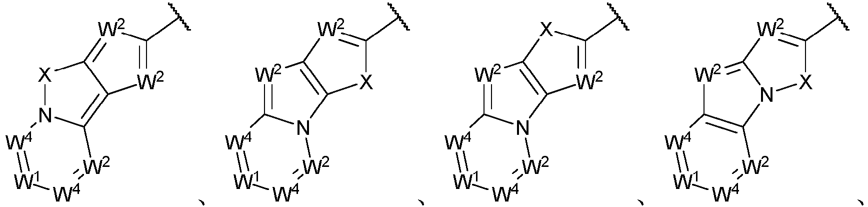 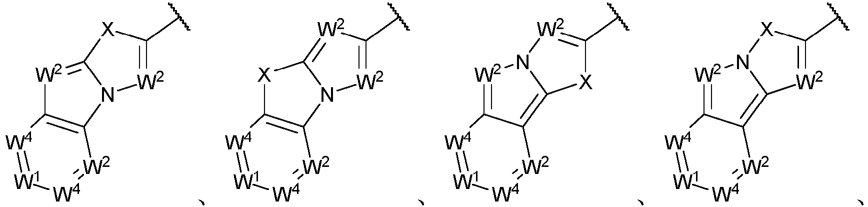 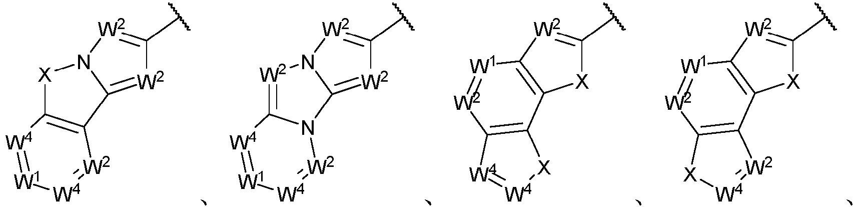 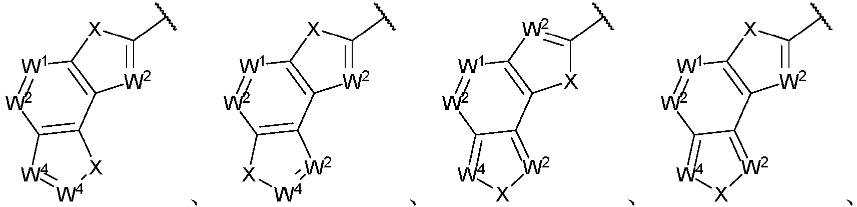 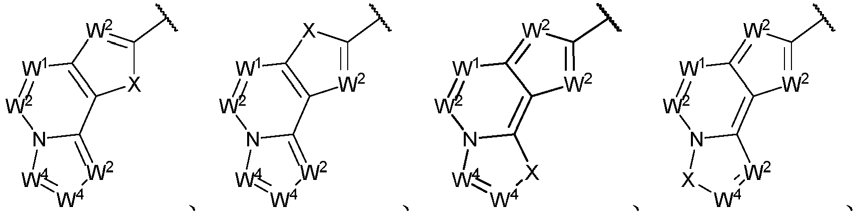 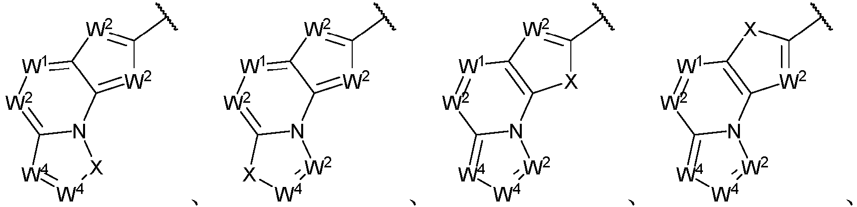 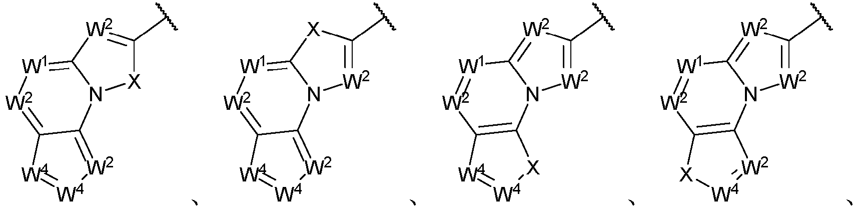 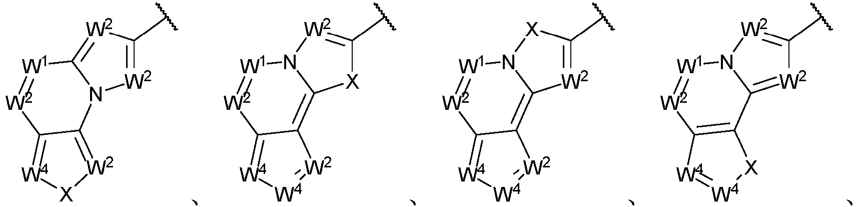 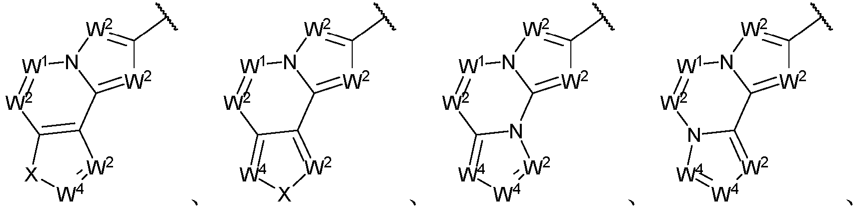 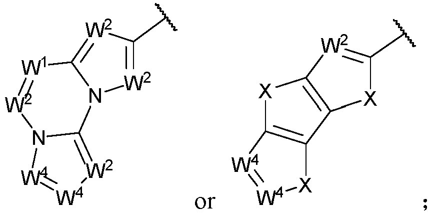 wherein each X is independently selected from NR^{a}, O, or S; each W¹ is independently selected from CR or N; each W² is independently selected from CR⁰ or N; and each W⁴ is independently selected from CR¹ or N.

4. The compound, or a stereoisomer, tautomer, solvate, pharmaceutically acceptable salt, metabolite, isotope derivative, N-oxide, or prodrug thereof according to any one of the preceeding claims, **characterized in that** Cy represents: 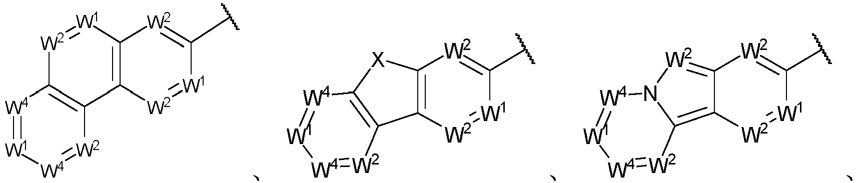 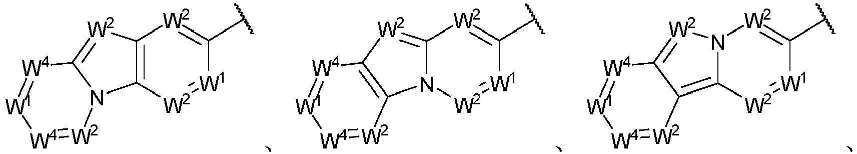 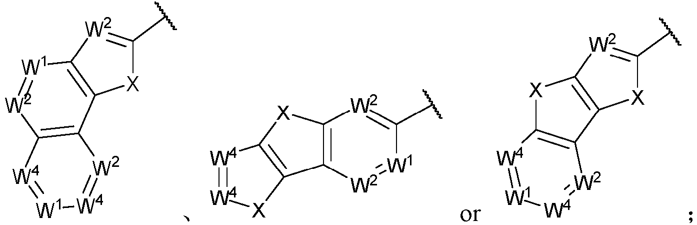 wherein each X is independently selected from NR^{a}, O, or S; each W¹ is independently selected from CR or N; each W² is independently selected from CR⁰ or N; and each W⁴ is independently selected from CR¹ or N.

5. The compound, or a stereoisomer, tautomer, solvate, pharmaceutically acceptable salt, metabolite, isotope derivative, N-oxide, or prodrug thereof according to any one of the preceeding claims, **characterized in that** each W¹ is independently selected from CR or N; wherein each R is independently selected from hydrogen, halogen, cyano, -R^{a}, or -OR^{a}; preferably, each R is independently selected from hydrogen, halogen, or -R^{a}; more preferably, each R is independently selected from hydrogen, C₁-C₆ alkyl.

6. The compound, or a stereoisomer, tautomer, solvate, pharmaceutically acceptable salt, metabolite, isotope derivative, N-oxide, or prodrug thereof according to any one of the preceeding claims, **characterized in that** each W² is independently selected from CR⁰ or N; wherein each R⁰ is independently selected from hydrogen, halogen, cyano, -R^{a}, or -OR^{a}; preferably, each R⁰ is independently selected from hydrogen, halogen, cyano, or -R^{a}; more preferably, each R⁰ is independently selected from hydrogen, cyano, or C₁-C₆ alkyl.

7. The compound, or a stereoisomer, tautomer, solvate, pharmaceutically acceptable salt, metabolite, isotope derivative, N-oxide, or prodrug thereof according to any one of the preceeding claims, **characterized in that** each W⁴ is independently selected from CR¹ or N; wherein each R¹ is independently selected from hydrogen, halogen, cyano, C₁-C₆ alkyl, C₃-C₈ cycloalkyl, -OR^{a}, -SR^{a}, -N R^{a}R^{b}, -C(O) R^{a}, - C(O)O R^{a}, -C(O)N R^{a}R^{b}, -N R^{a}C(O)R^{b}, -S(O)₂ R^{a}, or -S(O) R^{a}, wherein each C₁-C₆ alkyl may be independently substituted with 0, 1, 2, or 3 substituents selected from halogen, -O R^{a}, -S R^{a}, -N R^{a}R^{b}, -N R^{a}C(O)R^{b}, -C(O) R^{a}, -C(O)O R^{a}, -C(O)N R^{a}R^{b}, - S(O)₂ R^{a}, or -S(O) R^{a}; wherein the R^{b} in -N R^{a}C(O)R^{b} may optionally be substituted with 0, 1, or 2 substituents independently selected from -(C₀-C₃ alkylene)O R^{a}, -(C₀-C₃ alkylene)S R^{a}, and -(C₀-C₃ alkylene)N R^{a}R^{b}; preferably, the R^{b} in -N R^{a}C(O)R^{b} may optionally be substituted with 0, 1, or 2 substituents independently selected from -(C₀-C₃ alkylene)OH, -(C₀-C₃ alkylene)SH, and -(C₀-C₃ alkylene)NH₂; more preferably, in -N R^{a}C(O)R^{b}, R^{b} may optionally be substituted with 0, 1, or 2 substituents independently selected from -CH₂OH, -CH₂SH, and -CH₂NH₂.

8. The compound, or a stereoisomer, tautomer, solvate, pharmaceutically acceptable salt, metabolite, isotope derivative, N-oxide, or prodrug thereof according to any one of the preceeding claims, **characterized in that** W₄ independently represents CR¹ or N, wherein R¹ independently represents hydrogen, a halogen atom, a cyano group, a C₁-C₆ alkyl group, a C₃-C₈ cycloalkyl group, -OR^{a}, -SR^{a}, -N R^{a}R^{b}, -C(O)R^{a}, -C(O)N R^{a}R^{b}, -N R^{a}C(O)R^{b}, or -S(O)₂R^{a}, wherein the C₁-C₆ alkyl group may optionally be substituted with 0, 1, 2, or 3 substituents independently selected from halogen atoms, -OR^{a}, -SR^{a}, -N R^{a}R^{b}, and -N R^{a}C(O)R^{b}, wherein, in -N R^{a}C(O)R^{b}, R^{b} may optionally be substituted with 0, 1, or 2 substituents independently selected from -(C₀-C₃ alkylene)OR^{a}, -(C₀-C₃ alkylene)SR^{a}, and -(C₀-C₃ alkylene)N R^{a}R^{b}; preferably, in -N R^{a}C(O)R^{b}, R^{b} may optionally be substituted with 0, 1, or 2 substituents independently selected from -(C₀-C₃ alkylene)OH, -(C₀-C₃ alkylene)SH, and -(C₀-C₃ alkylene)NH₂; more preferably, in -N R^{a}C(O)R^{b}, R^{b} may optionally be substituted with 0, 1, or 2 substituents independently selected from -CH₂OH, -CH₂SH, and -CH₂NH₂.

9. The compound, or a stereoisomer, tautomer, solvate, pharmaceutically acceptable salt, metabolite, isotope derivative, N-oxide, or prodrug thereof according to any one of the preceding claims, **characterized in that** W⁴ independently represents CR¹ or N, wherein R¹ independently represents hydrogen, halogen, cyano, C₁-C₆ alkyl, C₃-C₈ cycloalkyl, -(C₀-C₆ alkylene)OR^{a}, -(C₀-C₆ alkylene)SR^{a}, -(C₀-C₆ alkylene)NR^{a}R^{b}, or -(C₀-C₆ alkylene)NR^{a}C(O)R^{b}.

10. The compound, or a stereoisomer, tautomer, solvate, pharmaceutically acceptable salt, metabolite, isotope derivative, N-oxide, or prodrug thereof according to any one of the preceding claims, **characterized in that** R^{L} and R^{L}' each independently represent hydrogen or fluorine; preferably, R^{L} and R^{L}' are both hydrogen.

11. The compound, or a stereoisomer, tautomer, solvate, pharmaceutically acceptable salt, metabolite, isotope derivative, N-oxide, or prodrug thereof according to any one of the preceding claims, **characterized in that** R² represents a halogen, -R^{a}, - OR^{a}, -SR^{a}, nitro, cyano, -NR^{a}R^{b}, or -NR^{a}C(O)R^{b}; preferably, R² represents a halogen or -R^{a}; more preferably, R² is -CF₃.

12. The compound, or a stereoisomer, tautomer, solvate, pharmaceutically acceptable salt, metabolite, isotope derivative, N-oxide, or prodrug thereof according to any one of the preceding claims, **characterized in that** R³ represents a C₁-C₆ alkyl, C₃-C₁₀ cycloalkyl, or 3- to 10-membered heterocycloalkyl group, each of which may independently be substituted with 0, 1, 2, or 3 substituents selected from oxo, halogen, cyano, -R^{a}, -(C₀-C₆ alkylene)OR^{a}, -(C₀-C₆ alkylene)SR^{a}, -(C₀-C₆ alkylene)NR^{a}R^{b}, - (C₀-C₆ alkylene)NR^{a}C(O)R^{b}, -(C₀-C₆ alkylene)C(O)R^{a}, -(C₀-C₆ alkylene)C(O)OR^{a}, - (C₀-C₆ alkylene)C(O)NR^{a}R^{b}, -(C₀-C₆ alkylene)S(O)₂R^{a}, -(C₀-C₆ alkylene)S(O)R^{a}, - (C₀-C₆ alkylene)S(O)₂NR^{a}R^{b}, or -(C₀-C₆ alkylene)P(O)R^{a}R^{b}.

13. The compound, or a stereoisomer, tautomer, solvate, pharmaceutically acceptable salt, metabolite, isotope derivative, N-oxide, or prodrug thereof according to any one of the preceding claims, **characterized in that** R³ represents a C₆-C₁₀ aryl or a 5- to 10-membered heteroaryl group, each of which may independently be substituted with 0, 1, 2, or 3 substituents selected from halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}.

14. The compound, or a stereoisomer, tautomer, solvate, pharmaceutically acceptable salt, metabolite, isotope derivative, N-oxide, or prodrug thereof according to any one of the preceding claims, **characterized in that** R³ represents -NR^{M}R^{N}, -NHR^{M}, -OR^{M}, or -SR^{M}, wherein R^{M} and R^{N} are each independently selected from C₁-C₆ alkyl, -(C₀-C₆ alkylene)(C₃-C₁₀ cycloalkyl), -(C₀-C₆ alkylene)(3- to 10-membered heterocycloalkyl), -(C₀-C₆ alkylene)(C₆-C₁₀ aryl), or -(C₀-C₆ alkylene)(5- to 10-membered heteroaryl); R^{M} and R^{N} may each optionally be substituted with 0, 1, 2, or 3 substituents selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}; wherein, when R^{M} or R^{N} represents a -(C₀-C₆ alkylene)(3- to 10-membered heterocycloalkyl) containing a nitrogen atom and the substituent is located on said nitrogen atom, the carbon atom adjacent to said nitrogen atom in the substituent may be further substituted with an oxo group.

15. The compound, or a stereoisomer, tautomer, solvate, pharmaceutically acceptable salt, metabolite, isotope derivative, N-oxide, or prodrug thereof according to any one of the preceding claims, **characterized in that** R³ represents -NR^{M}R^{N}, -NHR^{M}, -OR^{M}, or -SR^{M}, wherein R^{M} and R^{N} each independently represent C₁-C₆ alkyl, -(C₀-C₆ alkylene)(C₃-C₁₀ cycloalkyl), -(C₀-C₆ alkylene)(3- to 10-membered heterocycloalkyl), -(C₀-C₆ alkylene)(C₆-C₁₀ aryl), or -(C₀-C₆ alkylene)(5- to 10-membered heteroaryl); each of R^{M} and R^{N} is optionally substituted with 0, 1, 2, or 3 substituents independently selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}.

16. The compound, or a stereoisomer, tautomer, solvate, pharmaceutically acceptable salt, metabolite, isotope derivative, N-oxide, or prodrug thereof according to any one of the preceding claims, **characterized in that** R³ represents -NR^{M}R^{N}, -NHR^{M}, -OR^{M}, or -SR^{M}, wherein each of R^{M} and R^{N} independently represents hydrogen or C₁-C₆ alkyl, -(C₀-C₆ alkylene)(C₃-C₁₀ cycloalkyl), -(C₀-C₆ alkylene)(3- to 10-membered heterocycloalkyl), -(C₀-C₆ alkylene)(C₆-C₁₀ aryl), or -(C₀-C₆ alkylene)(5- to 10-membered heteroaryl); each of R^{M} and R^{N} may independently be substituted with 0, 1, 2, or 3 substituents selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, and -(C₀-C₆ alkylene)P(O)R^{a}R^{b}.

17. The compound, or a stereoisomer, tautomer, solvate, pharmaceutically acceptable salt, metabolite, isotope derivative, N-oxide, or prodrug thereof according to any one of the preceding claims, **characterized in that** R³ represents -NHR^{M}, -OR^{M}, or -SR^{M}, wherein R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl); wherein the R^{M} is independently optionally substituted with 0, 1, 2, or 3 substituents selected from oxo, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, and -(C₀-C₆ alkylene)P(O)R^{a}R^{b}.

18. The compound, or a stereoisomer, tautomer, solvate, pharmaceutically acceptable salt, metabolite, isotope derivative, N-oxide, or prodrug thereof according to any one of the preceding claims, **characterized in that** R³ represents -NHR^{M}, -OR^{M}, or -SR^{M}, wherein R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl), wherein the R^{M} may optionally be substituted with 0, 1, 2, or 3 substituents independently selected from oxo, halogen, cyano, -R^{a}, -(C₀-C₆ alkylene)OR^{a}, -(C₀-C₆ alkylene)SR^{a}, - (C₀-C₆ alkylene)NR^{a}R^{b}, -NR^{a}C(O)R^{b}, -C(O)R^{a}, -C(O)NR^{a}R^{b}, -S(O)₂R^{a}, -S(O)R^{a}, - S(O)₂NR^{a}R^{b}, or -P(O)R^{a}R^{b}; more preferably, the R^{M} is independently substituted with 0, 1, 2, or 3 substituents selected from R^{a}, -(C₀-C₆ alkylene)OR^{a}, -(C₀-C₆ alkylene)SR^{a}, -(C₀-C₆ alkylene)NR^{a}R^{b}, and -C(O)NR^{a}R^{b}.

19. The compound, or a stereoisomer, tautomer, solvate, pharmaceutically acceptable salt, metabolite, isotope derivative, N-oxide, or prodrug thereof according to any one of the preceding claims, **characterized in that** R³ represents -NHR^{M}, -OR^{M}, or -SR^{M}, wherein each R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl), wherein the R^{M} may optionally be substituted with 0, 1, 2, or 3 substituents independently selected from oxo, halogen, cyano, -R^{a}, -OR^{a}, -SR^{a}, -NR^{a}R^{b}, -NR^{a}C(O)R^{b}, -C(O)R^{a}, -C(O)NR^{a}R^{b}, -S(O)₂R^{a}, -S(O)R^{a}, -S(O)₂NR^{a}R^{b}, or -P(O)R^{a}R^{b}; more preferably, each R^{M} is independently substituted with 0, 1, 2, or 3 substituents selected from -R^{a}, -OR^{a}, -SR^{a}, -NR^{a}R^{b}, and -C(O)NR^{a}R^{b}.

20. The compound, or a stereoisomer, tautomer, solvate, pharmaceutically acceptable salt, metabolite, isotope derivative, N-oxide, or prodrug thereof according to claim 1, **characterized in that** R³ represents -NHR^{M}, -OR^{M}, or -SR^{M}, wherein each R^{M} is independently selected from C₁-C₆ alkyl, -(C₀-C₆ alkylene)(C₃-C₁₀ cycloalkyl), and -(C₀-C₆ alkylene)(3- to 10-membered heterocycloalkyl). Each R^{M} may optionally be substituted with 0, 1, 2, or 3 substituents independently selected from oxo, halogen, cyano, -OR^{a}, -SR^{a}, -NR^{a}R^{b}, -NR^{a}C(O)R^{b}, -C(O)R^{a}, -C(O)NR^{a}R^{b}, -S(O)₂R^{a}, -S(O)R^{a}, - S(O)₂NR^{a}R^{b}, or -P(O)R^{a}R^{b}; more preferably, each R^{M} is independently substituted with 0, 1, 2, or 3 substituents selected from -OR^{a}, -SR^{a}, -NR^{a}R^{b}, and -C(O)NR^{a}R^{b}.

21. The compound, or a stereoisomer, tautomer, solvate, pharmaceutically acceptable salt, metabolite, isotope derivative, N-oxide, or prodrug thereof according to any one of the preceding claims, **characterized in that** R³ represents -NR^{M}R^{N}, -NHR^{M}, -OR^{M}, or -SR^{M}, and preferably R³ represents -NHR^{M}, -OR^{M}, or -SR^{M}, wherein R^{M} and R^{N} are each independently selected from -(C₀-C₆ alkylene)(3- to 10-membered heterocycloalkyl) containing a nitrogen atom, which may be optionally substituted with 0, 1, 2, or 3 substituents selected from oxo, nitro, halogen, cyano, -R^{a}, -(C₀-C₆ alkylene)OR^{a}, -(C₀-C₆ alkylene)SR^{a}, -(C₀-C₆ alkylene)NR^{a}R^{b}, -(C₀-C₆ alkylene)NR^{a}C(O)R^{b}, -(C₀-C₆ alkylene)C(O)R^{a}, -(C₀-C₆ alkylene)C(O)OR^{a}, -(C₀-C₆ alkylene)C(O)NR^{a}R^{b}, -(C₀-C₆ alkylene)S(O)₂R^{a}, -(C₀-C₆ alkylene)S(O)R^{a}, -(C₀-C₆ alkylene)S(O)₂NR^{a}R^{b}, or -(C₀-C₆ alkylene)P(O)R^{a}R^{b}, wherein the substituents are located on the nitrogen atom, and the carbon atom adjacent to the nitrogen atom in the substituent may be further substituted with oxo.

22. The compound, or a stereoisomer, tautomer, solvate, pharmaceutically acceptable salt, metabolite, isotope derivative, N-oxide, or prodrug thereof according to any one of the preceding claims, **characterized in that** R^{a} and R^{b} are each independently hydrogen, C₁-C₃ alkyl, or C₃-C₆ cycloalkyl, wherein the alkyl and cycloalkyl groups may optionally be substituted with 0, 1, 2, or 3 halogen atoms.

23. The compound, or a stereoisomer, tautomer, solvate, pharmaceutically acceptable salt, metabolite, isotope derivative, N-oxide, or prodrug thereof according to any one of the preceding claims, **characterized in that** R^{a} and R^{b} are each independently hydrogen or C₁-C₃ alkyl, wherein the alkyl group may optionally be substituted with 0, 1, 2, or 3 halogen atoms.

24. The compound, or a stereoisomer, tautomer, solvate, pharmaceutically acceptable salt, metabolite, isotope derivative, N-oxide, or prodrug thereof according to any one of the preceding claims, wherein the compound is selected from any one of the compounds listed in the following table:
| | | | |
|---|---|---|---|
| 1 | 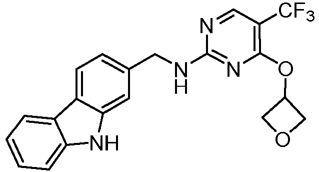 | 2 | 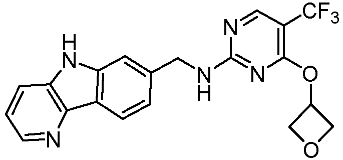 |
| 3 | 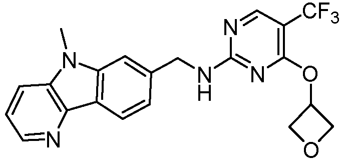 | 4 | 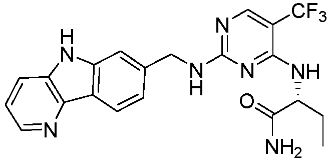 |
| 5 | 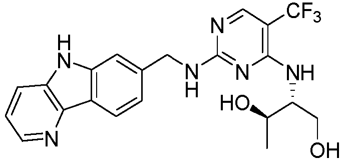 | 6 | 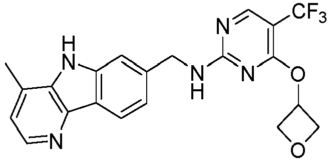 |
| 7 | 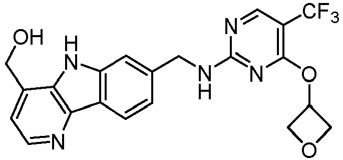 | 8 | 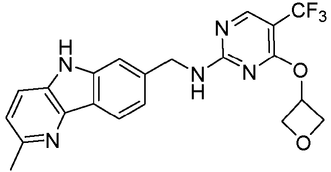 |
| 9 | 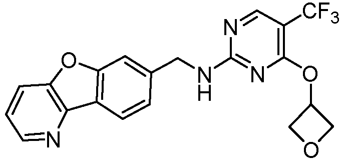 | 10 | 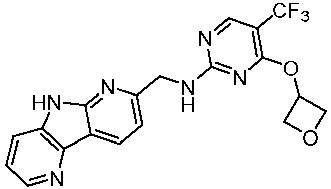 |
| 11 | 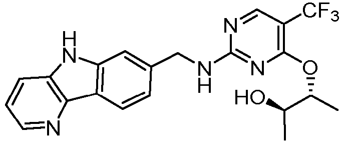 | 12 | 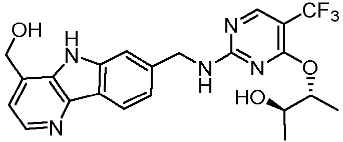 |
| 13 | 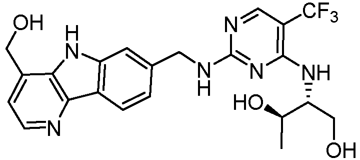 | 14 | 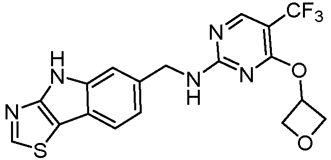 |
| 15 | 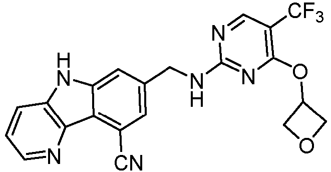 | 16 | 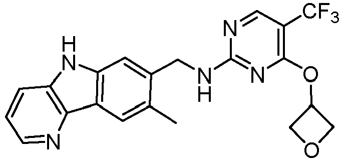 |
| 17 | 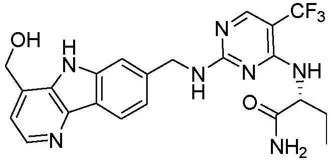 | 18 | 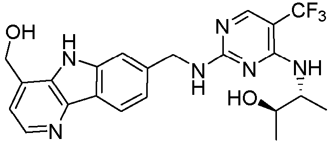 |
| 19 | 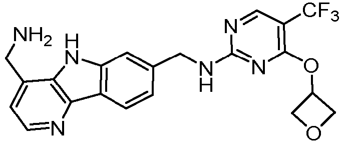 | 20 | 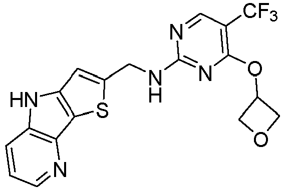 |
| 21 | 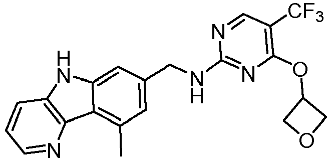 | 22 | 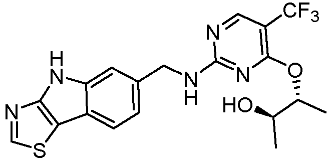 |
| 23 | 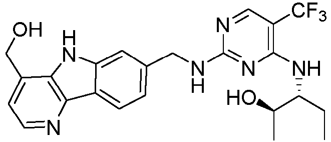 | 24 | 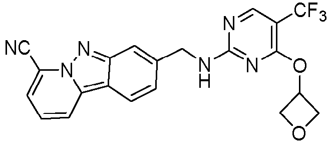 |
| 25 | 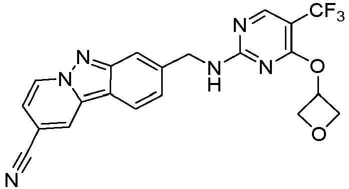 | 26 | 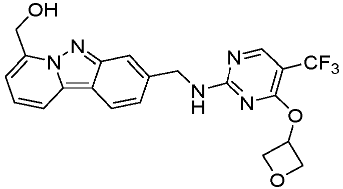 |
| 27 | 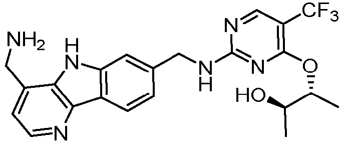 | 28 | 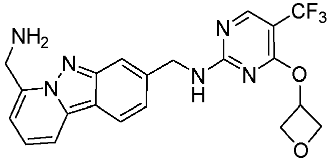 |
| 29 | 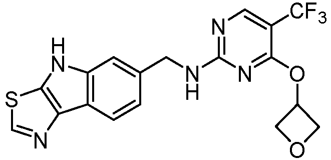 | 30 | 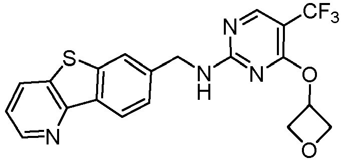 |
| 31 | 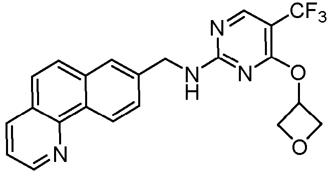 | 32 | 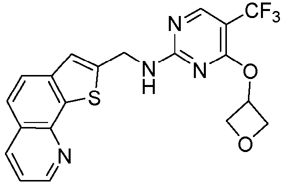 |
| 33 | 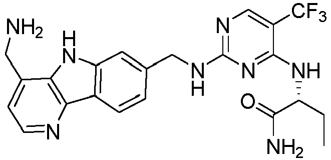 | 34 | 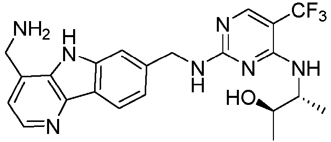 |
| 35 | 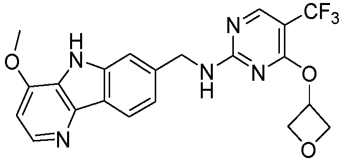 | 36 | 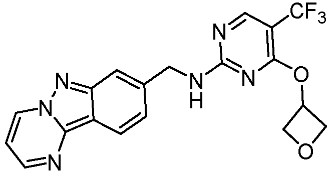 |
| 37 | 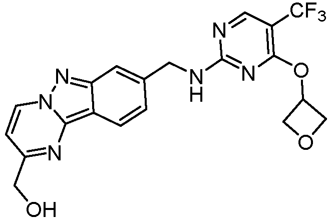 | 38 | 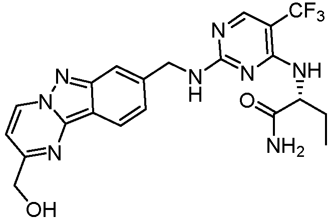 |
| 39 | 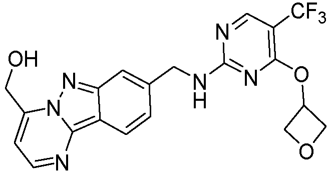 | 40 | 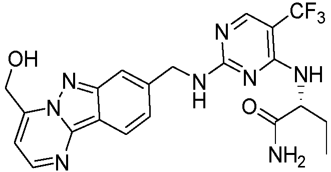 |
| 41 | 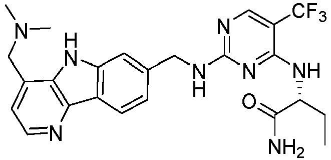 | 42 | 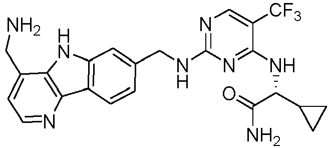 |
| 43 | 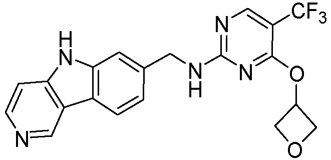 | 44 | 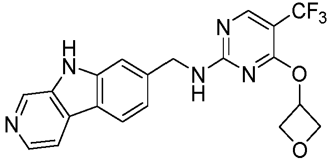 |
| 45 | 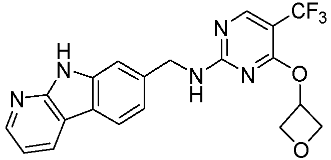 | 46 | 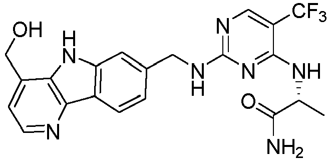 |
| 47 | 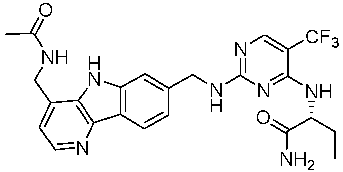 | 48 | 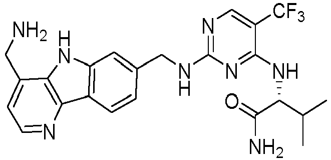 |
| 49 | 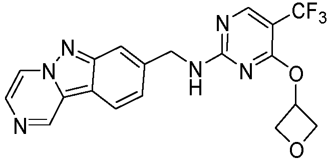 | 50 | 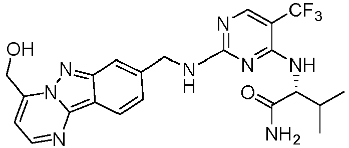 |
| 51 | 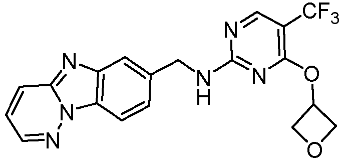 | 52 | 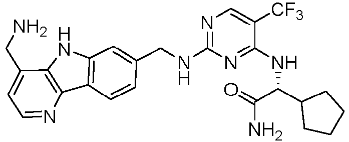 |
| 53 | 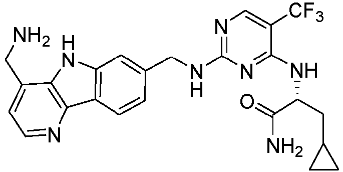 | 54 | 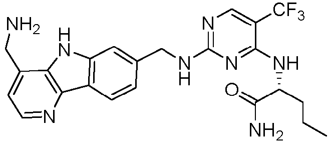 |
| 55 | 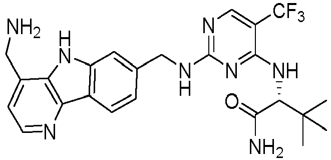 | 56 | 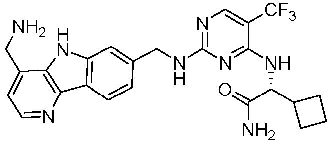 |
| 57 | 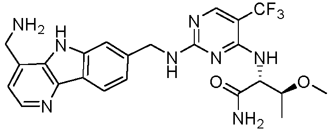 | 58 | 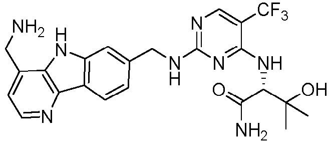 |
| 59 | 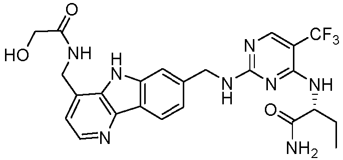 | 60 | 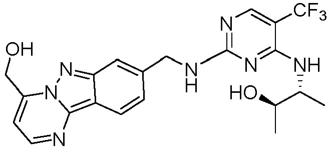 |
| 61 | 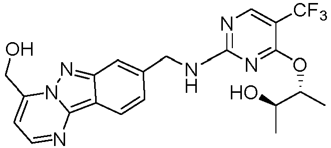 | 62 | 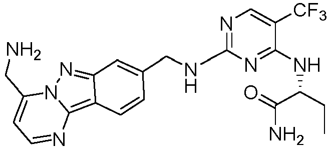 |
| 63 | 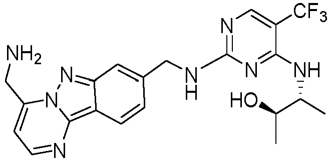 | 64 | 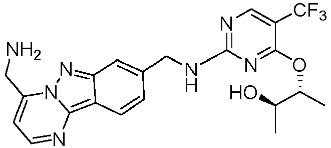 |
| 65 | 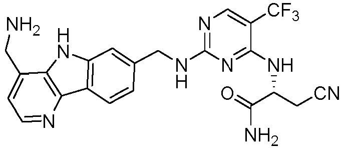 | 66 | 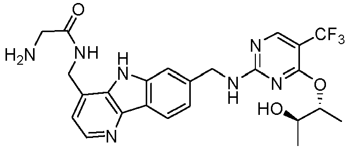 |
| 67 | 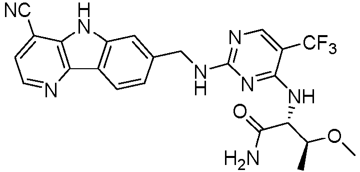 | 68 | 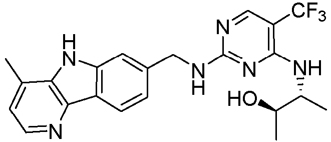 |
| 69 | 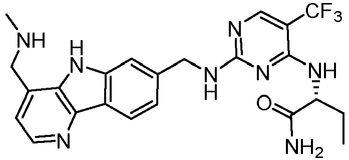 | 70 | 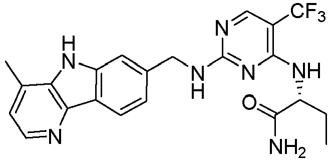 |
| 71 | 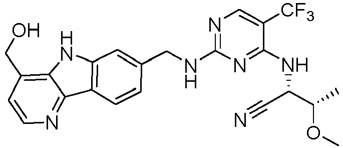 | 72 | 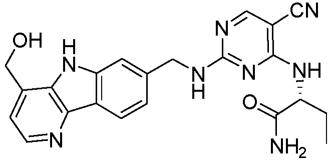 |
| 73 | 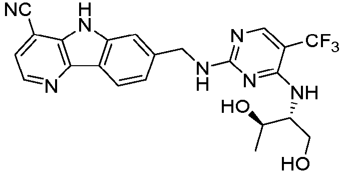 | 74 | 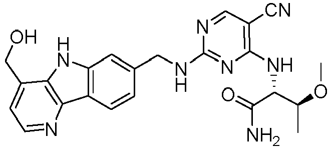 |
| 75 | 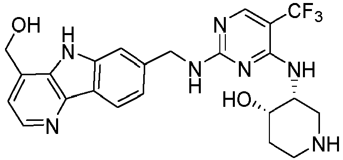 | 76 | 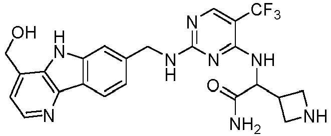 |
| 77 | 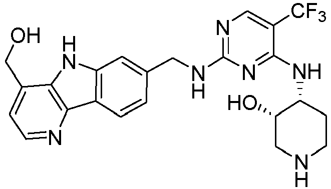 | 78 | 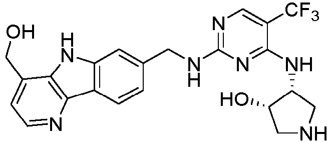 |
| 79 | 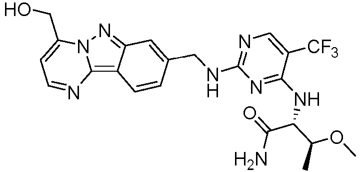 | 80 | 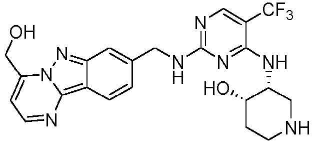 |
| 81 | 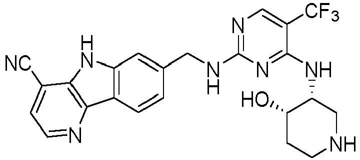 | 82 | 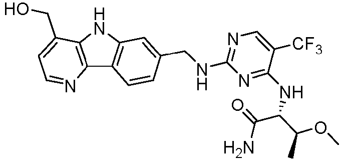 |
| 83 | 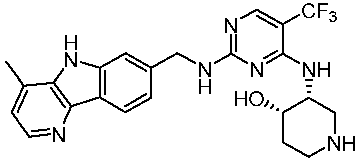 | 84 | 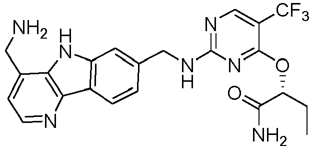 |
| 85 | 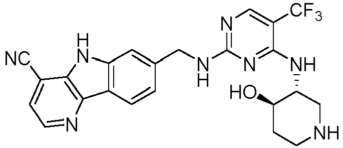 | 86 | 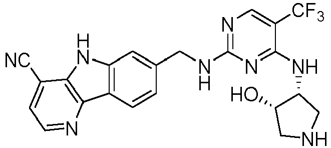 |
| 87 | 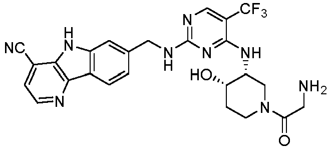 | 88 | 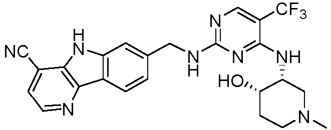 |
| 89 | 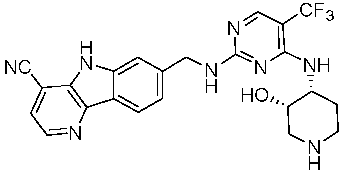 | 90 | 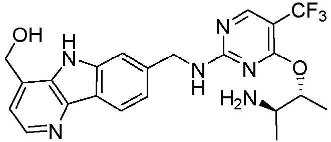 |
| 91 | 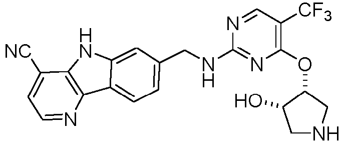 | 92 | 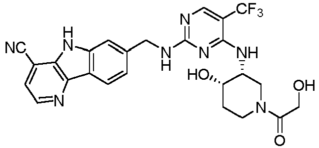 |
| 93 | 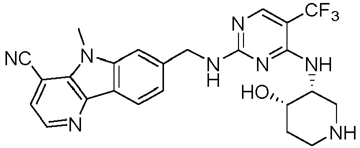 | 94 | 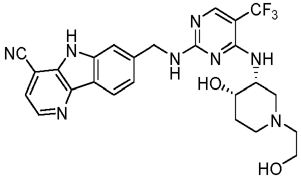 |
| 95 | 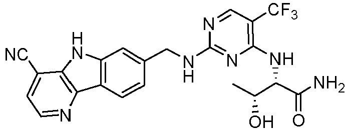 | 96 | 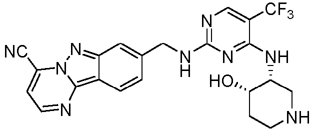 |
| 97 | 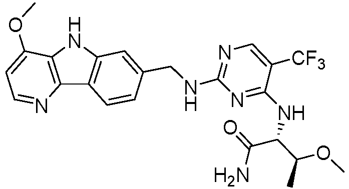 | 98 | 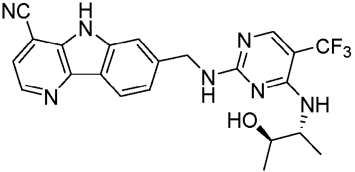 |
| 99 | 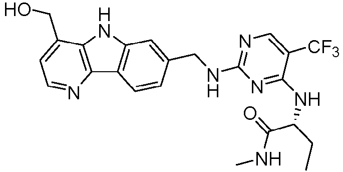 | 10 0 | 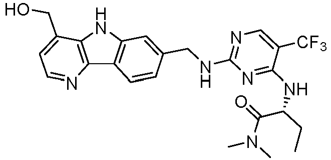 |
| 101 | 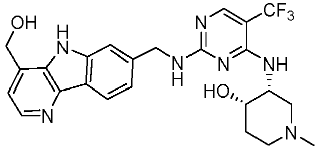 | 102 | 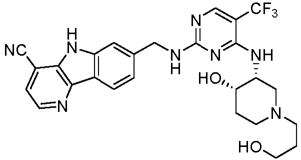 |
| 103 | 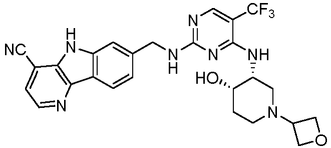 | 104 | 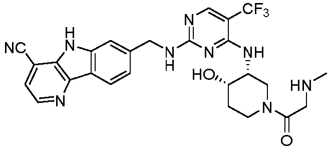 |

25. A pharmaceutical composition comprising the compound, or a stereoisomer, tautomer, solvate, pharmaceutically acceptable salt, metabolite, isotope derivative, N-oxide, or prodrug thereof according to any one of claims 1-24, and a pharmaceutically acceptable carrier, diluent or excipient.

26. Use of the compound, or a stereoisomer, tautomer, solvate, pharmaceutically acceptable salt, metabolite, isotope derivative, N-oxide, or prodrug thereof according to any one of claims 1-24, or the pharmaceutical composition according to claim 25, in the preparation of a medicament for the prevention or treatment of a disease or disorder associated with Cyclin K protein.

27. The use according to claim 26, wherein the disease or disorder is selected from tumor, cancer, viral infection, inflammation-related disease, and autoimmune disease.

28. A method for treating a disease or disorder associated with Cyclin K protein, comprising administering to a mammal in need thereof the compound, or a stereoisomer, tautomer, solvate, pharmaceutically acceptable salt, metabolite, isotope derivative, N-oxide, or prodrug thereof according to any one of claims 1-24, or the pharmaceutical composition according to claim 25.
